# EUROPEAN PATENT SPECIFICATION

(11) **EP 1 974 039 B1**
(45) Date of publication and mention of the grant of the patent: **25.08.2010**
(21) Application number: 06803867.8
(22) Date of filing: 19.09.2006
(51) Int. Cl.: C12N 15/82, C12N 9/00, A23L 1/217

(54) **LOW ACRYLAMIDE FOODS**
LEBENSMITTEL MIT WENIG ACRYLAMID
ALIMENTS A FAIBLE TENEUR D'ACRYLAMIDE

(30) Priority: 20.09.2005 US 718335 P; 28.07.2006 US 833788 P
(43) Date of publication of application: 01.10.2008
(60) Divisional application: 10004151.6
(73) Proprietor: J.R. Simplot Company, Boise, ID 83706 (US)
(72) Inventor: ROMMENS, Caius, Boise, ID 83706 (US)
(74) Representative: Woods, Geoffrey Corlett
(86) International application number: PCT/US2006/036515
(87) International publication number: WO 2007/035752

(56) References cited:
- WO-A-2004/026042
- OLSSON KERSTIN ET AL: "Tuber components affecting acrylamide formation and colour in fried potato: Variation by variety, year, storage temperature and storage time." JOURNAL OF THE SCIENCE OF FOOD AND AGRICULTURE, vol. 84, no. 5, 15 April 2004 (2004-04-15), pages 447-458, XP002435624 ISSN: 0022-5142
- MOROT-GAUDRY-TALARMAIN Y ET AL: "Nitrite accumulation and nitric oxide emission in relation to cellular signaling in nitrite reductase antisense tobacco" PLANTA (BERLIN), vol. 215, no. 5, September 2002 (2002-09), pages 708-715, XP002435555 ISSN: 0032-0935
- GOSHIMA NAOKI ET AL: "Emission of nitrous oxide (N2O) from transgenic tobacco expressing antisense NiR mRNA" PLANT JOURNAL, vol. 19, no. 1, July 1999 (1999-07), pages 75-80, XP002435627 ISSN: 0960-7412
- KITA A ET AL: "Effective ways of decreasing acrylamide content in potato crisps during processing" JOURNAL OF AGRICULTURAL AND FOOD CHEMISTRY, AMERICAN CHEMICAL SOCIETY. WASHINGTON, US, vol. 52, no. 23, 2004, pages 7011-7016, XP003004151 ISSN: 0021-8561
- PEDRESCHI FRANCO ET AL: "Reduction of acrylamide formation in potato slices during frying" LEBENSMITTEL-WISSENSCHAFT & TECHNOLOGIE, vol. 37, no. 6, 2004, pages 679-685, XP002448988 ISSN: 0023-6438

## Description

### FIELD OF THE INVENTION

The present invention relates to genetic methods for down-regulating and up-regulating genes in a plant, for example in the starch-rich storage organs of these plants, to lower the level of acrylamide that accumulates upon processing-associated heating of these organs.

### BACKGROUND

The heating of foods that contain both free asparagine and reducing sugars results in the production of acrylamide. Acrylamide is an industrial chemical used worldwide to synthesize polyacrylamide. Exposure to this reactive compound results in rapid absorption and even distribution among tissues (Barber et al., Neurotoxicology 2001, 22, 341-353). In rodents, the toxicological effects of high concentrations of acrylamide (>2 mg/kg body weight) include neurological symptoms, decreased fertility, and cancer (Friedman, J. Agric. Food Chem, 2003, 51, 4504-4526).

Occupational exposure to high levels of acrylamide is also known to elevate the incidence of neurotoxicity in humans (LoPachin, Neurotoxicology, 2004, 25, 617-630) but there is no documented effect of acrylamide on human reproduction or carcinogenesis. Both acrylamide and its oxidized metabolite glycidamide react with the amino-terminal valine of hemoglobin to form adducts. The extent of adduct formation is a good marker for exposure levels and implies daily intakes approximating 100 µg (Tareke et al., J. Agric. Food Chem., 2002, 50, 4998-5006). Although drinking water, cosmetics, and smoking were initially believed to represent the only main sources for background exposure to acrylamide (Bergmark, Chem. Res. Toxicol., 1997, 10, 78-84), recent analyses indicate that consumption of fried and baked starchy foods contribute to about 36% of acrylamide intake (Becker and Pearson, Dietary habits and nutrient intake in Sweden 1997-98. *Riksmaten* 1997-98, 1999).

Dietary acrylamide is largely derived from heat-induced reactions between the amino group of the free amino acids asparagine and the carbonyl group of reducing sugars (Mottram et al., Nature, 2002, 419, 448-449; Stadler et al., Nature, 2002, 419, 449-450). Fresh potato tubers contain very high levels of asparagine but relatively low concentrations of the reducing sugars glucose and fructose. However, reducing sugars accumulate during cold storage through expression of cold-induced invertases, which catalyze the conversion of sucrose into glucose and fructose.

Previous attempts to limit the accumulation of acrylamide in starchy foods have not resulted in practical and cost-effective applications. A first method of the prior art is based on modifying processing parameters such as surface-to-volume ratio, temperature, and frying time. Although application of such methods may be partially effective in lowering the accumulation of acrylamide, it alters the sensory characteristics of the final food product by reducing color, modifying shape, and altering taste and texture. Such alterations are undesirable.

A second method is based on the incubation of partially-processed food products with asparaginase (EC 3.5.1.1) or glurninase (EC 3.4.1.2). prior to heating (see World Patent application 2004/030468 A3 and World Patent application 2004/026042 A1). This method is expensive and only readily applicable to materials such as wheat flour that can easily be mixed with the enzyme.

A third method adds an asparagine competitor such as glycine to the partially-processed food (World Patent Application 2005/025330 A1 for potato tubers, US Patent Application 2004/0081724 A1 for roasted coffee beans, World Patent Application 2005/004620 A1 for cocoa beans, World Patent Application 2005/004628 A1 for corn-based foods). This method is only partially effective, requires high concentrations of the additive, and is too costly to apply broadly.

A fourth method adds a reducing sugar-altering enzyme comprising aldose reductase to the food material prior to heating (See US Patent 6989167). This method is not highly effective and too costly to apply broadly.

A fifth method coats the food with a reagent selected from the group consisting of an amino acid-containing compound, an amino acid salt, an amino acid amide, an amino acid ester, and mixtures thereof, prior to heating (see World Patent application 2005/077203A3). This method is only partially effective and too costly to apply broadly.

A sixth method is based on the selection of germplasm that contains unusually low levels of both reducing sugars and asparagine. The availability of such germplasm would make it possible to introgress the 'low sugar' and 'low asparagine' traits into varieties that are acceptable for broad use in the food industry. However, no low asparagine crop plants have yet been identified. Even if such germplasm would be discovered in the future, it would take at least 15 to 20 years to introgress the desired traits into commercial varieties.

A seventh method genetically modifies the crop to reduce the levels of reducing sugars. This method is based on down-regulating the expression of genes involved in starch degradation such as the potato starch-associated R1 and phosphorylase-L genes (see, for instance, US Patent Application 2003/0221213 A1). Although partially effective, processed foods derived from the modified crops still contain about a third of the acrylamide levels that are found in control products.

Thus, there is an important need for methods to reduce acrylamide levels in processed foods that are obtained from starchy crops. Such methods should be in a cost-effective manner without lowering the sensory characteristics of the food. The present invention provides such methods.
Olsson et al (J. Sci. Food Agri., 2004, 447-458) relates to tuber components affecting acrylamide formation and colour in fried potato. WO 2004/026042 relates to a method for reducing acrylamide formation in thermally processed foods.

### SUMMARY OF THE INVENTION

The invention provides a method for reducing the acrylamide content in a heat-processed plant product, comprising reducing asparagine levels in the plant that is used to produce the product by expressing in the plant a polynucleotide that has the complete or partial sense or antisense sequence of a gene that encodes an enzyme that catalyses the synthesis of asparagine from aspartate.

The invention further provides a method for producing an edible plant product from a plant tissue with reduced asparagine, comprising (1) increasing the level of asparaginase in the plant tissue or (2) decreasing the level of asparagine synthetase in the plant tissue.

The invention further provides a method for producing an edible plant product with low levels of acrylamide, comprising (i) downregulating the expression of an asparagine biosynthetic gene and/or upregulating the expression of a gene involved in asparagine metabolism, and (ii) downregulating the expression or activity of at least one of (a) the R1 gene and (b) the phosphorylase L gene in the tissue of a plant that produces a vegetable, seed, or fruit from which the product is made.

The invention further provides a method for producing an edible transgenic plant product that has a lower acrylamide level after it is heated than the acrylamide level of an equivalently heated product from a non-transgenic plant, comprising reducing asparagine levels in the transgenic plant by altering the expression level of a gene involved in asparagine biosynthesis or asparagine metabolism in the plant.

The invention further provides a method for over-expressing a gene in a potato tuber by operably linking that gene to a sequence that displays at least 70% identity with at least part of the sequence shown in SEQ ID NO.: 13.

The invention further provides a method for reducing the acrylamide content in a heat-processed plant product, comprising downregulating the expression of an asparagine synthetase gene in plant that is used to produce a heat-processed plant product.

In one embodiment, the invention provides a new strategy for reducing the levels of acrylamide in a processed food that was obtained by heating the starchy tissues of a crop. Uniquely, this strategy employs methods in genetic engineering to reduce the levels of asparagine in the starchy tissues of a crop plant by at least 50%.

In one embodiment, the levels of asparagine in the starchy tissues of a crop plant are reduced by lowering the level of asparagine biosynthesis and/or increasing the level of asparagine metabolism. Either mechanism may entail down-regulating or up-regulating genes that are directly or indirectly involved in each asparagine pathway. In one aspect, the gene involved in asparagine metabolism encodes an asparaginase. In one aspect, the gene involved in asparagine biosynthesis encodes an asparagine synthetase.

In another aspect, genes that are indirectly involved in an asparagine pathway are selected from the group consisting of a glutamine synthetase (GS) gene, a nitrate reductase (NR) gene, a 14-3-3 gene, and a bexokinase (HXK) gene.

In one aspect, the level of asparagine biosynthesis is lowered by reducing the expression of at least one gene involved in asparagine biosynthesis.

In one aspect, lowered expression of a gene involved in asparagine biosynthesis is accomplished by introducing into a plant an expression cassette comprising, from 5' to 3', (i) a promoter, (ii) at least one copy of a sequence comprising at least a fragment of at least one gene involved in asparagine metabolism, and optionally, (iii) either a second promoter or a terminator, whereby the first and optional second promoter are positioned in the convergent orientation.

In one aspect, the expression cassette that is used to lower expression of a gene involved in asparagine biosynthesis contains two copies of a sequence comprising at least a fragment of the gene involved in asparagine metabolism.

In one aspect, the two copies are positioned as (i) inverted repeat, or (ii) direct repeat.

In one aspect, the gene involved in asparagine biosynthesis is isolated from potato, a wild potato such as *Solanum phureja,* sweet potato, yam, coffee tree, cocoa tree, wheat, maize, oats, sorghum, or barley, and encodes an asparagine synthetase.

In one aspect the asparagine synthetase gene comprises a sequence that shares at least 90% identity with at least a fragment of the sequence shown in SEQ ID.: 1.

In another aspect the asparagine synthetase gene comprises a sequence that shares at least 90% identity with at least a fragment of the sequence shown in SEQ ID.: 2.

In one aspect, overexpression of a gene involved in asparagine metabolism is accomplished by introducing into a plant an expression cassette comprising, from 5' to 3', a first promoter, a gene involved in asparagine biosynthesis, and a terminator.

In one aspect, the gene involved in asparagine metabolism is isolated from potato, a wild potato such as *Solanum phureja*, sweet potato, yarn, coffee tree, cocoa tree, wheat, maize, oats, sorghum, or barley, and encodes an asparaginase.

In one aspect the asparaginase gene comprises a sequence that shares at least 70% identity with the sequence shown in SEQ ID.: 9, 14, 31, 32, or 33.

In another aspect the asparaginase gene comprises a sequence that shares at least 70% identity with the sequence shown in SEQ ID.: 14.

In another aspect, the gene involved in asparagine biosynthesis is a glutamine synthetase or bexokinase gene.

In one aspect, the promoter is a promoter of (i) a potato granule bound starch synthase gene, (ii) a potato ADP glucose pyrophosphorylase gene, (iii) a potato patatin gene, (iv) a potato flavonoid mono-oxygenase gene.

In one aspect, the promoter of a potato granule bound starch synthase gene shares at least 70% identity with at least part of SEQ ID NO.: 8, the promoter of a potato ADP glucose pyrophosphorylase gene shares at least 70% identity with at least part of SEQ ID NO.: 7, the promoter of a potato patatin gene shares at least 70% identity with at least part of SEQ ID NO.: 22, and a promoter of a potato flavonoid mono-oxygenase gene shares at least 70% identity with at least part of SEQ ID NO.: 13.

In another aspect the promoter is the promoter of a gene that is expressed in a tuber, root, or seed of a starchy crop destined for food processing.

In another embodiment, the invention provides a method for reducing the levels of acrylamide in a food that was obtained by heating the tissues of a crop by simultaneously reducing the levels of both asparagine and reducing-sugars in the tissues. In one embodiment, the tissue is a starchy tissue of the crop or plant.

In one aspect, the simultaneous reduction in levels of asparagine and reducing-sugars is obtained by (i) either downregulating the expression of a gene involved in asparagine biosynthesis or overexpressing a gene involved in asparagine metabolism, and (ii) downregulating the expression of at least one gene involved starch degradation.

In one aspect, the expression of a gene in starch degradation is downregulated by introducing into a plant an expression cassette comprising, from 5' to 3', (i) a promoter, (ii) at least one copy of a sequence comprising at least a fragment of a gene involved in starch degradation, and optionally, (iii) either a second promoter or a terminator, whereby the first and optional second promoter are positioned in the convergent orientation.

In one aspect, a gene involved in starch degradation is selected from the group consisting of (i) a starch-associated *R1* gene, and (ii) a starch-associated phosphorylase-L gene.

In another embodiment, the invention provides a method for simultaneously reducing the levels of acrylamide in a food that was obtained by heating die tissues of a crop and increasing the sensory characteristics of this food by simultaneously reducing the levels of asparagine and reducing-sugars in the tissues. In one embodiment, the tissue is a starchy tissue of the crop or plant.

In one aspect, simultaneous reduction is accomplished by employing a 'multigene-targeting' construct comprising a first expression cassette comprising either (i) an asparaginase gene operably linked to a promoter or (ii) at least one copy of a fragment of an asparagine synthetase gene operably linked to a promoter and a second expression cassette comprising at least one copy of a DNA segment comprising both a fragment of the *R1* gene and a fragment of the phosphorylase gene operably linked to a promoter, whereby the first and second expression cassette can be the same expression cassette.

Based on U.S. Food an Drug Administration data (www.cfsan.fda.gov/∼dms/acrydata.html), a typical French fry produced at a restaurant of a large fast food chain contains more than 100 parts-per-billion (ppb) acrylamide. The average amount of acrylamide in such a typical French fry is 404 ppb, and the average daily intake levels of acrylamide through consumption of French fries is 0.07 microgrem/kilogram of bodyweight/day. Consequently, French fries represent 16% of the total dietary intake of acrylamide. The average amount of acrylamide in oven-baked French fries and potato chips produced by a commercial processorare 698 ppb and 597 ppb, respectively. Thus, potato-derived processed foods including French fries, over-baked fries, and potato chips represent 3 8% of the total dietary intake for acrylamide.

According to the present invention, the level of acrylamide that is present in a French fry, baked fry, or chip produced by a method of the present invention is lower than the level of acrylamide in a French fry, baked fry, or chip that is obtained from a method known in the art by greater than 2-fold, 3-fold, 4-fold, 5-fold, 6-fold, 7-fold, 8-fold, 9-fold,10-fold, 11-fold, 12-fold, 13-fold, 14-fold, 15-fold, 16-fold, 17-fold, 18-fold, 19-fold, 20-fold, or more than 20-fold.

In terms of parts per billion, a French fry produced by a method of the present invention may have between 1-20 ppb, 20-40 ppb, 40-60 ppb, 60-80 ppb, or 80-100 ppb acrylamide.

In terms of parts per billion, a oven-baked fry, potato chip, or hash brown produced by a method of the present invention may have between 1-20 ppb, 20-40 ppb, 40-60 ppb, 60-80 ppb, 80-100 ppb, 100-120 ppb, 120-140 ppb, 140-160 ppb, 160-180 ppb, or 180-200 ppb acrylamide.

The present invention is not limited to reducing the level of acrylamide in Fry and chip products of tubers. Other foodstuffs may be manipulated according to the present invention to reduce acrylamide levels.

The levels of acrylamide in breakfast cereals can be reduced from about 50-250 ppb to levels below 40 ppb, preferably to levels below 20 ppb.

The levels of acrylamide in crackers such as Dare Breton Thin Wheat Crackers and Wasa Original Crispbread Fiber Rye can be reduced from about 300-500 ppb to levels below 100 ppb, preferably to levels below 50 ppb.

The levels of acrylamide in chocolate such as Ghirardelli Unsweetened Cocoa or Hershey's Cocoa can be reduced from about 300-900 ppb to levels below 200 ppb, preferably to levels below 50 ppb.

The levels of acrylamide in cookies can be reduced from about 50-200 ppb to levels below 40 ppb, preferably to levels below 20 ppb.

The levels of acrylamide in ground coffee can be reduced from about 175-350 ppb to levels below 60 ppb, preferably to levels below 30 ppb.

The levels of acrylamide in wheat bread can be reduced from about 50-150 ppb to levels below 30 ppb, preferably to levels below 15 ppb.

It should be understood that many factors influence the levels of acrylamide in a final food product. Such factors include crop, variety, growing conditions, storage conditions of the harvested seed or tuber, and processing variables such as heating temperature, heating time, type of oil used for frying, and exposed surface.

Application of the methods described in the present invention will lower acrylamide levels by at least about 5%, at least about 10%, at least about 15%, at least about 20%, at least about 25%, at least about 30%, at least about 35%, at least about 40%, at least about 45%, 50%, by at least about 60%, by at least about 70%, by at least about 80%, by at least about 90% or by more than about 90%.

### BRIEF DESCRIPTION OF THE DRAWINGS

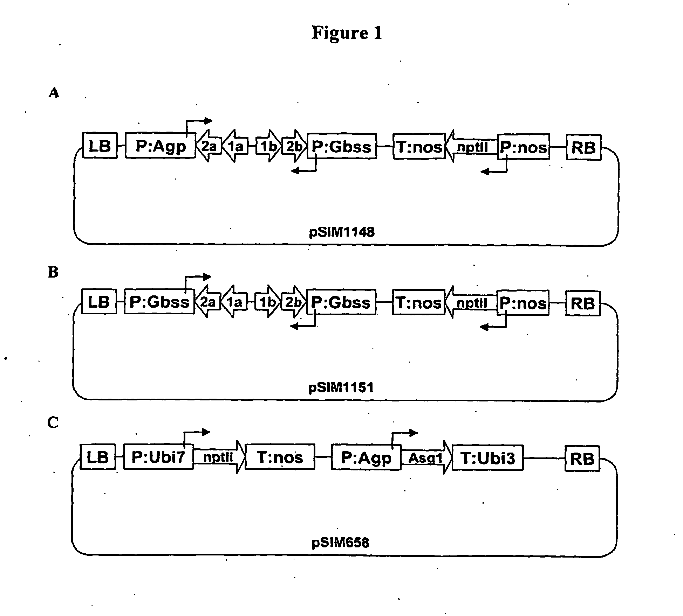

**Figure 1****:** Diagram of (A) pSIM1148, (B) pSIM1151, and (C) pSIM658. LB = left T-DNA border, P:Agp = potato Agp promoter, 2a = antisense copy of an ast2 gene fragment, 1 a= antisense copy of an ast1 gene fragment, 1b= sense copy of an ast1 gene fragment, 2b= sense copy of an ast2 gene fragment, P:Gbss = potato Gbss promoter, T:nos = terminator of the Agrobacterium nopaline synthase gene, P:nos = promoter of the Agrobacterium nopaline synthase gene, RB = right T-DNA border, P:Ubi7 = promoter of the potato Ubiquitin-7 gene.

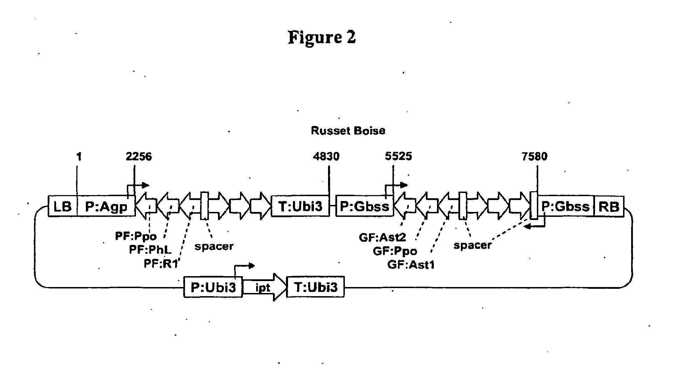

**Figure 2****:** Russet Boise construct. PF= promoter fragment, GF = gene fragment.

### DETAILED DESCRIPTION OF THE PREFERRED EMBODIMENTS

The present invention provides methods for reducing acrylamide levels.

The present invention uses terms and phrases that are well known to those practicing the art. Unless defined otherwise, all technical and scientific terms used herein have the same meaning as commonly understood by one of ordinary skill in the art to which this invention belongs. Generally, the nomenclature used herein and the laboratory procedures in cell culture, molecular genetics, and nucleic acid chemistry and hybridization described herein are those well known and commonly employed in the art. Standard techniques are used for recombinant nucleic acid methods, polynucleotide synthesis, microbial culture, cell culture, tissue culture, transformation, transfection, transduction, analytical chemistry, organic synthetic chemistry, chemical syntheses, chemical analysis, and pharmaceutical formulation and delivery. Generally, enzymatic reactions and purification and/or isolation steps are performed according to the manufacturers specifications. The techniques and procedures are generally performed according to conventional methodology (Molecular Cloning, A Laboratory Manual, 3rd. edition, edited by Sambrook & Russel Cold Spring Harbor Laboratory Press, Cold Spring Harbor, NY, 2001).

Acrylamide: Acrylamide as a monomer is considered toxic, directly affecting the nervous system. It may be considered a carcinogen. Acrylamide is readily absorbed through intact skin from aqueous solutions. Molecular formula is C3H5NO; structure: CH2=CH-CO-NH2.

Agrobacterium or bacterial transformation: as is well known in the field, Agrobacteria that are used for transforming plant cells are disarmed and virulent derivatives of, usually, Agrobacterium tumefaciens or Agrobacterium rhizogenes. Upon infection of plants, explants, cells, or protoplasts, the Agrobacterium transfers a DNA segment from a plasmid vector to the plant cell nucleus. The vector typically contains a desired polynucleotide that is located between the borders of a T-DNA or P-DNA. However, any bacteria capable of transforming a plant cell may be used, such as, Rhizobium trifolii, Rhizobium leguminosarum, Phyllobacterium myrsinacearum, SinoRhizobium meliloti, and MesoRhizobium loti.

**Angiosperm:** vascular plants having seeds enclosed in an ovary. Angiosperms are seed plants that produce flowers that bear fruits. Angiosperms are divided into dicotyledonous and monocotyledonous plant.

**Asparagine biosynthesis:** enzymatically-catalyzed reactions that occur in a plant to produce asparagine

**Asparagine metabolism:** enzymatically-catalyzed reactions that occur in a plant to convert asparagine into other compounds

**Asparaginase: Asparaginase,** which is found in various plant, animal and bacterial cells, is an enzyme involved in asparagine metabolism. It catalyses the deamination of asparagine to yield aspartic acid and an ammonium ion, resulting in a depletion of free circulatory asparagine.

**Asparagine synethetase:** This enzyme is involved in asparagine biosynthesis, and catalyzes the synthesis of asparagine from aspartate.

**Antibiotic Resistance:** ability of a cell to survive in the presence of an antibiotic. Antibiotic resistance, as used herein, results from the expression of an antibiotic resistance gene in a host cell. A cell may have resistance to any antibiotic. Examples of commonly used antibiotics include kanamycin and hygromycin.

**Dicotyledonous plant (dicot):** a flowering plant whose embryos have two seed halves or cotyledons, branching leaf veins, and flower parts in multiples of four or five. Examples of dicots include but are not limited to, potato, sugar beet, broccoli, cassava, sweet potato, pepper, poinsettia, bean, alfalfa, soybean, and avocado.

**Endogenous:** nucleic acid, gene, polynucleotide, DNA, RNA, mRNA, or cDNA molecule that is isolated either from the genome of a plant or plant species that is to be transformed or is isolated from a plant or species that is sexually compatible or interfertile with the plant species that is to be transformed, is "native" to, i.e., indigenous to, the plant species.

**Expression cassette:** polynucleotide comprising, from 5' to 3', (a) a first promoter, (b) a sequence comprising (i) at least one copy of a gene or gene fragment, or (ii) at least one copy of a fragment of the promoter of a gene, and (c) either a terminator or a second promoter that is positioned in the opposite orientation as the first promoter.

**Foreign:** "foreign," with respect to a nucleic acid, means that that nucleic acid is derived from non-plant organisms, or derived from a plant that is not the same species as the plant to be transformed or is not derived from a plant that is not interfertile with the plant to be transformed, does not belong to the species of the target plant. According to the present invention, foreign DNA or RNA represents nucleic acids that are naturally occurring in the genetic makeup of fungi, bacteria, viruses, mammals, fish or birds, but are not naturally occurring in the plant that is to be transformed. Thus, a foreign nucleic acid is one that encodes, for instance, a polypeptide that is not naturally produced by the transformed plant A foreign nucleic acid does not have to encode a protein product.

**Gene:** A gene is a segment of a DNA molecule that contains all the information required for synthesis of a product, polypeptide chain or RNA molecule that includes both coding and non-coding sequences. A gene can also represent multiple sequences, each of which may be expressed independently, and may encode slightly different proteins that display the same functional activity. For instance, the asparagine synthetase 1 and 2 genes can, together, be referred to as a gene.

**Genetic element:** a "genetic element" is any discreet nucleotide sequence such as, but not limited to, a promoter, gene, terminator, intron, enhancer, spacer, 5'-untranslated region, 3'-untranslated region, or recombinase recognition site.

**Genetic modification:** stable introduction of DNA into the genome of certain organisms by applying methods in molecular and cell biology.

**Gymnosperm:** as used herein, refers to a seed plant that bears seed without ovaries. Examples of gymnosperms include conifers, cycads, ginkgos, and ephedras.

**Introduction:** as used herein, refers to the insertion of a nucleic acid sequence into a cell, by methods including infection, transfection, transformation or transduction.

**Monocotyledonous plant (monocot):** a flowering plant having embryos with one cotyledon or seed leaf, parallel leaf veins, and flower parts in multiples of three. Examples of monocots include, but are not limited to maize, rice, oat, wheat, barley, and sorghum.

**Native:** nucleic acid, gene, polynucleotide, DNA, RNA, mRNA, or cDNA molecule that is isolated either from the genome of a plant or plant species that is to be transformed or is isolated from a plant or species that is sexually compatible or interfertile with the plant species that is to be transformed, is "native" to, i.e., indigenous to, the plant species.

**Native DNA:** any nucleic acid, gene, polynucleotide, DNA, RNA, mRNA, or cDNA molecule that is isolated either from the genome of a plant or plant species that is to be transformed or is isolated from a plant or species that is sexually compatible or interfertile with the plant species that is to be transformed, is "native" to, i.e., indigenous to, the plant species. In other words, a native genetic element represents all genetic material that is accessible to plant breeders for the improvement of plants through classical plant breeding. Any variants of a native nucleic acid also are considered "native" in accordance with the present invention. For instance, a native DNA may comprise a point mutation since such point mutations occur naturally. It is also possible to link two different native DNAs by employing restriction sites because such sites are ubiquitous in plant genomes.

**Native Nucleic Acid Construct:** a polynucleotide comprising at least one native DNA.

**Operably linked:** combining two or more molecules in such a fashion that in combination they function properly in a plant cell. For instance, a promoter is operably linked to a structural gene when the promoter controls transcription of the structural gene.

**Overexpression:** expression of a gene to levels that are higher than those in plants that are not transgenic.

**P-DNA:** a plant-derived transfer-DNA ("P-DNA") border sequence of the present invention is not identical in nucleotide sequence to any known bacterium-derived T-DNA border sequence, but it functions for essentially the same purpose. That is, the P-DNA can be used to transfer and integrate one polynucleotide into another. A P-DNA can be inserted into a tumor-inducing plasmid, such as a Ti-plasmid from Agrobacterum in place of a conventional T-DNA, and maintained in a bacterium strain, just like conventional transformation plasmids. The P-DNA can be manipulated so as to contain a desired polynucleotide, which is destined for integration into a plant genome via bacteria-mediated plant transformation. See Rommens et al. in WO2003/069980, US-2003-0221213, US-2004-0107455, and WO2005/004585, which are all incorporated herein by reference.

**Phenotype:** phenotype is a distinguishing feature or characteristic of a plant, which may be altered according to the present invention by integrating one or more "desired polynucleotides" and/or screenable/selectable markers into the genome of at least one plant cell of a transformed plant. The "desired polynucleotide(s)" and/or markers may confer a change in the phenotype of a transformed plant, by modifying any one of a number of genetic, molecular, biochemical, physiological, morphological, or agronomic characteristics or properties of the transformed plant cell or plant as a whole. Thus, expression of one or more, stably integrated desired polynucleotide(s) in a plant genome that yields the phenotype of reduced acrylamide concentrations in plant tissues.

**Plant tissue:** a "plant" is any of various photosynthetic, eukaryotic, multicellular organisms of the kingdom Plantae characteristically producing embryos, containing chloroplasts, and having cellulose cell walls. A part of a plant, i.e., a "plant tissue" may be treated according to the methods of the present invention to produce a transgenic plant. Many suitable plant tissues can be transformed according to the present invention and include, but are not limited to, somatic embryos, pollen, leaves, stems, calli, stolons, microtubers, and shoots. Thus, the present invention envisions the transformation of angiosperm and gymnosperm plants such as wheat, maize, rice, barley, oat, sugar beet, potato, tomato, alfalfa, cassava, sweet potato, and soybean. According to the present invention "plant tissue" also encompasses plant cells. Plant cells include suspension cultures, callus, embryos, meristematic regions, callus tissue, leaves, roots, shoots, gametophytes, sporophytes, pollen, seeds and microspores. Plant tissues may be at various stages of maturity and may be grown in liquid or solid culture, or in soil or suitable media in pots, greenhouses or fields. A plant tissue also refers to any clone of such a plant, seed, progeny, propagule whether generated sexually or asexually, and descendents of any of these, such as cuttings or seed. Of particular interest are potato, maize, and wheat.

**Plant transformation and cell culture:** broadly refers to the process by which plant cells are genetically modified and transferred to an appropriate plant culture medium for maintenance, further growth, and/or further development. Such methods are well known to the skilled artisan.

**Processing:** the process of producing a food from (1) the seed of, for instance, wheat, corn, coffee plant, or cocoa tree, (2) the tuber of, for instance, potato, or (3) the root of, for instance, sweet potato and yam comprising heating to at least 120°C. Examples of processed foods include bread, breakfast cereal, pies, cakes, toast, biscuits, cookies, pizza, pretzels, tortilla, French fries, oven-baked fries, potato chips, hash browns, roasted coffee, and cocoa.

**Progeny:** a "progeny" of the present invention, such as the progeny of a transgenic plant, is one that is born of, begotten by, or derived from a plant or the transgenic plant. Thus, a "progeny" plant, i.e., an "F1" generation plant is an offspring or a descendant of the transgenic plant produced by the inventive methods. A progeny of a transgenic plant may contain in at least one, some, or all of its cell genomes, the desired polynucleotide that was integrated into a cell of the parent transgenic plant by the methods described herein. Thus, the desired polynucleotide is "transmitted" or "inherited" by the progeny plant. The desired polynucleotide that is so inherited in the progeny plant may reside within a T-DNA or P-DNA construct, which also is inherited by the progeny plant from its parent. The term "progeny" as used herein, also may be considered to be the offspring or descendants of a group of plants.

**Promoter:** promoter is intended to mean a nucleic acid, preferably DNA that binds RNA polymerase and/or other transcription regulatory elements. As with any promoter, the promoters of the current invention will facilitate or control the transcription of DNA or RNA to generate an mRNA molecule from a nucleic acid molecule that is operably linked to the promoter. As stated earlier, the RNA generated may code for a protein or polypeptide or may code for an RNA interfering, or antisense molecule.

A promoter is a nucleic acid sequence that enables a gene with which it is associated to be transcribed. In prokaryotes, a promoter typically consists of two short sequences at -10 and -35 position upstream of the gene, that is, prior to the gene in the direction of transcription. The sequence at the -10 position is called the Pribnow box and usually consists of the six nucleotides TATAAT. The Pribnow box is essential to start transcription in prokaryotes. The other sequence at -35 usually consists of the six nucleotides TTGACA, the presence of which facilitates the rate of transcription.

Eukaryotic promoters are more diverse and therefore more difficult to characterize, yet there are certain fundamental characteristics. For instance, eukaryotic promoters typically He upstream of the gene to which they are most immediately associated. Promoters can have regulatory elements located several kilobases away from their transcriptional start site, although certain tertiary structural formations by the transcriptional complex can cause DNA to fold, which brings those regulatory elements closer to the actual site of transcription. Many eukaryotic promoters contain a "TATA box" sequence, typically denoted by the nucleotide sequence, TATAAA. This element binds a TATA binding protein, which aids formation of the RNA polymerase transcriptional complex. The TATA box typically lies within 50 bases of the transcriptional start site.

Eukaryotic promoters also are characterized by the presence of certain regulatory sequences that bind transcription factors involved in the formation of the transcriptional complex. An example is the E-box denoted by the sequence CACGTG, which binds transcription factors in the basic-belix-loop-helix family. There also are regions that are high in GC nucleotide content.

Hence, according to the methods of the present invention, a partial sequence, or a specific promoter "fragment" of a promoter, say for instance of the asparagine synthetase gene, that may be used in the design of a desired polynucleotide for use in the methods of the present invention may or may not comprise one or more of these elements or none of these elements. In one embodiment, a promoter fragment sequence for use in the methods of the present invention is not functional and does not contain a TATA box.

Another characteristic of the construct for use in the methods of the present invention is that it promotes convergent transcription of one or more copies of polynucleotide that is or are not directly operably linked to a terminator, via two opposing promoters. Due to the absence of a termination signal, the length of the pool of RNA molecules that is transcribed from the first and second promoters may be of various lengths.

Occasionally, for instance, the transcriptional machinery may continue to transcribe past the last nucleotide that signifies the "end" of the desired polynucleotide sequence. Accordingly, in this particular arrangement, transcription termination may occur either through the weak and unintended action of downstream sequences that, for instance, promote hairpin formation or through the action of unintended transcriptional terminators located in plant DNA flanking the transfer DNA integration site.

The desired polynucleotide for use in the methods of the present invention may be linked in two different orientations to the promoter. In one orientation, e.g., "sense", at least the 5'-part of the resultant RNA transcript will share sequence identity with at least part of at least one target transcript. In the other orientation designated as "antisense", at least the 5'-part of the predicted transcript will be identical or homologous to at least part of the inverse complement of at least one target transcript.

A plant promoter is a promoter capable of initiating transcription in plant cells whether or not its origin is a plant cell. Exemplary plant promoters include, but are not limited to, those that are obtained from plants, plant viruses, and bacteria such as Agrobacterium or Rhizobium which comprise genes expressed in plant cells. Examples of promoters under developmental control include promoters that preferentially initiate transcription in certain tissues, such as xylem, leaves, roots, or seeds. Such promoters are referred to as tissue-preferred promoters. Promoters which initiate transcription only in certain tissues are referred to as tissue-specific promoters. A cell type- specific promoter primarily drives expression in certain cell types in one or more organs, for example, vascular cells in roots or leaves. An inducible or repressible promoter is a promoter which is under environmental control. Examples of environmental conditions that may effect transcription by inducible promoters include anaerobic conditions or the presence of light. Tissue specific, tissue preferred, cell type specific, and inducible promoters constitute the class of non-constitutive promoters. A constitutive promoter is a promoter which is active under most environmental conditions, and in most plant parts.

A polynucleotide for use in the methods of the present invention is a nucleotide sequence, comprising a gene coding sequence or a fragment thereof, (comprising at least 15 consecutive nucleotides, preferably at least 30 consecutive nucleotides, and more preferably at least 50 consecutive nucleotides), a promoter, an intron, an enhancer region, a polyadenylation site, a translation initiation site, 5' or 3' untranslated regions, a reporter gene, a selectable marker or the like. The polynucleotide may comprise single stranded or double stranded DNA or RNA. The polynucleotide may comprise modified bases or a modified backbone. The polynucleotide may be genomic, an RNA transcript (such as an mRNA) or a processed nucleotide sequence (such as a cDNA). The polynucleotide may comprise a sequence in either sense or antisense orientations.

An isolated polynucleotide for use in the methods of the present invention is a polynucleotide sequence that is not in its native state, e.g., the polynucleotide is comprised of a nucleotide sequence not found in nature or the polynucleotide is separated from nucleotide sequences with which it typically is in proximity or is next to nucleotide sequences with which it typically is not in proximity.

Seed: a "seed" may be regarded as a ripened plant ovule containing an embryo, and a propagative part of a plant, as a tuber or spore. Seed may be incubated prior to Agrobacterium-mediated transformation, in the dark, for instance, to facilitate germination. Seed also may be sterilized prior to incubation, such as by brief treatment with bleach. The resultant seedling can then be exposed to a desired strain of Agrobacterium.

Selectable/screenable marker: a gene that, if expressed in plants or plant tissues, makes it possible to distinguish them from other plants or plant tissues that do not express that gene. Screening procedures may require assays for expression of proteins encoded by the screenable marker gene. Examples of selectable markers include the neomycin phosphotransferase (NptII) gene encoding kanamycin and geneticin resistance, the hygromycin phosphotransferase (HptII) gene encoding resistance to hygromycin, or other similar genes known in the art.

Sensory characteristics: panels of professionally trained individuals can rate food products for sensory characteristics such as appearance, flavor, aroma, and texture. A rating of French fries that are obtained from tubers that are down-regulated in R1 and phosphorylse-L gene expression levels is described in Example 4. French fries from tubers described in Example 5 will also display enhanced sensory characteristics. Thus, the methods of the present invention may be used to improve the sensory characteristics of a plant product obtained from a plant that has been modified according to the methods of the present invention to manipulate its asparagine biosynthesis and metabolism pathways.

Sequence identity: as used herein, "sequence identity" or "identity" in the context of two nucleic acid or polypeptide sequences includes reference to the residues in the two sequences which are the same when aligned for maximum correspondence over a specified region. When percentage of sequence identity is used in reference to proteins it is recognized that residue positions which are not identical often differ by conservative amino acid substitutions, where amino acid residues are substituted for other amino acid residues with similar chemical properties (e.g. charge or hydrophobicity) and therefore do not change the functional properties of the molecule. Where sequences differ in conservative substitutions, the percent sequence identity may be adjusted upwards to correct for the conservative nature of the substitution. Sequences which differ by such conservative substitutions are said to have "sequence similarity" or "similarity." Means for making this adjustment are well-known to those of skill in the art. Typically this involves scoring a conservative substitution as a partial rather than a full mismatch, thereby increasing the percentage sequence identity. Thus, for example, where an identical amino acid is given a score of 1 and a non conservative substitution is given a score of zero, a conservative substitution is given a score between zero and 1. The scoring of conservative substitutions is calculated, e.g., according to the algorithm of Meyers and Miller, Computer Applic. Biol. Sci., 4: 11 17 (1988) e.g., as implemented in the program PC/GENE (Intelligenetics, Mountain View, California, USA).

As used herein, percentage of sequence identity means the value determined by comparing two optimally aligned sequences over a comparison window, wherein the portion of the polynucleotide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity.

"Sequence identity" has an art-recognized meaning and can be calculated using published techniques. See COMPUTATIONAL MOLECULAR BIOLOGY, Lesk, ed. (Oxford University Press, 1988), BIOCOMPUTING: INFORMATICS AND GENOME PROJECTS, Smith, ed. (Academic Press, 1993), COMPUTER ANALYSIS OF SEQUENCE DATA, PART I, Griffin & Griffin, eds., (Humana Press, 1994), SEQUENCE ANALYSIS IN MOLECULAR BIOLOGY, Von Heinje ed., Academic Press (1987), SEQUENCE ANALYSIS PRIMER, Gribskov & Devereux, eds. (Macmillan Stockton Press, 1991), and Carillo & Lipton, SIAM J. Applied Math. 48: 1073 (1988). Methods commonly employed to determine identity or similarity between two sequences include but are not limited to those disclosed in GUIDE TO HUGE COMPUTERS, Bishop, ed., (Academic Press, 1994) and Carillo & Lipton, supra. Methods to determine identity and similarity are codified in computer programs. Preferred computer program methods to determine identity and similarity between two sequences include but are not limited to the GCG program package (Devereux et al., Nucleic Acids Research 12: 387 (1984)), BLASTP, BLASTN, FASTA (Atschul et al., J. Mol. Biol. 215: 403 (1990)), and FASTDB (Brutlag et al., Comp. App. Biosci. 6: 237 (1990)).

Silencing: The unidirectional and unperturbed transcription of either genes or gene fragments from promoter to terminator can trigger post-transcriptional silencing of target genes. Initial expression cassettes for post-transcriptional gene silencing in plants comprised a single gene fragment positioned in either the antisense (McCormick et al., United States patent 6617496; Shewmaker et al., United States patent 5107065) or sense (van der Krol et al., Plant Cell 2:291-299, 1990) orientation between regulatory sequences for transcript initiation and termination. In Arabidopsis, recognition of the resulting transcripts by RNA-dependent RNA polymerase leads to the production of double-stranded (ds) RNA. Cleavage of this dsRNA by Dicer-like (Dcl) proteins such as Dcl4 yields 21-nucleotide (nt) small interfering RNAs (siRNAs). These siRNAs complex with proteins including members of the Argonaute (Ago) family to produce RNA-induced silencing complexes (RISCs). The RISCs then target homologous RNAs for endonucleolytic cleavage.

More effective silencing constructs contain both a sense and antisense component, producing RNA molecules that fold back into hairpin structures (Waterhouse et al., Proc Natl Acad Sci U S A 95: 13959-13964, 1998). The high dsRNA levels produced by expression of inverted repeat transgenes were hypothesized to promote the activity of multiple Dcls. Analyses of combinatorial Dcl knockouts in Arabidopsis supported this idea, and also identified Dcl4 as one of the proteins involved in RNA cleavage.

One component of conventional sense, antisense, and double-strand (ds) RNA-based gene silencing constructs is the transcriptional terminator. WO 2006/036739 shows that this regulatory element becomes obsolete when gene fragments are positioned between two oppositely oriented and functionally active promoters. The resulting convergent transcription triggers gene silencing that is at least as effective as unidirectional 'promoter-to-terminato' transcription. In addition to short variably-sized and non-polyadanylated RNAs, terminator-free cassette produced rare longer transcripts that reach into the flanking promoter. Replacement of gene fragments by promoter-derived sequences further increased the extent of gene silencing.

In a preferred embodiment of the present invention the desired polynucleotide for use in the methods of the present invention comprises a partial sequence of a target gene promoter or a partial sequence that shares sequence identity with a portion of a target gene promoter. Hence, a desired polynucleotide for use in the methods of the present invention contains a specific fragment of a particular target gene promoter of interest.

The desired polynucleotide may be operably linked to one or more functional promoters. Various constructs contemplated for use in the methods of the present invention include, but are not limited to (1) a construct where the desired polynucleotide comprises one or more promoter fragment sequences and is operably linked at both ends to functional 'driver' promoters. Those two functional promoters are arranged in a convergent orientation so that each strand of the desired polynucleotide is transcribed; (2) a construct where the desired polynucleotide is operably linked to one functional promoter at either its 5'-end or its 3'-end, and the desired polynucleotide is also operably linked at its non-promoter end by a functional terminator sequence; (3) a construct where the desired polynucleotide is operably linked to one functional promoter at either its 5'-end or its 3'-end, but where the desired polynucleotide is not operably linked to a terminator; or (4) a cassette, where the desired polynucleotide comprises one or more promoter fragment sequences but is not operably linked to any functional promoters or terminators.

Hence, a construct for use in the methods of the present invention may comprise two or more 'driver' promoters which flank one or more desired polynucleotides or which flank copies of a desired polynucleotide, such that both strands of the desired polynucleotide are transcribed. That is, one promoter may be oriented to initiate transcription of the 5'-end of a desired polynucleotide, while a second promoter may be operably oriented to initiate transcription from the 3'-end of the same desired polynucleotide. The oppositely-oriented promoters may flank multiple copies of the desired polynucleotide. Hence, the "copy number" may vary so that a construct may comprise 2, 3, 4, 5, 6, 7, 8, 9, 10, 15, 20, 30, 40, 50, 60, 70, 80, 90, or 100, or more than 100 copies, or any integer in-between, of a desired polynucleotide, which may be flanked by the 'driver' promoters that are oriented to induce convergent transcription.

If neither cassette comprises a terminator sequence, then such a construct, by virtue of the convergent transcription arrangement, may produce RNA transcripts that are of different lengths.

In this situation, therefore, there may exist subpopulations of partially or fully transcribed RNA transcripts that comprise partial or full-length sequences of the transcribed desired polynucleotide from the respective cassette. Alternatively, in the absence of a functional terminator, the transcription machinery may proceed past the end of a desired polynucleotide to produce a transcript that is longer than the length of the desired polynucleotide.

In a construct that comprises two copies of a desired polynucleotide, therefore, where one of the polynucleotides may or may not be oriented in the inverse complementary direction to the other, and where the polynucleotides are operably linked to promoters to induce convergent transcription, and there is no functional terminator in the construct, the transcription machinery that initiates from one desired polynucleotide may proceed to transcribe the other copy of the desired polynucleotide and vice versa. The multiple copies of the desired polynucleotide may be oriented in various permutations: in the case where two copies of the desired polynucleotide are present in the construct, the copies may, for example, both be oriented in same direction, in the reverse orientation to each other, or in the inverse complement orientation to each other, for example.

In an arrangement where one of the desired polynucleotides is oriented in the inverse complementary orientation to the other polynucleotide, an RNA transcript may be produced that comprises not only the "sense" sequence of the first polynucleotide but also the "antisense" sequence from the second polynucleotide. If the first and second polynucleotides comprise the same or substantially the same DNA sequences, then the single RNA transcript may comprise two regions that are complementary to one another and which may, therefore, anneal. Hence, the single RNA transcript that is so transcribed, may form a partial or full hairpin duplex structure.

On the other hand, if two copies of such a long transcript were produced, one from each promoter, then there will exist two RNA molecules, each of which would share regions of sequence complementarity with the other. Hence, the "sense" region of the first RNA transcript may anneal to the "antisense" region of the second RNA transcript and vice versa. In this arrangement, therefore, another RNA duplex may be formed which will consist of two separate RNA transcripts, as opposed to a hairpin duplex that forms from a single self-complementary RNA transcript.

Alternatively, two copies of the desired polynucleotide may be oriented in the same direction so that, in the case of transcription read-through, the long RNA transcript that is produced from one promoter may comprise, for instance, the sense sequence of the first copy of the desired polynucleotide and also the sense sequence of the second copy of the desired polynucleotide. The RNA transcript that is produced from the other convergently-oriented promoter, therefore, may comprise the antisense sequence of the second copy of the desired polynucleotide and also the antisense sequence of the first polynucleotide. Accordingly, it is likely that neither RNA transcript would contain regions of exact complementarity and, therefore, neither RNA transcript is likely to fold on itself to produce a hairpin structure. On the other hand the two individual RNA transcripts could hybridize and anneal to one another to form an RNA duplex.

Hence, in one aspect, the methods of the present invention may use a construct that lacks a terminator or lacks a terminator that is preceded by self-splicing ribozyme encoding DNA region, but which comprises a first promoter that is operably linked to the desired polynucleotide.

**Tissue:** any part of a plant that is used to produce a food. A tissue can be a tuber of a potato, a root of a sweet potato, or a seed of a maize plant

**Transcriptional terminators:** The expression DNA constructs used in the methods of the present invention typically have a transcriptional termination region at the opposite end from the transcription initiation regulatory region. The transcriptional termination region may be selected, for stability of the mRNA to enhance expression and/or for the addition of polyadenylation tails added to the gene transcription product. Translation of a nascent polypeptide undergoes termination when any of the three chain-termination codons enters the A site on the ribosome. Translation termination codons are UAA, UAG, and UGA.

In the methods of instant invention, transcription terminators are derived from either a gene or, more preferably, from a sequence that does not represent a gene but intergenic DNA. For example, the terminator sequence from the potato ubiquitin gene may be used and is depicted in SEQ TD NO: 5.

**Transfer DNA (T-DNA):** a transfer DNA is a DNA segment delineated by either T-DNA borders or P-DNA borders to create a T-DNA or P-DNA, respectively. A T-DNA is a genetic element that is well-known as an element capable of integrating a nucleotide sequence contained within its borders into another genome. In this respect, a T-DNA is flanked typically, by two "border" sequences. A desired polynucleotide for use in the methods of the present invention and a selectable marker may be positioned between the left border-like sequence and the right border-like sequence of a T-DNA. The desired polynucleotide and selectable marker contained within the T-DNA may be operably linked to a variety of different, plant-specific (i.e., native), or foreign nucleic acids, like promoter and terminator regulatory elements that facilitate its expression, i.e., transcription and/or translation of the DNA sequence encoded by the desired polynucleotide or selectable marker.

**Transformation of plant cells:** A process by which a nucleic acid is stably inserted into the genome of a plant cell. Transformation may occur under natural or artificial conditions using various methods well known in the art. Transformation may rely on any known method for the insertion of nucleic acid sequences into a prokaryotic or eukaryotic host cell, including Agrobacterium-mediated transformation protocols such as 'refined transformation' or 'precise breeding', viral infection, whiskers, electroporation, microinjection, polyethylene glycol-treatment, heat shock, lipofection and particle bombardment.

**Transgenic plant:** a transgenic plant is one that comprises at least one cell genome in which an exogenous nucleic acid has been stably integrated. As used herein, a transgenic plant is a plant that comprises only one genetically modified cell and cell genome, or is a plant that comprises some genetically modified cells, or is a plant in which all of the cells are genetically modified. A transgenic plant as described herein may be one that comprises expression of the desired polynucleotide, i.e., the exogenous nucleic acid, in only certain parts of the plant. Thus, a transgenic plant may contain only genetically modified cells in certain parts of its structure.

**Variant:** a "variant," as used herein, is understood to mean a nucleotide or amino acid sequence that deviates from the standard, or given, nucleotide or amino acid sequence of a particular gene or protein. The terms, "isoform," "isotope," and "analog" also refer to "variant" forms of a nucleotide or an amino acid sequence. An amino acid sequence that is altered by the addition, removal or substitution of one or more amino acids, or a change in nucleotide sequence, may be considered a "variant' sequence. The variant may have "conservative" changes, wherein a substituted amino acid has similar structural or chemical properties, e.g., replacement of leucine with isoleucine. A variant may have "nonconservative" changes, e.g., replacement of a glycine with a tryptophan. Analogous minor variations may also include amino acid deletions or insertions, or both. Guidance in determining which amino acid residues may be substituted, inserted, or deleted may be found using computer programs well known in the art such as Vector NTI Suite (InforMax, MD) software. "Variant" may also refer to a "shuffled gene" such as those described in Maxygen-assigned patents.

It is understood that the present invention is not limited to the particular methodology, protocols, vectors, and reagents, etc., described herein, as these may vary. It is also to be understood that the terminology used herein is used for the purpose of describing particular embodiments only, and is not intended to limit the scope of the present invention. It must be noted that as used herein and in the appended claims, the singular forms "a," "an," and "the" include plural reference unless the context clearly dictates otherwise. Thus, for example, a reference to "a gene" is a reference to one or more genes and includes equivalents thereof known to those skilled in the art and so forth. Indeed, one skilled in the art can use the methods described herein to express any native gene (known presently or subsequently) in plant host systems.

### Polynucleotide Sequences

The methods of the present invention relates to an isolated nucleic molecule comprising a polynucleotide having a sequence selected from any of the polynucleotide sequences of SEQ ID NOs: 1, 2, 14, or 31-33. The methods of the present invention also relate to functional fragments of the polynucleotide sequences of SEQ ID NOs: 1, 2, 9, 14, or 31-33. The invention further relates to complementary nucleic acids, or fragments thereof to any of the polynucleotide sequences of SEQ ID NOs: 1, 2, 9, 14, or 31-33. as well as a nucleic acid, comprising at least 15 contiguous bases, which hybridizes to any of the polynucleotide sequences of SEQ ID NOs: 1, 2, 9, 14, or 31-32.

By "isolated" nucleic acid molecule(8) is intended a nucleic add molecule, DNA or RNA, which has been removed from its native environment. For example, recombinant DNA molecules contained in a DNA construct are considered isolated for the purposes of the present invention. Further examples of isolated DNA molecules include recombinant DNA molecules maintained in heterologous host cells or purified (partially or substantially) DNA molecules in solution. Isolated RNA molecules include in vitro RNA transcripts of the DNA molecules for use in the methods of the present invention. Isolated nucleic acid molecules, as described herein further include such molecules produced synthetically.

Nucleic acid molecules for use in the methods of the present invention may be in the form of RNA, such as mRNA, or in the form of DNA, including, for instance, cDNA and genomic DNA obtained by cloning or produced synthetically. The DNA or RNA may be double-stranded or single-stranded. Single-stranded DNA may be the coding strand, also known as the sense strand, or it may be the non-coding strand, also referred to as the anti-sense strand.

Unless otherwise indicated, all nucleotide sequences determined by sequencing a DNA molecule herein were determined using an automated DNA sequencer (such as the Model 373 from Applied Biosystems, Inc.). Therefore, as is known in the art for any DNA sequence determined by this automated approach, any nucleotide sequence determined herein may contain some errors. Nucleotide sequences determined by automation are typically at least about 95% identical, more typically at least about 96% to at least about 99.9% identical to the actual nucleotide sequence of the sequenced DNA molecule. The actual sequence can be more precisely determined by other approaches including manual DNA sequencing methods well known in the art. As is also known in the art, a single insertion or deletion in a determined nucleotide sequence compared to the actual sequence will cause a frame shift in translation of the nucleotide sequence such that the predicted amino acid sequence encoded by a determined nucleotide sequence may be completely different from the amino acid sequence actually encoded by the sequenced DNA molecule, beginning at the point of such an insertion or deletion.

Each "nucleotide sequence" set forth herein is presented as a sequence of deoxyribonucleotides (abbreviated A, G. C and T). However, by "nucleotide sequence" of a nucleic acid molecule or polynucleotide is intended, for a DNA molecule or polynucleotide, a sequence of deoxyribonucleotides, and for an RNA molecule or polynucleotide, the corresponding sequence of ribonucleotides (A, G, C and U) where each thymidine deoxynucleotide (T) in the specified deoxynucleotide sequence in is replaced by the ribonucleotide uridine (U).

The methods of the present invention may also use fragments of the isolated nucleic acid molecules described herein. Preferably, DNA fragments comprise at least 15 nucleotides, and more preferably at least 20 nucleotides, still more preferably at least 30 nucleotides in length.

The methods of the present application may use nucleic acid molecules which are at least 90%, 95%, 96%, 97%, 98%, 99% or 100% identical to a nucleic acid sequence described in SEQ ID NOs: 1, 2. Preferred, however, are nucleic acid molecules which are at least 95%, 96%, 97%, 98%, 99% or 100% identical to the nucleic add sequence shown in any of SEQ ID NOs: 1, 2. Differences between two nucleic acid sequences may occur at the 5' or 3' terminal positions of the reference nucleotide sequence or anywhere between those terminal positions, interspersed either individually among nucleotides in the reference sequence or in one or more contiguous groups within the reference sequence.

As a practical matter, whether any particular nucleic acid molecule is at least 95%, 96%, 97%, 98% or 99% identical to a reference nucleotide sequence refers to a comparison made between two molecules using standard algorithms well known in the art and can be determined conventionally using publicly available computer programs such as the BLASTN algorithm. See Altschul et al., Nucleic Acids Res. 25:3389-3402 (1997).

### Sequence Analysis

Methods of alignment of sequences for comparison are well-known in the art. Optimal alignment of sequences for comparison may be conducted by the local homology algorithm of Smith and Waterman, Adv. Appl. Math. 2: 482 (1981); by the homology alignment algorithm of Needleman and Wunsch, J. Mol. Biol. 48: 443 (1970); by the search for similarity method of Pearson and Lipman, Proc. Natl. Acad. Sci. 85: 2444 (1988); by computerized implementations of these algorithms, including, but not limited to: CLUSTAL in the PC/Gene program by Intelligenetics, Mountain View, California; GAP, BESTFIT, BLAST, FASTA, and TFASTA in the Wisconsin Genetics Software Package, Genetics Computer Group (GCG), 575 Science Dr., Madison, Wisconsin, USA; the CLUSTAL program is well described by Higgins and Sharp, Gene 73: 237 244 (1988); Higgins and Sharp, CABIOS 5: 151 153 (1989); Corpet, et al., Nucleic Acids Research 16: 10881-90 (1988); Huang, et al., Computer Applications in the Biosciences 8: 155-65 (1992), and Pearson, et al., Methods in Molecular Biology 24: 307-331 (1994).

The BLAST family of programs which can be used for database similarity searches includes: BLASTN for nucleotide query sequences against nucleotide database sequences; BLASTX for nucleotide query sequences against protein database sequences; BLASTP for protein query sequences against protein database sequences; TBLASTN for protein query sequences against nucleotide database sequences; and TBLASTX for nucleotide query sequences against nucleotide database sequences. See, Current Protocols in Molecular Biology, Chapter 19, Ausubel, et al., Eds., Greene Publishing and Wiley-Interscience, New York (1995); Altschul et al., J. Mol. Biol., 215:403-410 (1990); and, Altschul et al., Nucleic Acids Res. 25:3389-3402 (1997).

Software for performing BLAST analyses is publicly available, e.g., through the National Center for Biotechnology Information (http://www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold. These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, a cutoff of 100, M=5, N=-4, and a comparison of both strands. For amino acid sequences, the BLASTP program uses as defaults a wordlength (W) of 3, an expectation (E) of 10, and the BLOSUM62 scoring matrix (see Henikoff & Henikoff (1989) Proc. Natl. Acad. Sci. USA 89:10915).

In addition to calculating percent sequence identity, the BLAST algorithm also performs a statistical analysis of the similarity between two sequences (see, e.g., Karlin & Altschul, Proc. Nat'l. Acad. Sci. USA 90:5873-5877 (1993)). One measure of similarity provided by the BLAST algorithm is the smallest sum probability (P(N)), which provides an indication of the probability by which a match between two nucleotide or amino acid sequences would occur by chance.

Multiple alignment of the sequences can be performed using the CLUSTAL method of alignment (Higgins and Sharp (1989) CABIOS. 5:151 153) with the default parameters (GAP PENALTY=10, GAP LENGTH PENALTY=10). Default parameters for pairwise alignments using the CLUSTAL method are KTUPLE 1, GAP PENALTY=3, WINDOW=5 and DIAGONALS SAVED=5.

The following running parameters are preferred for determination of alignments and similarities using BLASTN that contribute to the E values and percentage identity for polynucleotide sequences: Unix running command: blastall -p blastn -d embldb -e 10 -G0 -E0 -r 1 -v 30 -b 30 -i queryseq -o results; the parameters are: -p Program Name [String]; -d Database [String]; -e Expectation value (E) [Real]; -G Cost to open a gap (zero invokes default behavior) [Integer]; -E Cost to extend a gap (zero invokes default behavior) [Integer]; -r Reward for a nucleotide match (blastn only) [Integer]; -v Number of one-line descriptions (V) [Integer]; -b Number of alignments to show (B) [Integer]; -i Query File [File In]; and -o BLAST report Output File [File Out] Optional.

The "hits" to one or more database sequences by a queried sequence produced by BLASTN, FASTA, BLASTP or a similar algorithm, align and identify similar portions of sequences. The hits are arranged in order of the degree of similarity and the length of sequence overlap. Hits to a database sequence generally represent an overlap over only a fraction of the sequence length of the queried sequence.

The BLASTN, FASTA and BLASTP algorithms also produce "Expect" values for alignments. The Expect value (E) indicates the number of hits one can "expect" to see over a certain number of contiguous sequences by chance when searching a database of a certain size. The Expect value is used as a significance threshold for determining whether the hit to a database, such as the preferred EMBL database, indicates true similarity. For example, an E value of 0.1 assigned to a polynucleotide hit is interpreted as meaning that in a database of the size of the EMBL database, one might expect to see 0.1 matches over the aligned portion of the sequence with a similar score simply by chance. By this criterion, the aligned and matched portions of the polynucleotide sequences then have a probability of 90% of being the same. For sequences having an E value of 0.01 or less over aligned and matched portions, the probability of finding a match by chance in the EMBL database is 1% or less using the BLASTN or FASTA algorithm.

According to one embodiment "variant" polynucleotides, with reference to each of the polynucleotides for use in the methods of the present invention, preferably comprise sequences having the same number or fewer nucleic acids than each of the polynucleotides for use in the methods of the present invention and producing an B value of 0.01 or less when compared to the polynucleotide for use in the methods of the present invention. That is, a variant polynucleotide is any sequence that has at least a 99% probability of being the same as the polynucleotide of the present invention, measured as having an E value of 0.01 or less using the BLASTN, FASTA, or BLASTP algorithms set at parameters described above.

Alternatively, variant polynucleotides for use in the methods of the present invention hybridize to the polynucleotide sequences recited in SEQ ID NOs: 1, 2, 9, 14, or 31-33 or complements, reverse sequences, or reverse complements of those sequences, under stringent conditions.

The methods of the present invention also relate to polynucleotides that differ from the disclosed sequences but that, as a consequence of the degeneracy of the genetic code, encode a polypeptide which is the same as that encoded by a polynucleotide for use in the methods of the present invention. Thus, polynucleotides comprising sequences that differ from the polynucleotide sequences recited in SEQ ID NOs: 1, 2, 9; 14, or 31-33 or complements, reverse sequences, or reverse complements thereof as a result of conservative substitutions are contemplated by and may be used in the methods of the present invention. Additionally, polynucleotides comprising sequences that differ from the polynucleotide sequences recited in SEQ ID NOs: 1, 2, 9, 14, or 31-33 or complements, reverse complements or reverse sequences thereof, as a result of deletions and/or insertions totaling less than 10% of the total sequence length are also contemplated by and may be used in the methods of the present invention.

In addition to having a specified percentage identity to polynucleotide sequence, variant polynucleotides preferably have additional structure and/or functional features in common with the polynucleotide. In addition to sharing a high degree of similarity in their primary structure to polynucleotides for use in the methods of the present invention, polynucleotides having a specified degree of identity to, or capable of hybridizing to a polynucleotide preferably have at least one of the following features: (i) they contain an open reading frame or partial open reading frame encoding a polypeptide having substantially the same functional properties as the polypeptide for use in the methods of the present invention; or (ii) they have domains in common.

### Source of elements and DNA sequences

Any or all of the elements and DNA sequences that are described herein may be endogenous to one or more plant genomes. Accordingly, in one particular embodiment of the present invention, all of the elements and DNA sequences, which are selected for the ultimate transfer cassette are endogenous to, or native to, the gnome of the plant that is to be transformed. For instance, all of the sequences may come from a potato genome. Alternatively, one or more of the elements or DNA sequences may be endogenous to a plant genome that is not the same as the species of the plant to be transformed, but which function in any event in the host plant cell. Such plants include potato, tomato, and alfalfa plants. The methods of the present invention also relate to the use of one or more genetic elements from a plant that is interfertile with the plant that is to be transformed.

In this regard, a "plant" for use in the methods of the present invention includes, but is not limited to potato, tomato, avocado, alfalfa, sugarbeet, cassava, sweet potato, soybean, pea, bean, maize, wheat, rice, barley, and sorghum. Thus, a plant may be a monocot or a dicot. "Plant" and "plant material," also encompasses plant cells, seed, plant progeny, propagule whether generated sexually or asexually, and descendents of any of these, such as cuttings or seed. "Plant material" may refer to plant cells, cell suspension cultures, callus, embryos, meristematic regions, callus tissue, leaves, roots, shoots, gametophytes, sporophytes, pollen, seeds, germinating seedlings, and microspores. Plants may be at various stages of maturity and may be grown in liquid or solid culture, or in soil or suitable media in pots, greenhouses or fields. Expression of an introduced leader, trailer or gene sequences in plants may be transient or permanent

In this respect, a plant-derived transfer-DNA ("P-DNA") border sequence of the present invention is not identical in nucleotide sequence to any known bacterium-derived T-DNA border sequence, but it functions for essentially the same purpose. That is, the P-DNA can be used to transfer and integrate one polynucleotide into another. A P-DNA can be inserted into a tumor-inducing plasmid, such as a Ti-plasmid from Agrobacterum in place of a conventional T-DNA, and maintained in a bacterium strain, just like conventional transformation plasmids. The P-DNA can be manipulated so as to contain a desired polynucleotide, which is destined for integration into a plant genome via bacteria-mediated plant transformation. See Rommens et al. in WO2003/069980, US-2003-0221213, US-2004-0107455, and WO2005/004585.

Thus, a P-DNA border sequence is different by 1, 2, 3, 4, 5, 6, 7, 8, 9, 10, 11, 12, 13, 14, 15,16, 17, 18, 19, 20, or more nucleotides from a known T-DNA border sequence from an Agrobacterium species, such as Agrobacterium tumefaciens or Agrobacterium rhizogenes.

A P-DNA border sequence is not greater than 99%, 98%, 97%, 96%, 95%, 94%, 93%, 92%, 91%, 90%, 89%, 88%, 87%, 86%, 85%, 84%, 83%, 82%, 81%, 80%, 79%, 78%, 77%, 76%, 75%, 74%, 73%, 72%, 71%, 70%, 69%, 68%, 67%, 66%, 65%, 64%, 63%, 62%, 61%, 60%, 59%, 58%, 57%, 56%, 55%, 54%, 53%, 52%, 51% or 50% similar in nucleotide sequence to an Agrobacterium T-DNA border sequence.

Methods were developed to identify and isolate transfer DNAs from plants, particularly potato and wheat, and made use of the border motif consensus described in US-2004-0107455.

In this respect, a plant-derived DNA for use in the methods of the present invention, such as any of the sequences, cleavage sites, regions, or elements disclosed herein is functional if it promotes the transfer. and integration of a polynucleotide to which it is linked into another nucleic acid molecule, such as into a plant chromosome, at a transformation frequency of about 99%, about 98%, about 97%, about 96%, about 95%, about 94%, about 93%, about 92%, about 91%, about 90%, about 89%, about 88%, about 87%, about 86%, about 85%, about 84%, about 83%, about 82%, about 81%, about 80%, about 79%, about 78%, about 77%, about 76%, about 75%, about 74%, about 73%, about 72%, about 71 %, about 70%, about 69%, about 68%, about 67%, about 66%, about 65%, about 64%, about 63%, about 62%, about 61%, about 60%, about 59%, about 58%, about 57%, about 56%, about 55%, about 54%, about 53%, about 52%, about 51%, about 50%, about 49%, about 48%, about 47%, about 46%, about 45%, about 44%, about 43%, about 42%, about 41%, about 40%, about 39%, about 38%, about 37%, about 36%, about 35%, about 34%, about 33%, about 32%, about 31%, about 30%, about 29%, about 28%, about 27%, about 26%, about 25%, about 24%, about 23%, about 22%, about 21%, about 20%, about 15%, or about 5% or at least about 1%,

Any of such transformation-related sequences and elements can be modified or mutated to change Transformation efficiency. Other polynucleotide sequences may be added to a transformation sequence of the present invention. For instance, it may be modified to possess 5'- and 3'- multiple cloning sites, or additional restriction sites. The sequence of a cleavage site as disclosed herein, for example, may be modified to increase the likelihood that backbone DNA from the accompanying vector is not integrated into a plant genome.

Any desired polynucleotide may be inserted between any cleavage or border sequences described herein. For example, a desired polynucleotide may be a wild-type or modified gene that is native to a plant species, or it may be a gene from a non-plant genome. For instance, when transforming a potato plant, an expression cassette can be made that comprises a potato-specific promoter that is operably linked to a desired potato gene or fragment thereof and a potato-specific terminator. The expression cassette may contain additional potato genetic elements such as a signal peptide sequence fused in frame to the 5'-end of the gene, and a potato transcriptional enhancer. The methods of the present invention are not limited to such an arrangement and a transformation cassette maybe constructed such that the desired polynucleotide, while operably linked to a promoter, is not operably linked to a terminator sequence.

When a transformation-related sequence or element, such as those described herein, are identified and isolated from a plant, and if that sequence or element is subsequently used to transform a plant of the same species, that sequence or element can be described as "native" to the plant genome.

Thus, a "native" genetic element refers to a nucleic acid that naturally exists in, originates from, or belongs to the genome of a plant that is to be transformed. In the same vein, the term "endogenous" also can be used to identify a particular nucleic acid, e.g., DNA or RNA, or a protein as "native" to a plant. Endogenous means an element that originates within the organism. Thus, any nucleic acid, gene, polynucleotide, DNA, RNA, mRNA, or cDNA molecule that is isolated either from the genome of a plant or plant species that is to be transformed or is isolated from a plant or species that is sexually compatible or interfertile with the plant species that is to be transformed, is "native" to, i.e., indigenous to, the plant species. In other words, a native genetic element represents all genetic material that is accessible to plant breeders for the improvement of plants through classical plant breeding. Any variants of a native nucleic acid also are considered "native" in accordance with the present invention. In this respect, a "native" nucleic acid may also be isolated from a plant or sexually compatible species thereof and modified or mutated so that the resultant variant is greater than or equal to 99%, 98%, 97%, 96%, 95%, 94%, 93%, 92%, 91%, 90%, 89%, 88%, 87%, 86%, 85%, 84%, 83%, 82%, 81%, 80%, 79%, 78%, 77%, 76%, 75%, 74%, 73%, 72%, 71%, 70%, 69%, 68%, 67%, 66%, 65%, 64%, 63%, 62%, 61 %, or 60% similar in nucleotide sequence to the unmodified, native nucleic acid isolated from a plant. A native nucleic acid variant may also be less than about 60%, less than about 55%, or less than about 50% similar in nucleotide sequence.

A "native" nucleic acid isolated from a plant may also encode a variant of the naturally occurring protein product transcribed and translated from that nucleic acid. Thus, a native nucleic acid may encode a protein that is greater than or equal to 99%, 98%, 97%, 96%, 95%, 94%, 93%, 92%, 91%, 90%, 89%, 88%, 87%, 86%, 85%, 84%, 83%, 82%, 81%, 80%, 79%, 78%, 77%, 76%, 75%, 74%, 73%, 72%, 71%, 70%, 69%, 68%, 67%, 66%, 65%, 64%, 63%, 62%, 61%, or 60% similar in amino acid sequence to the unmodified, native protein expressed in the plant from which the nucleic acid was isolated.

### Promoters

The polynucleotides for use in the methods of the present invention can be used for specifically directing the expression of polypeptides or proteins in the tissues of plants. The nucleic acids for use in the methods of the present invention can also be used for specifically directing the expression of antisense RNA, or RNA involved in RNA interference (RNAi) such as small interfering RNA (siRNA), in the tissues of plants, which can be useful for inhibiting or completely blocking the expression of targeted genes. As used herein, "coding product" is intended to mean the ultimate product of the nucleic acid that is operably linked to the promoters. For example, a protein or polypeptide is a coding product, as well as antisense RNA or siRNA which is the ultimate product of the nucleic acid coding for the antisense RNA. The coding product may also be non-translated mRNA. The terms polypeptide and protein are used interchangeably herein. As used herein, promoter is intended to mean a nucleic acid, preferably DNA that binds RNA polymerase and/or other transcription regulatory elements. As with any promoter, the promoters of the current invention will facilitate or control the transcription of DNA or RNA to generate an mRNA molecule from a nucleic acid molecule that is operably linked to the promoter. The RNA may code for a protein or polypeptide or may code for an RNA interfering, or antisense molecule. As used herein. "operably linked" is meant to refer to the chemical fusion, ligation, or synthesis of DNA such that a promoter-nucleic acid sequence combination is formed in a proper orientation for the nucleic acid sequence to be transcribed into an RNA segment. The promoters for use in the methods of the current invention may also contain some or all of the 5' untranslated region (5'UTR) of the resulting mRNA transcript. On the other hand, the promoters for use in the methods of the current invention do not necessarily need to possess any of the 5' UTR.

A promoter, as used herein, may also include regulatory elements. Conversely, a regulator element may also be separate from a promoter. Regulatory elements confer a number of important characteristics upon a promoter region. Some elements bind transcription factors that enhance the rate of transcription of the operably linked nucleic acid. Other elements bind repressors that inhibit transcription activity. The effect of transcription factors on promoter activity may determine whether the promoter activity is high or low, i.e. whether the promoter is "strong" or "weak."

In another embodiment, a constitutive promoter may be used for expressing the polynucleotide sequences.

In another embodiment, a variety of inducible plant gene promoters can be used for expressing the polynucleotide sequences. Inducible promoters regulate gene expression in response to environmental, hormonal, or chemical signals. Examples of hormone inducible promoters include auxin-inducible promoters (Baumann et al. Plant Cell 11:323-334(1999)), cytokinin-inducible promoter (Guevara-Garcia Plant Mol. BioL 38:743-753(1998)), and gibberellin-responsive promoters (Shi et al. Plant Mol. BioL 38:1053-1060(1998)). Additionally, promoters responsive to heat, light, wounding, pathogen resistance, and chemicals such as methyl jasmonate or salicylic acid, may be used for expressing the inventive polynucleotide sequences.

In one embodiment, the promoter is a granule bound starch synthase promoter, a potato ADP-glucose pyrophosphorylase gene promoter, or a flavonoid 3'-monooxygenase gene promoter. In another embodiment, the promoter is a seed-specific promoter.

The methods of the present invention also relate to polynucleotides that differ from the disclosed sequences but that, as a consequence of the degeneracy of the genetic code, encode a polypeptide which is the same as that encoded by a polynucleotide of the present invention. Thus, polynucleotides comprising sequences that differ from the polynucleotide sequences recited in SEQ ID NOs: 1, 2, 9, 14, or 31-33 or complements, reverse sequences, or reverse complements thereof, as a result of conservative substitutions are contemplated for use in the methods of the present invention. Additionally, polynucleotides comprising sequences that differ from the polynucleotide sequences recited in SEQ ID NOs: 1, 2, 9, 14, or 31-32 or complements, reverse complements or reverse sequences thereof, as a result of deletions and/or insertions totaling less than 10% of the total sequence length are also contemplated for use in the methods of present invention.

to addition to having a specified percentage identity to a polynucleotide sequence, variant polynucleotides preferably have additional structure and/or functional features in common with the inventive polynucleotide. In addition to sharing a high degree of similarity in their primary structure to polynucleotides for use in the methods of the present invention, polynucleotides having a specified degree of identity to, or capable of hybridizing to a polynucleotide preferably have at least one of the following features: (i) they contain an open reading frame or partial open reading frame encoding a polypeptide having substantially the same functional properties as the polypeptide encoded by the polynucleotide; or (ii) they have domains in common.

### Source of elements and DNA sequences

Any or all of the elements and DNA sequences that are described herein may be endogenous to one or more plant genomes. Accordingly, in one particular embodiment of the present invention, all of the elements and DNA sequences, which are selected for the ultimate transfer cassette are endogenous to, or native to, the genome of the plant that is to be transformed. For instance, all of the sequences may come from a potato genome. Alternatively, one or more of the elements or DNA sequences may be endogenous to a plant genome that is not the same as the species of the plant to be transformed, but which function in any event in the host plant cell. Such plants include potato, tomato, and alfalfa plants. The present invention also encompasses use of one or more genetic elements from a plant that is interfertile with the plant that is to be transformed.

In this regard, a "plant" for use in the methods of the present invention includes, but is not limited to potato, tomato, alfalfa, sugarbeet, cassava, sweet potato, soybean, pea, bean, maize, wheat, rice, barley, and sorghum. "Plant" and "plant material," also encompasses plant cells, seed, plant progeny, propagule whether generated sexually or sexually, and descendents of any of these, such as cuttings or seed. "Plant material" may refer to plant cells, cell suspension cultures, callus, embryos, meristematic regions, callus tissue, leaves, roots, shoots, gametophytes, sporophytes, pollen, seeds, terminating seedlings, and microspores. Plants may be at various stages of maturity and may be grown in liquid or solid culture, or in soil or suitable media in pots, greenhouses or fields. Expression of an introduced leader, trailer or gene sequences in plants may be transient or permanent.

### Nucleic Acid Constructs

The methods of present invention relate to constructs comprising the isolated nucleic acid molecules and polypeptide sequences described herein. In one embodiment, the DNA constructs for use in the methods of the present invention are Ti-plasmids derived from A. tumefaciens.

In developing the nucleic acid constructs, the various components of the construct or fragments thereof will normally be inserted into a convenient cloning vector, e.g, a plasmid that is capable of replication in a bacterial host, e.g., E. coli. Numerous vectors exist that have been described in the literature, many of which are commercially available. After each cloning, the cloning vector with the desired insert may be isolated and subjected to further manipulation, such as restriction digestion, insertion of new fragments or nucleotides, ligation, deletion, mutation, resection, etc. to tailor the components of the desired sequence. Once the constructs has been completed, it may then be transferred to an appropriate vector for further manipulation in accordance with the manner of transformation of the host cell.

A recombinant DNA molecule for use in the methods of the invention may typically include a selectable marker so that transformed cells can be easily identified and selected from non-transformed cells. Examples of such markers include, but are not limited to, a neomycin phosphotransferase (nptII) gene (Potrykus et al, Mol. Gen. Genet. 199:183-188 (1985)), which confers kanamycin resistance. Cells expressing the nptII gene can be selected using an appropriate antibiotic such as kanamycin or G418. Other commonly used selectable markers include the bar gene, which confers bialaphos resistance; a mutant EPSP synthase gene (Hinchee et al., Bio/Technology 6:915-922 (1988)), which confers glyphosate resistance; and a mutant acetolactate synthase gene (ALS), which confers imidazolinone or sulphonylurea resistance (European Patent Application 154,204,1985).

Additionally, vectors may include an origin of replication (replicons) for a particular host cell. Various prokaryotic replicons are known to those skilled in the art, and function to direct autonomous replication and maintenance of a recombinant molecule in a prokaryotic host cell.

The methods of the invention also relate to host cells which comprise the DNA constructs described herein. As used herein, a host cell refers to the cell in which the coding product is ultimately expressed. Accordingly, a host cell can be an individual cell, a cell culture or cells as part of an organism. The host cell can also be a portion of an embryo, endosperm, sperm or egg cell, or a fertilized egg.

Accordingly, the methods of the present invention also relate to plants or plant cells, comprising the DNA constructs described herein. Preferably the plants are angiosperms or gymnosperms. The expression construct may be used to transform a variety of plants, both monocotyledonous (e.g. wheat, turf grass, maize, rice, oat, wheat, barley, sorghum, orchid, iris, lily, onion, banana, sugarcane, and pahn), dicotyledonous (e.g., Arabidopsis, potato, tobacco, tomato, avocado, pepper, sugarbeet, broccoli, cassava, sweet potato, cotton, poinsettia, legumes, alfalfa, soybean, pea, bean, cucumber, grape, brassica, carrot, strawberry, lettuce, oak, maple, walnut, rose, mint, squash, daisy, and cactus, oaks, eucalyptus, maple), and Gymnosperms (e.g., Scots pine; see Aronen. Finnish Forest Res. Papers, Vol. 595, 1996), white spruce (Bills et al., Biotechnology 11:84-89, 1993), and larch (Huang et al., In Vitro Cell 27:201-207, 1991).

### Plant Transformation and Regeneration

The present polynucleotides and polypeptides may be introduced into a host plant cell by standard procedures known in the art for introducing recombinant sequences into a target host cell. Such procedures include, but are not limited to, transfection, infection, transformation, natural uptake, electroporation, biolistics and Agrobacterium. Methods for introducing foreign genes into plants are known in the art and can be used to insert a construct of the invention into a plant host, including, biological and physical plant transformation protocols. See, for example, Mild et al., 1993, "Procedure for Introducing Foreign DNA into Plants", In: Methods in Plant Molecular Biology and Biotechnology, Glick and Thompson, eds., CRC Press, Inc., Boca Raton, pages 67-88. The methods chosen vary with the host plant, and include chemical transfection methods such as calcium phosphate, microorganism-mediated gene transfer such as Agrobacterium (Horsch et al, Science 227:1229-31, 1985), electroporation, micro-injection, and biolistic bombardment. Preferred transformation methods include precise breeding (see: United States patent applications 2003/0221213 Al, 200410107455 Al, and 2005/0229267 A1) and refined transformation (see United States patent application 2005/0034188 A1).

Accordingly, the present invention also relates to plants or plant cells, comprising the polynucleotides or polypeptides described herein. In one embodiment, the plants are angiosperms or gymnosperms. Beyond the ordinary meaning of plant, the term "plants" is also intended to mean the fruit, seeds, flower, strobilus etc. of the plant The plant used in the method of the current invention may be a direct transfectant, meaning that the vector was introduced directly into the plant, such as through Agrobacterium, or the plant may be the progeny of a transfected plant The progeny may also be obtained by asexual reproduction of a transfected plant. The second or subsequent generation plant may or may not be produced by sexual reproduction, i.e., fertilization. Furthermore, the plant can be a gametophyte (haploid stage) or a sporophyte (diploid stage).

In this regard, the present invention contemplates transforming a plant with one or more transformation elements that genetically originate from a plant. The methods of the present invention encompasses an "all-native" approach to transformation, whereby only transformation elements that are native to plants are ultimately integrated into a desired plant via transformation. In this respect, the methods of the present invention encompass transforming a particular plant species with only genetic transformation elements that are native to that plant species. The native approach may also mean that a particular transformation element is isolated from the same plant that is to be transformed, the same plant species, ot from a plant that is sexually interfertile with the plant to be transformed.

On the other hand, the plant that is to be transformed, may be transformed with a transformation cassette that contains one or more genetic elements and sequences that originate from a plant of a different species. It may be desirable to use, for instance, a cleavage site, that is native to a potato genome in a transformation cassette or plasmid for transforming a tomato or pepper plant.

The methods of the present invention are not limited, however, to native or all-native approach. A transformation cassette or plasmid for use in the methods of the present invention can also comprise sequences and elements from other organism, such as from a bacterial species.

The following examples are set forth as representative of specific and preferred embodiments of the present invention. These examples are not to be construed as limiting the scope of the invention in any manner.

### EXAMPLES

### Example 1

### Down-regulated expression of asparagine synthetase genes

This example demonstrates that the down-regulated expression of a gene involved in asparagine biosynthesis in the starchy tissues of a crop lowers the amount of asparagine in these starchy tissues and, consequently, lowers the amount of acrylamide in a food obtained from heating these starchy tissues.

The sequence of the potato asparagine synthetase-1 (*Ast1*) gene is shown in SEQ ID NO.: 1. The partial sequence of the potato asparagine synthetase-2 (*Ast2*) gene is shown in SEQ ID NO.: 2.

Fragments of these genes, shown in SEQ ID NO.: 3 and 4, were linked to create SEQ ID NO.: 5. Two copies of the resulting DNA segment were inserted, as inverted repeat and separated by the spacer shown in SEQ ID NO.: 6, between the convergently-oriented promoters of the ADP-glucose pyrophosphorylase (*Agp*) and granule-bound starch synthase (*Gbss*) genes (SEQ ID NO.: 7 and 8, respectively).

The resulting silencing construct was inserted between the borders of a T-DNA that already contained an expression cassette for the neomycin phosphotransferase (*nptII*) selectable marker gene.

A binary vector carrying this T-DNA, designated pSIM1148 **(****Figure 1A****),** was introduced into Agrobacterium LBA4404 as follows. Competent LB4404 cells (50 µL) are incubated for 5 min on ice in the presence of 1 µg of vector DNA, frozen for about 15 s in liquid nitrogen, and incubated at 37°C for 5 min. After adding 1 mL of liquid broth, the treated cells are grown for 3 h at 28°C and plated on liquid broth/agar containing streptomycin (100 mg/L) and kanamycin (100 mg/L). The vector DNAs are then isolated from overnight cultures of individual LBA4404 colonies and examined by restriction analysis to confirm the presence of intact plasmid DNA.

Ten-fold dilutions of overnight-grown *Agrobacterium* cultures were grown for 5-6 hours, precipitated for 15 minutes at 2,800 RPM, washed with MS liquid medium (Phytotechnology) supplemented with sucrose (3%, pH 5.7), and resuspended in the same medium to 0.2 OD/600nm. The resuspended cells were mixed and used to infect 0.4-0.6 mm internodal segments of the potato variety "Ranger Russet".

Infected stems were incubated for two days on co-culture medium (1/10 MS salts, 3% sucrose, pH 5.7) containing 6 g/L agar at 22°C in a Percival growth chamber (16 hrs light) and subsequently transferred to callus induction medium (CIM, MS medium supplemented with 3% sucrose 3, 2.5 mg/L of zeatin riboside, 0.1 mg/L of naphthalene acetic acid, and 6g/L of agar) containing timentin (150 mg/L) and kanamycin (100 mg/L). After one month of culture on CIM, explants were transferred to shoot induction medium (SIM, MS medium supplemented with 3% sucrose, 2.5 mg/L of zeatin riboside, 0.3 mg/L of giberellic acid GA3, and 6g/L of agar) containing timentin and kanamycin (150 and 100 mg/L respectively) until shoots arose. Shoots arising at the end of regeneration period were transferred to MS medium with 3% sucrose, 6 g/L of agar and timentin (150mg/L). Transgenic plants were transferred to soil and placed in a greenhouse.

After three months, tubers were harvested and analyzed for asparagine levels according to the Official Methods of Analysis of AOAC INTERNATIONAL (2002), 17th Edition, AOAC INTERNATIONAL, Gaithersburg, MD, USA Official Method 982.30. This analysis demonstrated that 17 of 26 pSIM1148 plants contained only about 25% to 50% of the asparagine that was present in control plants **(Table 1)**

Tubers from some of the low asparagine plants were cut, blanched, par-fried, and finish-fried to produce French fries. These French fries were ground to a fine powder in liquid nitrogen that was shipped on dry ice to Covance laboratories. At Covance, acrylamide levels were determined by performing liquid chromatography/mass spectrometry/mass spectrometry (LC/MS/MS) (United States Food and Drug Administration, Center for Food Safety and Applied Nutrition Office of Plant & Dairy Foods and Beverages, "Detection and Quantitation of Acrylamide in Foods", 2002). **Table 2** shows that fries from the low asparagine pSIM48 plants accumulated less than about a third of the acrylamide that is produced in control fries.

As an alternative to using the Agp and Gbss promoters as regulatory elements, it is also possible to employ two Gbss promoters. A construct containing the inverted repeat that comprises the *Ast1* and *Ast2* gene fragments inserted between two Gbss promoters was introduced between T-DNA borders to produce the binary vector pSIM1151 **(****Figure 1B****)**. This vector was used to produce transgenic potato lines in a similar manner as described for pSIM1148. Furthermore, *Ast* gene-derived sequences can be inserted between a promoter and a terminator.

For any *Ast* gene, there may be other sequences having a high degree of sequence similarity. It is possible to use such homologous sequences to produce silencing constructs. For instance, fragments of a tomato *Ast* gene may be used to silenced the homologous *Ast* gene in potato.

Following identification of a plant-derived *Ast* gene, gene-specific primers are designed for PCR-amplification of the gene. PCR amplification is performed according to methods known in the art and then the PCR amplified asparaginase gene is cloned into a cloning vector.

*Ast* genes can be either identified by searching databases or isolated from plant DNA. One example of *Ast* genes from a crop other than potato is the *Ast* genes from wheat shown in SEQ ID NO.: 34 and 35. Simultaneous silencing of these genes in the wheat grain will make it possible to reduce asparagine levels in flour and, consequently, acrylamide levels in, for instance, bread, biscuits, cookies, or crackers.

Instead of using *Ast* gene fragments for production of a silencing construct; it is also possible to employ fragments obtained from the promoters of the Ast genes. Such promoters can be isolated by applying methods such as inverse PCR, and two copies of specific 150-600-basepair promoter fragments can then be inserted as inverted repeat between either a promoter and terminator or two convergently-oriented promoters (see: Rommens et aL, World Patent application 2006/036739 A2.

### Example 2

### Overexpression of asparaginase genes

This example demonstrates that overexpression of a gene involved in asparagine metabolism in the starchy tissues of a crop lowers the amount of asparagine in these starchy tissues and, consequently, lowers the amount of acrylamide in a food obtained from heating these starchy tissues.

The sequence of the potato asparaginase (*Asg1*) gene from the potato variety Ranger Russet is shown in SEQ m NO.: 9. The corresponding open reading flame and predicted amino acid sequence are shown in SEQ ID NO.: 10 and 11, respectively. A binary vector containing the *Asg1* gene inserted between the Agp promoter and Ubi3 terminator (SEQ ID NO.: 12) is designated pSIM658 **(****Figure 1C****).**

Expression levels of the *Asg1* gene in tubers were determined by applying quantitative real-time reverse transcriptase PCR. **Table 3** shows that tubers of 18 of 25 pSIM6S8 plants overexpressed the *Asg1* gene about 2 to 20-fold. These tubers were used to produce French fries. Chemical analyses demonstrated that fries of two of the transgenic lines contained reduced levels of acrylamide **(Table 4).**

It is also possible to employ other tuber-specific promoters to drive expression of the *Asg1* gene. Plasmid pSIM757 contains the Gbss promoter operably linked to the *Asg1* gene. Transformation of potato with the transfer DNA of this plasmid produces kanamycin resistant plants that overexpress the *Asg1* gene. Other promoters that can be used to drive asparaginase expression in potato tubers can be selected from the group consisting of patatin promoters, cold-inducible promoters, and flavonoid-3'-mono-oxygenase (Fmo) promoters. One Fmo promoter is shown in SEQ ID NO.: 13. This promoter is active in semi-mature and mature tubers but not in mini-tubers.

Instead of using *Asg1,* it is also possible to exploit other asparaginase genes. SEQ ID NO.: 14 shows the cDNA sequence of the alternative potato asparaginase *Asg2* gene. Other examples of asparaginase genes include the asparaginase gene of *E*. *coli* (accession number Z1051m; SEQ ID NO.: 31), Agrobacterium (accession Atu3044; SEQ ID NO.: 32), barley (accession AF308474; SEQ ID NO.: 33), and any gene encoding a protein with the motif pfam01112.12 (Marchler-Bauer et al., Nucleic Acids Res 33, D192-6, 2005):

The sequence of the asparaginase gene from wheat is shown in SEQ ID NO: 15. The predicted amino acid sequence is depicted in SEQ ID NO: 16.

For any asparaginase gene, there may be other asparaginase sequences having a high degree of sequence similarity. For example, a plant-derived asparaginase gene may be identified by searching databases such as those maintained by NCBI.

Following identification of a plant-derived asparaginase gene, gene-specific primers are designed for PCR-amplification of the asparaginase gene. PCR amplification is performed according to methods known in the art and then the PCR amplified asparaginase gene is cloned into a cloning vector.

The asparaginase is operably linked to a seed-specific promoter such as the wheat puroindoline gene promoter depicted in SEQ ID NO: 17. A transfer DNA comprising the resulting expression cassette is introduced using conventional transformation methods for the production of low-asparagine wheat. Flour derived from the wheat seed will accumulate less acrylamide during heating.

### Example 3

### Overexpression of a glutamine synthetase

Overexpression of a glutamine synthetase gene will result in reduced levels of asparagine. Any sequence encoding a protein with glutamine synthetase activity can be operably linked to a promoter that is expressed in a desired plant organ such as a potato tuber. Potato contains three related glutamine synthetase genes, shown in SEQ ID NOs.: 28-30. A DNA segment comprising a fragment of each of these genes can be used to effectively down-regulate glutamine synthetase activity. For this purpose, at least one copy of the segment can be inserted between either two convergent promoters or a promoter and terminator. The resulting expression cassette can be introduced into the plant-of-interest by employing any transformation method. Transgenic plants producing low-asparagine plant organs can then be selected for. Heat processing of these plant organs will provide products that contain less acrylamide than products obtained from the corresponding. For instance, a processed transgenic tuber will yield French fries that contain lower acrylamide levels than French fries obtained from untransformed tubers. The result of glutamine synthetase overexpression on asparagine levels have been described in by Harrison and coworkers (Plant Physiology 133: 252-262, 2003). However, these authors could not have anticipated the unexpected consequences of reduced asparagine levels on a strongly decreased heat-induced accumulation of acrylamide.

Similarly, it is possible to downregulate the expression of an endogenous gene displaying nitrate reductase activity. For this purpose, at least one copy of part of the gene or promoter of a nitrate reductase gene can be expressed. Transgenic plants can be screened for nitrate reductase gene expression levels, and lines displaying reduced levels can subsequently be screened for reduced asparagine levels. It is also possible to silence a hexose kinase gene and increase aspartic acid levels while reducing asparagine levels. A correlation between overexpression of either the nitrate reductase and hexokinase genes with increased asparagine levels have been described previously (Roland et al., Annu Rev Plant Biol 57: 675-709, 2006; Szopa, Biochem Soc Trans 30: 405-410, 2002). However, the authors did not anticipate the opposite approach to eventually reduce acrylamide levels in foods.

Obviously, there are other strategies to modify asparagine levels in plants by altering the expression of genes that are directly or indirectly involved in the synthesis or metabolism of asparagine. Our results imply that any of these methods can be used to produce low-acrylamide foods.

### Example 4

### Simultaneous down-regulated expression of starch degradation and asparagine biosynthesis genes

This example demonstrates that the simultaneous down-regulated expression of genes involved in starch degradation and asparagine biosynthesis, respectively, in starchy tissues of a crop can lower acrylamide accumulation in a food obtained from heating these starchy tissues.

Transgenic plants producing tubers with low levels of reducing sugars and asparagine were generated in two steps. First, plants were transformed with the P-DNA of binary vector pSIM371. This P-DNA contains two copies of a polynucleotide comprising fragments of the PPO (SEQ ID NO.: 18), R1 (SEQ ID NO.: 19), and phL (SEQ ID NO.: 20) gene, inserted as inverted repeat between the Gbss promoter and Ubi3 terminator.

An Agrobacterium strain carrying both pSIM371 and the LifeSupport vector pSIM368, which contains expression cassettes for both the *nptII* and *codA* genes inserted between T-DNA borders, was used to infect 21,900 potato stem explants. After a two-day co-cultivation period, the infected explants were subjected for five days to kanamycin to select for transient *nptII* gene expression. To prevent the proliferation of cells containing stably integrated T-DNAs, explants were subsequently transferred to media containing 5-fluorocytosine (5FC). This chemical is converted into toxic 5-fluorouracil (5FU) by the *codA* gene product (Perera et al., 1993). A total of 3,822 shoots that survived the double selection were genotyped for presence of the P-DNA and absence of any foreign DNA from either T-DNA or plasmid backbone. This analysis identified 256 all-native DNA (intragenic) shoots that were allowed to root, planted into soil, and grown for six weeks in growth chambers. To screen for PPO activity, a catechol solution was pipetted onto the cut surfaces of harvested ∼2-cM mini-tubers. Fourty-eight lined that were inhibited in catechol-induced tuber browning were grown in the greenhouse for three months to produce semi-mature tubers that were biochemically assessed for residual levels of PPO activity. This analysis demonstrated that employment of the PPO gene silencing construct lowered PPO activity by about 90%. Both the 48 intragenic lines and untransformed Ranger Russet and Russet Burbank control plants were subsequently propagated and grown in the field in Idaho, Aberdeen.

The mature tubers of all intragenic and control lines were analyzed for glucose levels after three and six-month of cold-storage. Most lines (43) displayed a greater reduction in cold-induced sweetening (∼60%) than obtained with control lines that had been silenced for only one of the starch-associated genes. French fries derived from the silenced tubers of plants 371-28 and 371-38 contained less than a third of the neurotoxin acrylamide that accumulated in control fries **(Table 4).** Such a reduction was anticipated because acrylamide is largely derived from heat-induced reactions between the carbonyl group of reducing sugars and asparagine (Mottram et al., 2002; Stadler et al., 2002).

The sensory characteristics of modified French fries were evaluated by a panel of eight professionally trained individuals. French fries derived from tubers of the modified Ranger Russet displayed a better visual appearance than fries from either Ranger Russet or Russet Burbank. Furthermore, the intragenic fries displayed a significantly better overall aroma as sensed by the olfactory epithelium which is located in the roof of the nasal cavity. A similar trend was observed for tubers that had been stored for ten weeks at 4°C. In fact, the cold-stored intragenic lines 371-28, 30, 38, and 68 still met or exceeded the sensory attributes of fresh untransformed varieties.

One low sugar potato line was retransformed with pSIM1148. Compared to French fries from the original pSIM1148 plants, fries from the kanamycin resistant double transformants will generally display further reduced levels of acrylamide. Hus, the double transformants produce tubers that can be used to obtain fries that (i) contain reduced levels of acrylamide and (ii) display enhanced sensory characteristics.

### Example 5

### All-native DNA transformation methods to reduce both asparagine levels and cold-induced sweetening in potato tubers

This example describes the employment of all-native DNA transformation methods to reduce both asparagine levels and cold-induced sweetening in potato tubers. Processed foods obtained from these tubers will contain reduced levels of acrylamide.

The transfer DNA used for transformation contains two expression cassettes inserted between potato-derived border regions is shown in SEQ ID NO.: 23 **(****Figure 2****).** The first cassette comprises two copies of a DNA segment comprising promoter fragments of the Ppo (SEQ ID NO.: 24), PhL (SEQ ID NO.: 25), and R1 (SEQ ID NO.: 26) gene, inserted as inverted repeat between a functionally active promoter of the Agp gene and the terminator of the ubiquitin-3 gene. The second cassette comprises two copies of a DNA segment comprising fragments of the Ast1, Ast2, and Ppo (SEQ ID NO.: 27) genes inserted as inverted repeat between two functionally active and convergently-oriented promoters of the Gbss gene.

A plasmid containing both the transfer DNA and an expression cassette for the Agrobacterium isopentenyl transferase (ipt) gene is introduced into Agrobacterium LBA4404, and the resulting strain is used to transform potato varieties such as Ranger Russet and Atlantic by employing marker-free transformation methods (see: Craig Richael, "Generation of marker-free and backbone-free transgenic plants using a single binary approach, Provisional Patent application 60/765,177, which is incorporated herein by reference). Transformed plants that do not display a cytokine phenotype, as typified by stunted growth and an inability to root, are allowed to produce tubers. Tubers of some of the lines will display low levels of Ppo enzyme activity, as can be tested for by pipetting 0.5 mL of 50 mM catechol onto freshly cut tuber surfaces. Levels of Ppo enzyme activity can be more accurately determined by mixing pulverized tubers (1 gram) for 1 hour in 50 mM 3-(N-morpholino) propane-sulfonic acid buffer at pH 6.5 (5 mL). After precipitation of the solid fraction, the change of OD41 0 can be determined over time. The lines of tubers than contain less than 25% of Ppo enzyme activities will be further tested by incubating tubers at about 4°C. After at least one month, glucose levels can be determined by, for instance, using the glucose oxidase/peroxidase reagent (Megazyme, Ireland). The lines of tubers that both display >75% reduced ppo activity levels and >50% reduced cold-induced sweetening can be analyzed for free asparagine levels. If free asparagine levels are reduced by about >50%, tubers can be processed and analyzed for acrylamide levels. Transformed lines that do contain low asparagine levels, in addition to the low Ppo and low cold-induced sweetening, can be considered for bulk-up and commercial production. French fries derived from tubers of the preferred lines contain less reducing sugars, thus making it possible to reduce blanch time and preserve the original potato taste. Furthermore, their visual appeal is enhanced by the absence of sugar ends and black spot bruise. French fries also have a better aroma and accumulate reduced levels of acrylamide, as can be determined by sensory panels trained to rate fries for sensory characteristics.

### Example 6.

### TILLING

Genes involved in the biosynthesis of asparagine, such as asparagine synthetase, cal also be down-regulated in their expression by mutating them. One method to accomplish this goal is designated as 'Targeting Induced Local Lesions IN Genomes' (TILLING). This method combines the efficiency of ethyl methanesulfonate (EMS)-induced mutagenesis with the ability of denaturing high-performance liquid chromatography (DHPLC) to detect base pair changes by heteroduplex analysis. The method generates a wide range of mutant alleles, is fast and automatable, and is applicable to any organism that can be chemically mutagenized. In the basic TILLING method, seeds are mutagenized by treatment with EMS. The resulting M1 plants are self-fertilized, and the M2 generation of individuals is used to prepare DNA samples for mutational screening while their seeds are inventoried. DNA samples are pooled, and pools are arrayed on microtiter plates and subjected to gene-specific PCR (McCallum et al., Nat Biotechnol 18: 455-457).

There are various alternatives to TILLING. For instance, it is possible to employ different types of mutagen such as fast neutrons or diepoxybutane (DEB). All these methods can be linked to reverse genetics platforms that allow the screening and isolation of mutants for pre-selected genes. Methods have been described in detail in, for instance Wang et al., Floriculture, Ornamental and Plant Biotechnology, Volume I, 2006, Global Science Books.

Furthermore, it is possible to simply screen available germplasm for a low-asparagine phenotype. Thus, molecular plant breeding, mutation breeding, and line selection all provide methods that make it possible to obtain 'low asparagine' varieties.

### Example 7

### Reducing asparagine levels

Asparagine levels can also be reduced by modifying grower practices. For instance, the asparagine synthetase gene is suppressed by carbon (Koch KE. Carbohydrate-modulated gene expression in plants. Annu Rev Plant Physiol Plant Mol Biol 47:509-540, 1996). It is also possible to reduce asparagine accumulation by reducing the nitrogen/sulfur ratio in soil. The relatively low nitrogen levels will result in reduced concentrations of N-rich compounds and an increase in S-containing metabolites such as cysteine, glutathione, and S-adenosylmethionine. Thus, soils that contain relatively high C, high S, and low N can be used to produce foods with relatively low asparagine.

### TABLES

**Table 1. Asparagine levels in tubers of three month-old greenhouse-grown potato lines.**

| *Line* | *Asparagine level (mgl100 g)* |
|---|---|
| Untransformed Ranger Russet - 1 | 150 |
| Untransformed Ranger Russet - 2 | 130 |
| Untransformed Ranger Russet - 3 | 100 |
| Untransformed Russet Burbank - 1 | 230 |
| Untransformed Russet Burbank - 2 | 210 |
| Transgenic kanamycin resistant control -1 | 200 |
| Transgenic kanamycin resistant control -2 | 220 |
| Transgenic kanamycin resistant control -3 | 110 |
| Transgenic kanamycin resistant control -4 | 200 |
| Transgenic kanamycin resistant control -5 | 130 |
| Transgenic line 1148-1 1 | 160 |
| Transgenic line 1148-3 | 90 |
| Transgenic line 1148-4 | 70 |
| Transgenic line 1148-5 | 150 |
| Transgenic line 1148-6 | 80 |
| Transgenic line 1148-7 | 70 |
| Transgenic line 1148-8 | 80 |
| Transgenic line 1148-10 | 110 |
| Transgenic line 1148-11 | 160 |
| Transgenic line 1148-13 | 80 |
| Transgenic line 1148-14 | 210 |
| Transgenic line 1148-15 | 110 |
| Transgenic line 1148-17 | 50 |
| Transgenic line 1148-18 | 90 |
| Transgenic line 1148-19 | 80 |
| Transgenic line 1148-21 | 60 |
| Transgenic line 1148-22 | 170 |
| Transgenic line 1148-23 | 80 |
| Transgenic line 1148-24 | 80 |
| Transgenic line 1148-25 | 90 |
| Transgenic line 1148-26 | 80 |
| Transgenic line 1148-28 | 310 |

**Table 2. Acrylamide levels in French fries obtained from tubers of three month-old greenhouse-grown potato lines. Levels were determined according to the united States Food and Drug administration, Center for Food Safety and Applied Nutrition Office of the Plant & Dairy Foods and Beverages, "Detection and Quantitation of Acrylamide in Foods" (2002).**

| *Line* | *Acrylamide level (parts per billion)* |
|---|---|
| Untransformed Ranger Russet - 2 | 126 |
| Transgenic kanamycin resistant control -1 | 127 |
| Transgenic line 1148-7 | 46.6 |
| Transgenic line 1148-17 | <20.0 |
| Transgenic line 1148-19 | <20.0 |
| Transgenic line 1148-21 | 38.6 |
| Transgenic line 1148-24 | 23.1 |

**Table 3. Expression levels of the Asparaginase-1 gene in potato tubers of six week-old growth chamber-grown potato lines as determined by quantitative real time RT-PCR.**

| *Line* | *Relative* *Expression* | *Standard Error* |
|---|---|---|
| Transgenic kanamycin resistant control -1 | 22.4 | 8.8 |
| Transgenic kanamycin resistant control -2 | 8.9 | 4.2 |
| Transgenic kanamycin resistant control -3 | 23.7 | 4.2 |
| Transgenic kanamycin resistant control-4 | 27.5 | 3.4 |
| Untransformed Ranger Russet - 1 | 22.4 | 8.8 |
| Transgenic line 658-1 | 101.6 | 13.4 |
| Transgenic line 658-2 | 58.8 | 8.1 |
| Transgenic line 658-3 | 912.9 | 57 |
| Transgenic line 658-4 | 165.9 | 52.1 |
| Transgenic line 658-5 | 75.8 | 6.3 |
| Transgenic line 658-7 | 101.2 | 10.7 |
| Transgenic line 658-8 | 289.3 | 59.6 |
| Transgenic line 658-9 | 99.0 | 9.8 |
| Transgenic line 658-11 | 92.1 | 8.2 |
| Transgenic line 658-12 | 85.9 | 29.2 |
| Transgenic line 658-14 | 390.2 | 5.0 |
| Transgenic line 658-15 | 57.9 | 8.0 |
| Transgenic line 658-16 | .8.4 | 1.7 |
| Transgenic line 658-17 | 64.7 | 4.2 |
| Transgenic line 658-18 | 112.1 | 21.8 |
| Transgenic line 658-19 | 196.7 | 46.6 |
| Transgenic line 658-20 | 101.9 | 31.2 |
| Transgenic line 658-21 | 58.3 | 3.7 |
| Transgenic line 658-22 | 81.4 | 20.5 |
| Transgenic line 658-23 | 85.7 | 17.8 |
| Transgenic line 658-24 | 281.0 | 63.7 |
| Transgenic line 658-25 | 229.0 | 67.1 |
| Transgenic line 658-26 | 110.7 | 16.2 |
| Transgenic line 658-27 | 220.3 | 66.9 |
| Transgenic line 658-28 | 151.1 | 17.5 |

**Table 4. Acrylamide levels in French fries obtained from tubers of three month-old greenhouse-grown potato lines.**

| *Line* | *Acrylamide level (parts per billion)* |
|---|---|
| Untransformed Ranger Russet - 1 | 1150 |
| Untransformed Ranger Russet - 2 | 1200 |
| Untransformed Russet Burbank - 1 | 958 |
| Untransformed Russet Burbank - 2 | 1230 |
| Intragenic line 371-28-1 | 211 |
| Intragenic line 371-28-2 | 281 |
| Intragenic line 371-38-1 | 152 |
| Intragenic line 371-38-2 | 184 |

### SEQUENCES

SEQ ID NO: 1 (asparagine synthetase 1)

ATGTGTGGAATTTTGGCTTTGTTGGGTTGTTCGGATGATTCTCAGGCTAAAAGGGTTCGAGTTCTTGAG
CTTTCTCGCAGGTTGAAGCATCGTGGACCGGATTGGAGTGGAATATTTCAATATGGTGATTTTTACTTG
GCACATCAACGTCTAGCAATTATCGACCCTGCTTCTGGTGATCAACCTCTGTTTAATGAAGACAAAAAG
ATTGTTGTTACTGTTAATGGAGAGATCTACAATCATGAAAAACTTCGAAAACTTATGCCTAATCACAAG
TTTAGGACTGGAAGTGATTGTGATGTTATTGCTCATCTTTATGAAGAATATGGAGAAAATTTTGTTGAC
ATGCTGGATGGAGTGTTCTCTTTTGTATTATTGGATACTCGCGATAATAGCTTTCTTGCTGCTCGTGAT
GCCATCGGAATTACACCCCTCTATATTGGTTGGGGACTTGATGGCTCTGTGTGGATATCATCTGAGCTG
AAGGGCTTGAATGATGATTGTGAACATTTTGAAGTTTTCCCTCCGGGGCACTTGTACTCTAGCAAGAAC
GGAGGGCTTAGGAGATGGTACAATCCCGCTTGGTTCTCTGAAGCAATTCCTTCCACTCCTTATGACACT
TTGGTTCTGAGGCGTGCCTTCGAAAATGCTGTTATCAAACGGTTGATGACTGATGTCCCCTTTGGCGTT
CTGCTCTCGGGGGGACTTGATTCGTCTTTGGTTGCTTCTGTCACTGACTCGATACTTGGCTGGAACAAA
AGCTGCAAGCAATGGGGAGCACAACTTCATTCCTTCTGTGTTGGTCTCGAGGGCTCACCAGATCTCAAG
GCTGCAAAAGAAGTTGCTGACTTTTTAGGAACCGTTCACCATGAGTTTCACTTTACTGTTCAGGACGGT
ATTGATGCTATTGAAGATGTTATATATCATATCGAGACGTATGATGTAACAACAATAAGAGCCAGCACT
CCTATGTTCCTTATGTCGCGTAAGATTAAATCACTAGGAGTGAAGATGGTCATATCAGGGGAAGGCGCT
GACGAAATTTTTGGTGGTTACTTGTACTTCCACAAGGCTCCCAACAAGGAAGAGTTCCACACGGAAACA
TGTCGCAAGATAAAAGCGCTTCACCAGTATGACTGTTTAAGAGCAAACAAGGCTACATCCGCGTGGGGC
TTAGAAGCTAGAGTACCATTTCTGGATAAAGAGTTCATCGATGTTGCCATGAGTATCGATCCCGAATGG
AAGATGATTAAGCATGATCAAGGAAGGATTGAGAAGTGGGTTCTTAGGAAGGCGTTTGATGATGAGGAG
CAACCGTACCTTCCAAAGCATATTCTGTACAGACAGAAAGAACAATTCAGCGATGGCGTAGGCTATAGT
TGGATCGATGGCCTCAAAGCACATGCTGAACAACATGTGACTGATAGGATGATGCTTAATGCTGCTCAT
ATCTTCCCACATAACACTCCGACTACAAAGGAAGGATACTATTACAGAATGATTTTCGAGAGGTTCTTC
CCACAGAACTCAGCAAGCCTGACCGTTCCTGGAGGACCGAGTATAGCTTGCAGCACGGCAAAAGCAATT
GAGTGGGATGCTTCTTGGTCGAACAACCTTGATCCTTCCGGTAGGGCTGCTATCGGTGTACATAACTCT
GCTTATGACAATCATCTATCTAGTGTTGCTAATGGGAATTTGGACACCCCGATCATCAATAATGTGCCA
AAGATGGTAGGCGTGGCGTGGCTGCAGAGCTCACAATAAGGAGCTAA

SEQ ID NO: 2 (asparagine synthetase 2)

CACTTTTCTCCATTTCAGAAGAAGCGAGAAAAAAGTTGCGAGCAATGTGTGGAATACTTGCAATTTTCG
GTTGCACTGATAATTCTCATGCCAAGCGTTCAAGAATCATCGAACTATCAAGAAGGTTGCGCCATAGAG
GACCTGATTGGAGTGGATTGCATAGCCATGAGGACTGTTATCTTGCTCATCAACGATTGGCAATAGTAG
ACCCAACTTCAGGAGATCAGCCGCTGTATAATGAGGACAAGACCATTGTTGTTGCGGTAAATGGAGAGA
TCTACAACCATAAGGAATTACGGGAGAAACTGAAGTCTCATCAGTTTCGAACTGAAAGTGATTGTGAAG
TTATTGCCCATCTTTATGAAGAATATGGAGAAAACTTCATTGACATGTTGGATGGGATGTTCTCTTTTG
TTCTTCTTGATACCCGGGATAAAAGTTTCATCGCTGCTCGGGATGCCATTGGCATTACACCCCTTTATA
TGGGGTGGGGTCTTGATGGCTCCATATGGTTTTCCTCAGAGATGAAAGCCTTAAGTGATGATTGTGAAC
GATTTGTTAGCTTCCTTCCCGGTCATATTTATTCAAGCAAAAATGGAGGACTTAGAAGATGGTACAACC

CACCATGGTTTTCGGAAACCATTCCTTCTACACCATATGATCCCCTTGTCTTACGGAAGGCTTTTGAGA
AGGCTGTAGTTAAGAGACTCATGACGGATGTACCATTTGGTGTGCTTCTCTCAGGCGGACTGGATTCTT
CACTTGTTGCTGCAGTGGCTAACCGTTATTTGGCTGATACAGAAGCCGGTCGACAATGGGGATCACAGT
TGCATACATTTTGCGTAGGCTTGAAGGGTTCTCCTGATCTGAAAGCTGCCAGAGAGGT

SEQ ID NO: 3 (Ast1 fragment)

TCACAAGTTTAGGACTGGAAGTGATTGTGATGTTATTGCTCATCTTTATGAAGAATATGGAGAAAATTT
TGTTGACATGCTGGATGGAGTGTTCTCTTTTGTATTATTGGATACTCGCGATAATAGCTTTCTTGCTGC
TCGTGATGCCATCGGAATTACACCCCTCTATATTGGTTGGGGACTTGATGGCTCTGTGTGGATATCATC
TGAGCTGAAGGGCTTGAATGATGATTGTGAACATTTTGAAGTTTTCCCTCCGGGGCACTTGTACTCTAG
CAAGAACGGAGGGCTTAGGAGATGGTACAATCCCGCTTGGTTCTCTGAAGCAATTCCTTCCACTCCTTA
TGA

SEQ ID NO: 4 (Ast2 fragment)

TCATCAGTTTCGAACTGAAAGTGATTGTGAAGTTATTGCCCATCTTTATGAAGAATATGGAGAAAACTT
CATTGACATGTTGGATGGGATGTTCTCTTTTGTTCTTCTTGATACCCGGGATAAAAGTTTCATCGCTGC
TCGGGATGCCATTGGCATTACACCCCTTTATATGGGGTGGGGTCTTGATGGCTCCATATGGTTTTCCTC
AGAGATGAAAGCCTTAAGTGATGATTGTGAACGATTTGTTAGCTTCCTTCCCGGTCATATTTATTCAAG
CAAAAATGGAGGACTTAGAAGATGGTACAACCCACCATGGTTTTCGGAAACCATTCCTTCTACACCATA
TGA

SEQ ID NO: 5

TCATATGGTGTAGAAGGAATGGTTTCCGAAAACCATGGTGGGTTGTACCATCTTCTAAGTCCTCCATTT
TTGCTTGAATAAATATGACCGGGAAGGAAGCTAACAAATCGTTCACAATCATCACTTAAGGCTTTCATC
TCTGAGGAAAACCATATGGAGCCATCAAGACCCCACCCCATATAAAGGGGTGTAATGCCAATGGCATCC
CGAGCAGCGATGAAACTTTTATCCCGGGTATCAAGAAGAACAAAAGAGAACATCCCATCCAACATGTCA
ATGAAGTTTTCTCCATATTCTTCATAAAGATGGGCAATAACTTCACAATCACTTTCAGTTCGAAACTGA
TGAGAATTCGAACTCTTTATCCAGAAATGGTACTCTAGCTTCTAAGCCCCACGCGGATGTAGCCTTGTT
TGCTCTTAAACAGTCATACTGGTGAAGCGCTTTTATCTTGCGACATGTTTCCGTGTGGAACTCTTCCTT
GTTTGGAGCCTTGTGGAAGTACAAGTAGCCACCAAAAATTTCGTCAGCACCTTCCCCTGATATGACCAT
CTTCACTCCTAGTGATTTAATCTTACGTGACATAAGGAACATAGGAGTGCTGGCTCTTATTGTTGTTAC
ATCATACGTCTCGATATGATATATAACATCTTCAATAGCATCAATCCCGTCCTGAACAGTAAAGTGAAA
CTCGTGGTGAACGGTTCCTAAAAAGTCAGCAACTTCTTTTGCAGCCTTGAGATCTGGTGAGCCCTCGAG
A

SEQ ID NO: 6

CTGCAGGTGTATGGGTGATCCTTCTCTTATTATACCGACTAAAGACATTGGTATTAAGGATATCTTATC
TTTTGAGGAGATTCCCGTTCAGATTCTGGAGCGTCAGGTTCGCAAGTTGAGAACCAATGAGGTAACATC
AGTCAAGGTCTTATGGAGGAATCAGCCGCGG

SEQ ID NO: 7 (P:Agp)

CAAGTGTCTGAGACAACCAAAACTGAAAGTGGGAAACCAAACTCTAAGTCAAAGACTTTATATACAAAA
TGGTATAAATATAATTATTTAATTTACTATCGGGTTATCGATTAACCCGTTAAGAAAAAACTTCAAACC
GTTAAGAACCGATAACCCGATAACAAAAAAAATCTAAATCGTTATCAAAACCGCTAAACTAATAACCCA
ATATTGATAAACCAATAACTTTTTTATTCGGGTTATCGGTTTCAGTTCTGTTTGGAACAATCCTAGTG
TCCTAATTATTGTTTTGAGAACCAAGAAAACAAAAACTTACGTCGCAAATATTTCAGTAAATACTTGTA
TATCTCAGTGATAATTGATTTCCAACATGTATAATTATCATTTACGTAATAATAGATGGTTTCCGAAAC
TTACGCTTCCCTTTTTTCTTTTGCAGTCGTATGGAATAAAAGTTGGATATGGAGGCATTCCCGGGCCTT
CAGGTGGAAGAGACGGAGCTGCTTCACAAGGAGGGGTTGTTGTACTTGAAAATGGGCATTTATTGTTC
GCAAACCTATCATGTTCCTATGGTTGTTTATTTGTAGTTTGGTGTTCTTAATATCGAGTGTTCTTTAGT
TTGTTCCTTTTAATGAAAGGATAATATCTGTGCAAAAATAAGTAAATTCGGTACATAAAGACATTTTTT
TTTGCATTTTCTGTTTATGGAGTTGTCAAATGTGAATTTATTTCATAGCATGTGAGTTTCCTCTCCTTT
TTCATGTGCCCTTGGGCCTTGCATGTTTCTTGCACCGCAGTGTGCCAGGGCTGTCGGCAGATGGACATA
AATGGCACACCGCTCGGCTCGTGGAAAGAGTATGGTCAGTTTCATTGATAAGTATTTACTCGTATTCGG
TGTTTACATCAAGTTAATATGTTCAAACACATGTGATATCATACATCCATTAGTTAAGTATAAATGCCA
ACTTTTTACTTGAATCGCCGAATAAATTTACTTACGTCCAATATTTAGTTTTGTGTGTCAAACATATCA
TGCACTATTTGATTAAGAATAAATAAACGATGTGTAATTTGAAAACCAATTAGAAAAGAAGTATGACGG
GATTGATGTTCTGTGAAATCACTGGTAAATTGGACGGACGATGAAATTTGATCGTCCATTTAAGCATAG
CAACATGGGTCTTTAGTCATCATCATTATGTTATAATTATTTTCTTGAAACTTGATACACCAACTTTCA
TTGGGAAAGTGACAGCATAGTATAAACTATAATATCAATTCTGGCAATTTCGAATTATTCCAAATCTCT
TTTGTCATTTCATTTCCTCCCCTATGTCTGCAAGTACCAATTATTTAAGTACAAAAAATCTTGATTAAA
CAATTTATTTTCTCACTAATAATCACATTTAATCATCAACGGTTCATACACGTCTGTCACTCTTTTTTT
ATTCTCTCAAGCGCATGTGATCATACCAATTATTTAAATACAAAAAATCTTGATTAAACAATTCAGTTT
CTCACTAATAATCACATTTAATCATCAACGGTTCATACACATCCGTCACTCTTTTTTTATTCTCTCAAG
CGCATGTGATCATACCAATTATTTAAATACAAAAAATCTTGATTAAACAATTCATTTTCTCACTAATAA
TCACATTTAATCATCAACGGTTTATACACGTCCGCCACTCTTTTTTTATTCTCTCAAGCGTATGTGATC
ATATCTAACTCTCGTGCAAACAAGTGAAATGACGTTCACTAATAAATAATCTTTTGAATACTTTGTTCA
GTTTAATTTATTTAATTTGATAAGAATTTTTTTATTATTGAATTTTTATTGTTTTAAATTAAAAATAAG
TTAAATATATCAAAATATCTTTTAATTTTATTTTTGAAAAATAACGTAGTTCAAACAAATTAAAATTGA
GTAACTGTTTTTCGAAAAATAATGATTCTAATAGTATATTCTTTTTCATCATTAGATATTTTTTTTAAG
CTAAGTACAAAAGTCATATTTCAATCCCCAAAATAGCCTCAATCACAAGAAATGCTTAAATCCCCAAAA
TACCCTCAATCACAAGACGTGTGTACCAATCATACCTATGGTCCTCTCGTAAATTCCGACAAAATCAGG
TCTATAAAGTTACCCTTGATATCAGTATTATAAAACTAAAAATCTCAGCTGTAATTCAAGTGCAATCAC
ACTCTACCACACACTCTCTAGTAGAGAGATCAGTTGATAACAAGCTTGTTAACG

SEQ ID NO: 8 (P:Gbss)

CGAACCATGCATCTCAATCTTAATACTAAAAAATGCAACAAAATTCTAGTGGAGGGACCAGTACCAGTA
CATTAGATATTATCTTTTATTACTATAATAATATTTTAATTAACACGAGACATAGGAATGTCAAGTGGT
AGCGGTAGGAGGGAGTTGGTTCAGTTTTTTAGATACTAGGAGACAGAACCGGAGGGGCCCATTGCAAGG
CCCAAGTTGAAGTCCAGCCGTGAATCAACAAAGAGAGGGCCCATAATACTGTCGATGAGCATTTCCCTA
TAATACAGTGTCCACAGTTGCCTTCCGCTAAGGGATAGCCACCCGCTATTCTCTTGACACGTGTCACTG
AAACCTGCTACAAATAAGGCAGGCACCTCCTCATTCTCACACTCACTCACTCACACAGCTCAACAAGTG
GTAACTTTTACTCATCTCCTCCAATTATTTCTGATTTCATGCATGTTTCCCTACATTCTATTATGAATC
GTGTTATGGTGTATAAACGTTGTTTCATATCTCATCTCATCTATTCTGATTTTGATTCTCTTGCCTACT
GAATTTGACCCTACTGTAATCGGTGATAAATGTGAATGCTTCCTCTTCTTCTTCTTCTCAGAAATC
AATTTCTGTTTTGTTTTTGTTCATCTGTAGCTTGGTAGATTCCCCTTTTTGTAGACCACACATCACG

SEQ ID NO: 9 (asparaginase-1)

ATGGGTTGGGCTATAGCGTTGCACGGTGGAGCTGGTGACATACCCAAGGATCTGCCGCCGGAGCTTCGT
GAGCCCAGAGAAGCCTCTCTTCGCTATTGCTTACAGATTGGCGTCGATGCTATCAAGGCCCAAAAATCC
CCTTTGGACGTTGTTGAACTCGTGGTATACTACTTACCAACTTTACCTATCATATCTTAAAGTATAGAA
TGTAGGATTTTGCCTTGCATCTGTTCAATTTCTCATCAAGACTCGCGATGGATATCACTTGTTACCATG
ATTAGAGGAAAAAATATGGGTTTGACTCATTTTCTCTCTCTCTCGTCAGGTTGTTTGGGGGAGGGATCT
TTGTTTACTTGTTTTTCTATTAGTACTATGTTAGGATGACTAGTGTTTGATTCTTATGATGAATAGCTT
TTTATCTATGGCTTATGAAATAATTGACTTACTGGATGTCTAGTAATTTCATGGATCTACATGACATCA
ACTATAAAAGCTTCTGCAAGTTGGAGTTCCTGATTTAAAGCTTCAAAAAGATTATAGAAACATGATCTC
TCTATTTGATCCTCTGAGATTGAGTTGGAGTTTTCACCTCAATATTCAATAACATTCTCTTGTGATGTC
TCCCTAAGTTGTCACCTCTCGCTAGCATGCAGGATGATACTATTGTTAATTTTGTTAACCGTGCTCTTG
CTCCCTGCTTTAGTTTTTCTTACAAACACATACTTCCACTACTTCAATTCGTGCAAGGGAAAGTGTCAT
TCCATATATGTGCGTAGAAACGCTCCTGAAAAACTTGGGTTCTCCGGGAGCCTTCCGTATAATGAGTTT
TTTTTTATTTTACCTTTTACTGATATTGTGATAGCTTTTAACTGTCTTGGATCAAGCAGGTGCGGGAAC
TAGAAAATAACCCATACTTCAATGCTGGTAGAGGGTCTGTCTTAACCAGCAATGGCACAGTAGAAATGG
AAGCATGCATCATGGATGGGAATACGAAAAACTGTGGAGCTGTTTCTGGCCTAACCACTGTTGTCAATG
CTATATCTCTGGCTAGGCTGGTCATGGAAAAAACTCCACATATATATCTTGCATTTGAGGGAGCGGAAG
CATTTGCGAGGGAGCAGGTCTGTAAAAATTTTCTAATTGTCTTCTCTCTTATGGACATGCCTGAAGAAA
ACGTTAAGAAAGTTATTGAAGTACTCAATGCTGATGAATGATATCTTGTATCCGGAAACTGGATGATGC
AAAGACTTGACGGATTATTCTCTCTGTTAATACTGTTTAATTACAGTTCTAATTTCTGATGGTCTGTTT
ATGGACTTCAAGCAGCTACCAGTTTCTCTAAGTTTTTCTGGTTAATTAATGTGACCTTTCTGGCATAGG
TGACTAATTCATTTTAAACTTACTATTAGTTAACTTCCTCTGATAATATAACTCCCTAGTTGTATGATT
GTATTATGTTCATTTTTCTAATCCTTTTTTACATAGAATCTATTTGATGAACTATGATGGTTCTGTTGT
CAAGGGGGTTGAAACCACGGACTCAAGCCATTTTATCACGCCAAGAAATATCGAGAGACTAAAACAAGC
AAAAGAAGCAAACAAAGTCCAGGTATATAACCCTATCTCTTCATTGTTATATCTTTGTTGCAAGATAGC
ATATTCATGCTTTTGGCCTTGATATTGATAGAAGTCCACTGTTTTCTTATTGTACTTGTTTTTATCTGA

CCTTTTGATTGTGAATTTTAAGAGCTTTGGTGTTTTGTTTTTGGAAAGCATCAACAAAAATATTATCCA
TTTGTACTTGGGTCTTCCTCTTGTGGCCTTCAAGTGAGTTTGGTTATGGCGTCGGCCTCAATAGCTTAA
ATGTAGTCATGTGGCTATGTCTGTAACAGAGCTTTTTAGTTCTATTCCCTTCTTGGCAACTCAGTCTCG
TGATTCAAGGCTCAATTCTTCTGTAATTCTTAACATCGAGATGCTTTCTGCTTTGGTATTTTTGGTTAA
CATTGCTGCTACCAATTTGCAGGTTGATTATAATACACGGCCTATACCAAAAGATGACAAAACACCAGC
TCCAAGTGGAGATAGTCAGCTTGGAACGGTTGGATGTGTAGCTGTTGACAGCTTTGGACATTTAGCTGC
TGCTACATCTACTGGAGGACTAGTAAACAAGATGGTTGGAAGGATAGGAGATACTCCCATTATTGGTGC
AGGTACATATGCAAACAAACTATGTGCAGTCTCTGCTACAGGCCAAGGTGAAGCTATAATCCGTGCGAC
TGTAGCAAGAGATGTGGCTGCTCTAATGGAGTATAAAGGGCTTTCTCTCAAGGAAGCAGCAGACTACGT
TATAGAGGAATCTGCGCCAAAAGGAACCACTGGCCTGATTGCTGTATCGGCCACTGGGGAAGTTAGCAT
GCCATTTAATACAACCGGAATGTTTAGAGCTTGTGCAACTGAAGATGGTCACACAGAATTAGCAATTTG
GTAACTTTTCATTAGAATAGATTAG

SEQ ID NO: 10 (asparaginase-1 cDNA)

ATGGGTTGGGCTATAGCGTTGCACGGTGGAGCTGGTGACATACCCAAGGATCTGCCGCCGGAGCTTCGT
GAGCCCAGAGAAGCCTCTCTTCGCTATTGCTTACAGATTGGCGTCGATGCTATCAAGGCCCAAAAATCC
CCTTTGGACGTTGTTGAACTCGTGGTGCGGGAACTAGAAAATAACCCATACTTCAATGCTGGTAGAGGG
TCTGTCTTAACCAGCAATGGCACAGTAGAAATGGAAGCATGCATCATGGATGGGAATACGAAAAACTGT
GGAGCTGTTTCTGGCCTAACCACTGTTGTCAATGCTATATCTCTGGCTAGGCTGGTCATGGAAAAAACT
CCACATATATATCTTGCATTTGAGGGAGCGGAAGCATTTGCGAGGGAGCAGGGGGTTGAAACCACGGAC
TCAAGCCATTTTATCACGCCAAGAAATATCGAGAGACTAAAACAAGCAAAAGAAGCAAACAAAGTCCAG
GTTGATTATAATACACGGCCTATACCAAAAGATGACAAAACACCAGCTCCAAGTGGAGATAGTCAGCTT
GGAACGGTTGGATGTGTAGCTGTTGACAGCTTTGGACATTTAGCTGCTGCTACATCTACTGGAGGACTA
GTAAACAAGATGGTTGGAAGGATAGGAGATACTCCCATTATTGGTGCAGGTACATATGCAAACAAACTA
TGTGCAGTCTCTGCTACAGGCCAAGGTGAAGCTATAATCCGTGCGACTGTAGCAAGAGATGTGGCTGCT
CTAATGGAGTATAAAGGGCTTTCTCTCAAGGAAGCAGCAGACTACGTTATAGAGGAATCTGCGCCAAAA
GGAACCACTGGCCTGATTGCTGTATCGGCCACTGGGGAAGTTAGCATGCCATTTAATACAACCGGAATG
TTTAGAGCTTGTGCAACTGAAGATGGTCACACAGAATTAGCAATTTGGTAACTTTTCATTAGAATAGAT
TAG

SEQ ID NO: 11 (asparaginase-1 protein

MGWAIALHGGAGDIPKDLPPELREPREASLRYCLQIGVDAIKAQKSPLDVVELVVRELENNPYFNAGRG
SVLTSNGTVEMEACIMDGNTKNCGAVSGLTTVVNAISLARLVMEKTPHIYLAFEGAEAFAREQGVETTD
SSHFITPRNIERLKQAKEANKVQVDYNTRPIPKDDKTPAPSGDSQLGTVGCVAVDSFGHLAAATSTGGL
VNKMVGRIGDTPIIGAGTYANKLCAVSATGQGEAIIRATVARDVAALMEYKGLSLKEAADYVIEESAPK
GTTGLIAVSATGEVSMPFNTTGMFRACATEDGHTELAIW

SEQ ID NO: 12 (ubiT)

TTGATTTTAATGTTTAGCAAATGTCCTATCAGTTTTCTCTTTTTGTCGAACGGTAATTTAGAGTTTTTT
TTGCTATATGGATTTTCGTTTTTGATGTATGTGACAACCCTCGGGATTGTTGATTTATTTCAAAACTAA
GAGTTTTTGCTTATTGTTCTCGTCTATTTGGATATCAATCTTAGTTTTATATCTTTTCTAGTTCTCTA
CGTGTTAAATGTTCAACACACTAGCAATTTGGCTGCAGCGTATGGATTATGGAACTATCAAGTCTGTGG
GATCGATAAATATGCTTCTCAGGAATTTGAGATTTTACAGTCTTTATGCTCATTGGGTTGAGTATAATA
TAGTAAAAAAATAGG

SEQ ID NO: 13 (P:Fmo)

CCTTAATTCTATACACTATTATTTCCTCTTTTATTCTACATTTCATTCTTAGCTTATTTTTTCGTAAAC
GTTACCCAGTGCCACTACCACCCGGTCCAAAACCATGGCCAATAATCGGAAACATAGTCCAATTAGGTC
CGAAGCCGCACCAGTCCACTGCATCAATGGCCCGAACTTACGGGCCACTCATGCACCTTCGCATGGGGT
TCGTGGACGTGGTGGTTGCGGCCTCAGCTTCGGTGGCGGCTCAATTTTTGAAAAATCATGACGCTAACT
TCTCGAGCCGCCCACCGAACTCTGGGGCGAAACACATGGCTTATAATTACCATGACCTTGTGTTTGCAC
CTTACGGACCACGGTGGCGTATGCTAAGGAAAATTTGTTCTGTTCATCTCTTTTCGGCTAAAGCTTTAG
ATGACTTCCGCCATGTCCGACAGGAAGAAGTCAGAACACTTACGCGCGCCTTAGCAAATGCTGGCCAAA
AGCCAATCAAATTAGGGCAGCTGTTGAACGTGTGCACCACGAATGCACTTGCGCGTGTGATGCTCGGGA
AGCGGGTATTCGCCGACGGTACTAACGGTATCGATCCACAAGCGGAGGAGTTCAAGTTAATGGTGGTGG
AGATGATGGTGCTCGCCGGCGTTTTCACATCGGCGATTTTATTCCGGCGCTTGATTGGATGGACATTCA
AGGCGTAGCAGGAAAAATGAAGAAACTCCACGCGCGTTTCGACGCGTTCTTAACCACGATCCTCGAAGA
ACACAAGGGAAAGCGAGTTGGAGAATCGAAGGAGCAGGGGGATTTGTTGAATACGTTGATCTCTCTGAA
AAATGAAGAAGACGATAATGGAGGAAAGCTTACTGATACAGAAATTAAAGCTTTACTTTGGGTACGCCT
CTTACAATTATCTCTTTATTTCAAATTGGACAAGTAAAAACAAATATGGATTTTTAGTATATCTAACAA
GTAAAAAGGAATAGAGGTAATAAATATGAAACTATGCCATTTTTCTTTGACGGACTAAAAATGGAAGTA
TGCTAATGTCCTAATTTATATGATAATGTTTGGCTTGAAACAATGTTGTTTAAGAAGTTAATTTTTATT
CGTCCTGCAATTTTAATGGTATGAGTTCGAATTTCAGGATATAGTTTGATCAATTGTTCTTATACAAAT
TCACTCTAATATTACAAACTTACAAATTTGAAGTTTAAAGATTTATCAGTTCAAATTTCATGATTTTTC
ACCTTTTCAAAGCCTTAAACTCGAATTATACAAGTGTGGGAGTTATT

SEQ ID NO: 14 (potato asparaginase-2)

ATGGGTGGTTGGGCTATAGCGGTGCACGGTGGCGCTGGTGTGGACCCAAATCTCCCAGCTGAACGTCAG
AAACAAGCTAAAGAACTCCTTACTCGTTGCCTTAACATTGGAATCTCCGCTCTTCGCTCTTCTCTACCT
GCCATTGATGTTGTTGAACTCGTTGTGAGAGAACTGGAAAGTGATCCTCTATTCAATTCGGGTCGTGGA
TCTGCATTAACTGCAAATGGAACAGTGGAAATGGAGGCGAGCATTATGGACGGCGACGGTAGACGATGC
GGCGCCGTTTCTGGTATCTCCACCGTGAAAAACCCAATCTCCCTCGCTCGCCTTATCATGGATAAATCC
CCTCATTCCTATCTCGGTTTCTCCGGCGCTGAAGAATTCGCCAAACAACAGGGCGTGGAGATGGTAGAC
AATGAATATTTCATCACCGAGGACAATGTTGGAATGCTGAAACTAGCCAAAGAGGCTAACACCATTTTG
TTCGATTACAGAATTCCATTAACTGGATTGGATTCCTGTGCGTCATCCGTTGAAAGCCCAATTCGCATG
AACGGATTACCGATAAGTGTTTACGCGCCGGAGACGGTGGGATGTGTGGTGGTAGACGGCCAAGGTAGG
TGCGCCGCCGCCACATCCACCGGTGGTTTAATGAACAAAATGACCGGTCGTATCGGTGACTCACCGCTG
ATTGGTGCTGGGACCTACGCAGGTGAGCTTTGTGGGGTGTCATGTACAGGGGAAGGAGAAGCTATCATA
CGTGGAACCCTAGCACGTGACGTGGCAGCAGTTATGGAATATAAGGAATTGGGCCTTCAAGAAGCAGTG
GACTTTGTGATTAAGAAGAGATTGGATAAAGGGTTTGCTGGGCTTATTGCTGTGTCTAATAAAGGGGAA
GTGGCTTATGGGTTTAATTGTAATGGAATGTTTAGAGGATGTGCTACTGAAGATGGATTTATGGATGTT
GGTATTTGGTAA

SEQ ID NO: 15 (wheat asparaginase)

ATGGCGCGCTGGGCCATCGCCATCCACGGAGGCGCGGGCGTGGACCCCAACCTGCCGGAGCACCGCCAG
GAGGAGGCCAAGCGCGTGCTGGCCCGGTGCCTGCAGGTCGGCGTCGACCTGCTCCGGGCTGGTGCGACG
GCGCTGGACGTCGTGGAGGCCGTGGTGCGGGAGCTGGAGACGGACCCCTGCTTCAACTCGGGCCGCGGC
TCCGCGCTCACACGCGCCGGCACCGTCGAGATGGAGGCCAGCATCATGGACGGCCGCGGCCGCCGCTGC
GGCGCCGTCTCCGGTGTGTCCACCGTTAAAAACCCCGTGTCCCTGGCCCGGCGCGTCATGGACAAGTCC
CCACACTCCTACCTCGCCTTCGACGGCGCCGAGGATTTCGCGCGCGAGCAGGGCCTGGAGGTTGTGGAT
AACAGCTACTTCATCACGGAGGAGAACGTGGGCATGCTCAAGCTCGCCAAGGAGGCCAACAGCATCCTC
TTCGACTACCGCATCCCGCTGGCGGGCACCGACACTTGCAGCGCGCAGGCAGCGGCGGTGGAGGGCCAC
GGCAGCAATGGCATGGTGATGAACGGGCTGCCCATCAGCATCTACGCGCAGGAGACGGTCGGGTGCGCG
GTGGTGGACTCTAACGGCTTCACGGCAGCGGCCACCTCGACCGGCGGGCTCATGAACAAGATGACGGGC
CGCATCGGCGACTCGCCCCTCATCGGCGCCGGCACCTACGCGTGCGGGCACTGCGCTGTGTCGTGCACC
GGCGAGGGCGAGGCCATCATCCGCTCCACGCTGGCGCGGGACGTGGCGGCCGTCATGGAGTACAAGGGC
CTCCCGCTGCAGGAGGCCGTGGACTTCTGCGTCAAGGAGCGGCTGGACGAGGGGTTCGCGGGGCTCATC
GCCGTGTCCGGCACCGGCGAGGTGGCGTACGGGTTCAACTGCACCGGCATGTTCAGGGGCTGCGCCACC
GAGGACGGCTTCATGGAGGTCGGCATCTGGGATTGA

SEQ ID NO: 16 (wheat asparaginase)

MARWAIAIHGGAGVDPNLPEHRQEEAKRVLARCLQVGVDLLRAGATALDVVEAVVRELETDPCFNSGRG
SALTRAGTVEMEASIMDGRGRRCGAVSGVSTVKNPVSLARRVMDKSPHSYLAFDGAEDFAREQGLEVVD
NSYFITEENVGMLKLAKEANSILFDYRIPLAGTDTCSAQAAAVEGHGSNGMVMNGLPISIYAQETVGCA
VVDSNGFTAAATSTGGLMNKMTGRIGDSPLIGAGTYACGHCAVSCTGEGEAIIRSTLARDVAAVMEYKG
LPLQEAVDFCVKERLDEGFAGLIAVSGTGEVAYGFNCTGMFRGCATEDGFMEVGIWD

SEQ ID NO: 17 (wheat puroindoline promoter)

CCTCGTCCACCTCCTAAGTTGGACCTCCGTGCGAGCGTTGCGGGCCGCCGCTCGGATGGCTTCTGGA
AGGCCGTTGTACATGGGCACAGGGTGAATCCCGACGAGAGCTGGCCATTGGCGCGCGCAAGTAGACGAC
GACGTCCCCGACGAAGAGCTGCCCGGCCAGCTCGCCTCACTCGCCTTTGATTAGAAATGAAAGATTGGG
AAGAAGCAGACTTGAGCCTGCAGGGAAAGGATAAGGTGACGATGCAGTCTCCACTCGTTCGGGGCGACA
CGCGTACTGAATGTATGAGAAACGTGACGTGGCAGAGACCCCAAGATACTCCCTCCATTTATTTTTAGT
CTGCATATAAAGTTTGGTCAAAGTCAAGATTTGTAAATTTTAACTAACTTTATAATAAAAAAATATCAA
CATTCACAATATGAAATAATTATTACCAGATGCGTGAAGAAATGTATTTCCATACTATATAGCCTTGGT
ATTGGAGATGTTCATATTTTTTTATATAAATGGGAAGTAGAGGCACTCTTCCATATAATGAAGTTTATA
ATATATGTGCTTATATTGTACTATAATTGTTTGAATAACTTAGCATATGTTCAGATGTATGATATCTGT
AATTTAAGCGCTTGAATTTTACATATAAATATTTATTAATAAATATGTACCCCTATAATAGCTAGGCCG
TGCAGTTGCACGGGTAGATGACTAGTGATTACAATCTTGTTTGTGTGCAAGTCAAGCTTATCTAGTTTA
CACGTAACAACTTGTAGAACATTACAAAATTTATGCTTGCTAATAACTTCTAGAACACTACAACACTTG
ACATGTAAAAGGAATTTGACGAGTCATGGCCTACTAAAGCAAGTTACATTACTAGTCTTATCTATCTTA
ACAGACCACACAAGATTACAAACTAAGTACCGTGCCAGCCATACTTATCTAGTTTATGCGTAACAATTT
GCAGAAAATTAGAAACTTAGTTTCAGAAAAATACGCAATCTAGATTAGTGTTTGAGCTGTAAAGTGAAT
AAGATGAGTCATGCATGTTATCACACCTTTTTGGTGGTGGAATGATAGTGCAACAACAAGGAACTTTAA
TGACCAGTCCAAGAATACACTTGTAAGTAGTGCCACCAAACAGAACATTCCAAATGATGATTTTTAGAA
GCATCCAAGCACTTTCCACACAAACAAATGCCAATTGTGAAAGAGATCATTCCATGGCAGCTATAAATA
GCCCCATAGCATGACGATCATCCTTCCTCATCCATCATTCTCATTAGTAGAGCGCATCATTTAAGCCAA
GCAAGCTGTGGTCAATACAAATCC

SEQ ID NO: 18 (PPO)

TTAGTCTCTATTGAATCTGCTGAGATTACACTTTGATGGATGATGCTCTGTTTTTGTTTTCTTGTTCTG
TTTTTTCCTCTGTTGAAATCAGCTTTGTTGCTTGATTTCATTGAAGTTGTTATTCAAGAATAAATCAGT
TACAATTATGTTTGGG

SEQ ID NO: 19 (R1)

ACCTTATTTCACTACCACTTTCCACTCTCCAATCCCCATACTCTCTGCTCCAATCTTCATTTGCTTCG
TGAATTCATCTTCATCGAATTTCTCGACGCTTCTTCGCTAATTTCCTCGTTACTTCACTAAAAATCGAC
GTTTCTAGCTGAACTTGAGTGAATTAAGCCAGTGGGAGGAT

SEQ ID NO: 20 (PhL)

TTAGAGTGTGGGTAAGTAATTAAGTTAGGGATTTGTGGGAAATGGACAAATATAAGAGAGTGCAGGGGA
GTAGTGCAGGAGATTTTCGTGCTTTTATTGATAAATAAAAAAAGGGTGACATTTAATTTCCACAAGAGG
ACGCAACACAACACACTTAATTCCTGTGTGTGAATCAATAATTGACTTCTCCAATCTTCATCAATAAAA
TAATTCACAATCCTCACTCTCTTATCACTCTCATTCGAAAAGCTAGATTTGCATAGAGAGCACAAA

SEQ ID NO: 21 (P:ubi7)

TCGAGCACATTGATTGAGTTTTATATGCAATATAGTAATAATAATAATATTTCTTATAAAGCAAGAGGT
CAATTTTTTTTTATTATACCAACGTCACTAAATTATATTTGATAATGTAAAACAATTCAATTTTACTTA
AATATCATGAAATAAACTATTTTTATAACCAAATTACTAAATTTTTCCAATAAAAAAAAGTCATTAAGA
AGACATAAAATAAATTTGAGTAAAAAGAGTGAAGTCGACTGACTTTTTTTTTTTTATCATAAGAAAATA
AATTATTAACTTTAACCTAATAAAACACTAATATAATTTCATGGAATCTAATACTTACCTCTTAGAAAT
AAGAAAAAGTGTTTCTAATAGACCCTCAATTTACATTAAATATTTTCAATCAAATTTAAATAACAAATA
TCAATATGAGGTCAATAACAATATCAAAATAATATGAAAAAAGAGCAATACATAATATAAGAAAGAAGA
TTTAAGTGCGATTATCAAGGTAGTATTATATCCTAATTTGCTAATATTTAAACTCTTATATTTAAGGTC
ATGTTCATGATAAACTTGAAATGCGCTATATTAGAGCATATATTAAAATAAAAAAATACCTAAAATAAA
ATTAAGTTATTTTTAGTATATATTTTTTTACATGACCTACATTTTTCTGGGTTTTTCTAAAGGAGCGTG
TAAGTGTCGACCTCATTCTCCTAATTTTCCCCACCACATAAAAATTAAAAAGGAAAGGTAGCTTTTGCG
TGTTGTTTTGGTACACTACACCTCATTATTACACGTGTCCTCATATAATTGGTTAACCCTATGAGGCGG
TTTCGTCTAGAGTCGGCCATGCCATCTATAAAATGAAGCTTTCTGCACCTCATTTTTTTCATCTTCTAT
CTGATTTCTATTATAATTTCTCTCAATTGCCTTCAAATTTCTCTTTAAGGTTAGAAATCTTCTCTATTT
TTGGTTTTTGTCTGTTTAGATTCTCGAATTAGCTAATCAGGTGCTGTTATAGCCCTTAATTTTGAGTTT
TTTTTCGGTTGTCTTGATGGAAAAGGCCTAAAATTTGAGTTTTTTTACGTTGGTTTGATGGAAAAGGCC
TACAATTGGAGTTTTCCCCGTTGTTTTGATGAAAAAGCCCCTAGTTTGAGATTTTTTTCTGTCGATTC
GATTCTAAAGGTTTAAAATTAGAGTTTTTACATTTGTTTGATGAAAAAGGCCTTAAATTTGAGTTTTTC
CGGTTGATTTGATGAAAAAGCCCTAGAATTTGTGTTTTTTCGTCGGTTTGATTCTGAAGGCCTAAAATT
TGAGTTTCTCCGGCTGTTTTGATGAAAAAGCCCTAAATTTGAGTTTCTCCGGCTGTTTTGATGAAAAAG
CCCTAAATTTGAGTTTTTTCCCCGTGTTTTAGATTGTTTGGTTTTAATTCTCGAATCAGCTAATCAGGG
AGTGTGAAAAGCCCTAAATTTGAGTTTTTTCGTTGTTCTGATTGTTGTTTTTATGAATTTGCAGATGC
AGATCTTTGTGAAAACTCTCACCGGAAAGACTATCACCCTAGAGGTGGAAAGTTCTGATACAATCGACA
ACGTTAAGGCTAAGATCCAGGATAAGGAAGGAATTCCCCCGGATCAGCAAAGGCTTATCTTCGCCGGAA
AGCAGTTGGAGGACGGACGTACTCTAGCTGATTACAACATCCAGAAGGAGTCTACCCTCCATTTGGTGC
TCCGTCTACGTGGAGGTG

SEQ ID NO: 22 (P:patatin)

ATCTCGAGCCGATCTTACTTTTATTGGCTTTGTTTATTATCATTTTTCACACTCTGTGGTTCAGTAAT
TGACCGGAGACTATACCATAGAAGACCTAATCACAACTTAGTCTTCTTTTTATTTTTCTTTATTTAG
AAGACCAATTGTTAAAAATATGAACTTGGTACTATTTCTAAGGTTTGTTTTTATGTTCTTTTGTTCATT
TTGCACTTATAATTTTACTGAATTGCAGTTTTTACATTATGTTTTAATAGTTAGCAGTTTCATGAATGA
TGAAGTTTATGTTGCCATATAGAGTAGTTTGTGATGATATACTTCATAAACTTTCACTTATGTTAAATT
TGTAATGATAAAATTTAATTATATTGTAAATCAAAAATTACTTATAAAATTGGGCATTAAAACATATGA
AAGACAAATTGTGTTACATATTTTACTTTTGACTCCAATATGAATATCTCAATTTAAATCTTTGTTTTA
TTTTCTCTTTCTCTTTACAGGTATAAAAGGTGAGAATTGAAGCAAGATTGATTGCAGGCTATGTGTCAC
CACATTATTGATACGTTGGAAGGAATTTTTACTTATATGTCTTTGTGTAGGAGTAATTTTGATATATT
TTAGTTAGATTTTTTTTTCATTGGACATATTTTACTTTTATTTAAGGAATTTGTAATGAGATATTATT
CTTTAGTATAATTTAAGTTATTTTTATTATATGATCATGGATGAATTTTGATACAAATATTTTTGTCAT
TAAATAAATTAATTCATCACAACTTGATTACTTTCAGTGACAAAAAATGTATTATCGTAGTACCCTTTA
TTGTTAAATATGAATACTTTTTATTTTTATTTTGTGACAATTGTAATTGTCACTACTTATGATAATATT
TAGTGACAATATATGTCGTTGGTAAAAGCAACACTTTTAGTGACAAAATGATAAATTTAATCACAAAAT
TATTAACCTTTTTTATAATAATAAATTTGTCCCTAATTTATACATTTAAGGACAAAATATTTTTTTGTA
AATAAAAATAGTCTTTAGTGACAATATTATATCTTTTCAACTACGAAATACATACAACTTTAGAGACAA
TTGATGTTGTCCCTGATTGAACTAAATAATTAGCGACGATATAGTTTTGTCGGTTGTAATAACCTTTTT
AGTGACAAAACATACTATTAACTACAAAAAAAGTTACACATTTTATGACAAATAATAAATTCATCACAA
ATGTTTATGCATTTGGGGACGATTTTTCTTTTTGTAGTTAATGCGTATTAGTTTTAGCGACGAAGCACT
AAATCGTTTTTGTATACTTTGAGTGACACACGTTTAGTGACGACTGATTGACGAAATTTTTTTGTCTCA
CAAAATTTTTAGTGACGAAACATGATTTATAGATGACGAAATTATTTGTCCCTCATAATCTAATTTGTT
GTAGTGATCATTACTCCTTTGTTTGTTTTATTTGTCATGTTAGTTCATTAAAAAAAAAATCTCTCTTCT
TATCAATTCTAACGTGTTTAATATCATAAGATTAAAAAATATTTTAATATATCTTTAATTTAAACCCAC
AAAGTTTAAATTTCTTCGTTAACTTAATTTGTCAAATCAGGCTCAAAGATTGTTTTTCATATCGGAATG
AGGATTTTATTTATTCTTTTAAAAATAAAGAGGTGTTGAGCTAAACAATTTCAAATCTCATCTCACATA
TGGGGTCAGCCACAAAAATAAAGAACGGTTGGAACGGATCTATTATATAATACTAATAAAGAATAGAAA
AAGGAAAGTGAGTGAGGTACGAGGGAGAGAATCTGTTTAATATCAGAGTCGATCATGTGTCAGTTTTAT
TGATATGACTTTGACTTCAACTGAGTTTAAGCAATTTTGATAAGGCGAGGAAAATCACAGTGCTGAATC
TAGAAAAATCTCATACAGTGTGAGATAAATCTCAACAAAAACGTTGAGTCCATAGAGGGGGTGTATGTG
ACACCCAACCTCAGCAAAAGAAAACCTCCCCTCAAGAAGGACATTTGCGGTGCTAAACAATTTCAAGTC
TCATCACACATATATATTTATATAATACTAATAAAGAATAGAAAAAGGAAAGGTAAACATCACTAACGAC
AGTTGCGGTGCAAAGAGAGTGAGGTAATAAACATCACTAACTTTTATTGGTTATGTCAAACTCAAAGTA
AAATTTCTCAACTTGTTTACGTGCCTATATATACCATGCTTGTTATATGCTCAAAGCACCAACAAAATT
TAAAAACAATTTGAACATTTGCAAAGGTACCGA

SEQ ID NO: 23

GGTACCAAGTGTCTGAGACAACCAAAACTGAAAGTGGAAACCAAACTCTAAGTCAAAGACTTTATATA
CAAAATGGTATAAATATAATTATTTAATTTACTATCGGGTTATCGATTAACCCGTTAAGAAAAAACTTC
AAACCGTTAAGAACCGATAACCCGATAACAAAAAAAATCTAAATCGTTATCAAAACCGCTAAACTAATA
ACCCAATATTGATAAACCAATAACTTTTTTATTCGGGTTATCGGTTTCAGTTCTGTTTGGAACAATCC
TAGTGTCCTAATTATTGTTTTGAGAACCAAGAAAACAAAAACTTACGTCGCAAATATTTCAGTAAATAC
TTGTATATCTCAGTGATAATTGATTTCCAACATGTATAATTATCATTTACGTAATAATAGATGGTTTCC
GAAACTTACGCTTCCCTTTTTTCTTTTGCAGTCGTATGGAATAAAAGTTGGATATGGAGGCATTCCCGG
GCCTTCAGGTGGAAGAGACGGAGCTGCTTCACAAGGAGGGGTTGTTGTACTTGAAAATGGGCATTTAT
TGTTCGCAAACCTATCATGTTCCTATGGTTGTTTATTTGTAGTTTGGTGTTCTTAATATCGAGTGTTCT
TTAGTTTGTTCCTTTTAATGAAAGGATAATATCTGTGCAAAAATAAGTAAATTCGGTACATAAAGACAT
TTTTTTTTGCATTTTCTGTTTATGGAGTTGTCAAATGTGAATTTATTTCATAGCATGTGAGTTTCCTCT
CCTTTTTCATGTGCCCTTGGGCCTTGCATGTTTCTTGCACCGCAGTGTGCCAGGGCTGTCGGCAGATGG
ACATAAATGGCACACCGCTCGGCTCGTGGAAAGAGTATGGTCAGTTTCATTGATAAGTATTTACTCGTA
TTCGGTGTTTACATCAAGTTAATATGTTCAAACACATGTGATATCATACATCCATTAGTTAAGTATAAA
TGCCAACTTTTTACTTGAATCGCCGAATAAATTTACTTACGTCCAATATTTAGTTTTGTGTGTCAAACA
TATCATGCACTATTTGATTAAGAATAAATAAACGATGTGTAATTTGAAAACCAATTAGAAAAGAAGTAT
GACGGGATTGATGTTCTGTGAAATCACTGGTAAATTGGACGGACGATGAAATTTGATCGTCCATTTAAG
CATAGCAACATGGGTCTTTAGTCATCATCATTATGTTATAATTATTTCTTGAAACTTGATACACCAAC
TTTCATTGGGAAAGTGACAGCATAGTATAAACTATAATATCAATTCTGGCAATTTCGAATTATTCCAAA
TCTCTTTTGTCATTTCATTTCCTCCCCTATGTCTGCAAGTACCAATTATTTAAGTACAAAAAATCTTGA
TTAAACAATTTATTTTCTCACTAATAATCACATTTAATCATCAACGGTTCATACACGTCTGTCACTCTT
TTTTTATTCTCTCAAGCGCATGTGATCATACCAATTATTTAAATACAAAAAATCTTGATTAAACAATTC
AGTTTCTCACTAATAATCACATTTAATCATCAACGGTTCATACACATCCGTCACTCTTTTTTTATTCTC
TCAAGCGCATGTGATCATACCAATTATTTAAATACAAAAAATCTTGATTAAACAATTCATTTTCTCACT
AATAATCACATTTAATCATCAACGGTTTATACACGTCCGCCACTCTTTTTTTATTCTCTCAAGCGTATG
TGATCATATCTAACTCTCGTGCAAACAAGTGAAATGACGTTCACTAATAAATAATCTTTTGAATACTTT
GTTCAGTTTAATTTATTTAATTTGATAAGAATTTTTTTATTATTGAATTTTTATTGTTTTAAATTAAAA
ATAAGTTAAATATATCAAAATATCTTTTAATTTTATTTTTGAAAAATAACGTAGTTCAAACAAATTAAA
ATTGAGTAACTGTTTTTCGAAAAATAATGATTCTAATAGTATATTCTTTTTCATCATTAGATATTTTTT
TTAAGCTAAGTACAAAAGTCATATTTCAATCCCCAAAATAGCCTCAATCACAAGAAATGCTTAAATCCC
CAAAATACCCTCAATCACAAGACGTGTGTACCAATCATACCTATGGTCCTCTCGTAAATTCCGACAAAA
TCAGGTCTATAAAGTTACCCTTGATATCAGTATTATAAAACTAAAAATCTCAGCTGTAATTCAAGTGCA
ATCACACTCTACCACACACTCTCTAGTAGAGAGATCAGTTGATAACAAGCTTGTTAACGGATCCCTAGT
AATACTGAGATTAGTTACCTGAGACTATTTCCTATCTTCTGTTTTGATTTGATTTATTAAGGAAAATTA
TGTTTCAACGGCCATGCTTATCCATGCATTATTAATGATCAATATATTACTAAATGCTATTACTATAGG
TTGCTTATATGTTCTGTAATACTGAATATGATGTATAACTAATACATACATTAAATTCTCTAATAAATC
TATCAACAGAAGCCTAAGAGATTAACAAATACTACTATTATCCAGACTAAGTTATTTTTCTGTTTACTA
CAGATCCTTCCAAGAACAAAAACTTAATAATTGTATGGCTGCTATACCATCAAACCAAACAATGTATAA
GAAATAATACTTGCATAACTAATGCACGCACTACTAATGCAAGCATTACTAATGCACCATATTTTGTAT
TTGTTCTTATACACTCTACCAAACGACCCCTTAGAGTGTGGGTAAGTAATTAAGTTAGGGATTTGTGGG
AAATGGACAAATATAAGAGAGTGCAGGGGAGTAGTGCAGGAGATTTTCGTGCTTTTATTGATAAATAAA

AAAAGGGTGACATTTAATTTCCACAAGAGGACCGAACACAACACACTTAATTCCTGTGTGTGAATCAAT
AATTGACTTCTCCAATCTTCATCAATAAAATAATTCACAATCCTCACTCTCAAAATTCTTATGTTAACC
AAATAAATTGAGACAAATTAATTCAGTTAACCAGAGTTAAGAGTAAAGTACTATTGCAAGAAAATATCA
AAGGCAAAAGAAAAGATCATGAAAGAAAATATCAAAGAAAAAGAAGAGGTTACAATCAAACTCCCATAA
AACTCCAAAAATAAACATTCAAATTGCAAAAACATCCAATCAAATTGCTCTACTTCACGGGGCCCACGC
CGGCTGCATCTCAAACTTTCCCACGTGACATCCCATAACAAATCACCACCGTAACCCTTCTCAAAACTC
GACACCTCACTCTTTTTCTCTATATTACAATAAAAAATATACGTGTCCGTGTATGGGTGATCCTTCTCT
TATTATACCGACTAAAGACATTGGTATTAAGGATATCTTATCTTTTGAGGAGATTCCCGTTCAGATTCT
GGAGCGTCAGGTTCGCAAGTTGAGAACCAATGAGGTAACATCAGTCAAGGTCTTATGGAGGAATCAGCC
ATGGGGACACGTATATTTTTTATTGTAATATAGAGAAAAAGAGTGAGGTGTCGAGTTTTGAGAAGGGTT
ACGGTGGTGATTTGTTATGGGATGTCACGTGGGAAAGTTTGAGATGCAGCCGGCGTGGGCCCCGTGAAG
TAGAGCAATTTGATTGGATGTTTTTGCAATTTGAATGTTTATTTTTGGAGTTTTATGGGAGTTTGATTG
TAACCTCTTCTTTTTCTTTGATATTTTCTTTCATGATCTTTTCTTTTGCCTTTGATATTTTCTTGCAAT
AGTACTTTACTCTTAACTCTGGTTAACTGAATTAATTTGTCTCAATTTATTTGGTTAACATAAGAATTT
TGAGAGTGAGGATTGTGAATTATTTTATTGATGAAGATTGGAGAAGTCAATTATTGATTCACACACAGG
AATTAAGTGTGTTGTGTTCGGTCCTCTTGTGGAAATTAAATGTCACCCTTTTTTTATTTATCAATAAAA
GCACGAAAATCTCCTGCACTACTCCCCTGCACTCTCTTATATTTGTCCATTTCCCACAAATCCCTAACT
TAATTACTTACCCACACTCTAAGGGGTCGTTTGGTAGAGTGTATAAGAACAAATACAAAATATGGTGCA
TTAGTAATGCTTGCATTAGTAGTGCGTGCATTAGTTATGCAAGTATTATTTCTTATACATTGTTTGGTT
TGATGGTATAGCAGCCATACAATTATTAAGTTTTTGTTCTTGGAAGGATCTGTAGTAAACAGAAAAATA
ACTTAGTCTGGATAATAGTAGTATTTGTTAATCTCTTAGGCTTCTGTTGATAGATTTATTAGAGAATTT
AATGTATGTATTAGTTATACATCATATTCAGTATTACAGAACATATAAGCAACCTATAGTAATAGCATT
TAGTAATATATTGATCATTAATAATGCATGGATAAGCATGGCCGTTGAAACATAATTTTCCTTAATAAA
TCAAATCAAAACAGAAGATAGGAAATAGTCTCAGGTAACTAATCTCAGTATTACTAGCTTTAATGTTTA
GCAAATGTCCTATCAGTTTTCTCTTTTTGTCGAACGGTAATTTAGAGTTTTTTTGCTATATGGATTTT
CGTTTTTGATGTATGTGACAACCCTCGGGATTGTTGATTTATTTCAAAACTAAGAGTTTTTGCTTATTG
TTCTCGTCTATTTTGGATATCAATCTTAGTTTTATATCTTTTCTAGTTCTCTACGTGTTAAATGTTCAA
CACACTAGCAATTTGGCTGCAGCGTATGGATTATGGAACTATCAAGTCTGTGGGATCGATAAATATGCT
TCTCAGGAATTTGAGATTTTACAGTCTTTATGCTCATTGGGTTGAGTATAATATAGTAAAAAAATAGGA
ATTCGAACCATGCATCTCAATCTTAATACTAAAAAATGCAACAAAATTCTAGTGGAGGGACCAGTACCA
GTACATTAGATATTATCTTTTATTACTATAATAATATTTTAATTAACACGAGACATAGGAATGTCAAGT
GGTAGCGGTAGGAGGGAGTTGGTTCAGTTTTTAGATACTAGGAGACAGAACCGGAGGGGCCCATTGCA
AGGCCCAAGTTGAAGTCCAGCCGTGAATCAACAAAGAGAGGGCCCATAATACTGTCGATGAGCATTTCC
CTATAATACAGTGTCCACAGTTGCCTTCCGCTAAGGGATAGCCACCCGCTATTCTCTTGACACGTGTCA
CTGAAACCTGCTACAAATAAGGCAGGCACCTCCTCATTCTCACACTCACTCACTCACACAGCTCAACAA
GTGGTAACTTTTACTCATCTCCTCCAATTATTTCTGATTTCATGCATGTTTCCCTACATTCTATTATGA
ATCGTGTTATGGTGTATAAACGTTGTTTCATATCTCATCTCATCTATTCTGATTTTGATTCTCTTGCCT
ACTGAATTTGACCCTACTGTAATCGGTGATAAATGTGAATGCTTCCTCTTCTTCTTCTTCTCAGAA
ATCAATTTCTGTTTTGTTTTTGTTCATCTGTAGCTTGGTAGATTCCCCTTTTTGTAGACCACACATCAC
CCGCGGTCATATGGTGTAGAAGGAATGGTTTCCGAAAACCATGGTGGGTTGTACCATCTTCTAAGTCCT
CCATTTTTGCTTGAATAAATATGACCGGGAAGGAAGCTAACAAATCGTTCACAATCATCACTTAAGGCT

TTCATCTCTGAGGAAAACCATATGGAGCCATCAAGACCCCACCCCATATAAAGGGGTGTAATGCCAATG
GCATCCCGAGCAGCGATGAAACTTTTATCCCGGGTATCAAGAAGAACAAAAGAGAACATCCCATCCAAC
ATGTCAATGAAGTTTTCTCCATATTCTTCATAAAGATGGGCAATAACTTCACAATCACTTTCAGTTCGA
AACTGATGAGAAAATTCATTGGGCTATACCTTGCTTTATATAATATGTAAAACAGTATAATTATTCAAT
TTTAGAGGCGGCTTCCATATCAATTATTCCAGGAAGCGGTAGGTGGGGGAACTCTTTATCCAGAAATGG
TACTCTAGCTTCTAAGCCCCACGCGGATGTAGCCTTGTTTGCTCTTAAACAGTCATACTGGTGAAGCGC
TTTTATCTTGCGACATGTTTCCGTGTGGAACTCTTCCTTGTTGGGAGCCTTGTGGAAGTACAAGTAACC
ACCAAAAATTTCGTCAGCGCCTTCCCCTGATATGACCATCTTCACTCCTAGTGATTTAATCTTACGCGA
CATAAGGAACATAGGAGTGCTGGCTCTTATTGTTGTTACATCATACGTCTCGATATGATATATAACATC
TTCAATAGCATCAATACCGTCCTGAACAGTAAAGTGAAACTCATGGTGAACGGTTCCTAAAAAGTCAGC
AACTTCTTTTGCAGCCTTGAGATCTGGTGAGCCCTCGAGACTGCAGCACTTTGGTTCTGAGGCGTGCCT
TCGAAAATGCTGTTATCAAACGGTTGATGACTGATGTCCCCTTTGGCGTTCTGCTCTCGGGGGGACTTG
ATTCGTCTTTGGTTGCTTCTGTCACTACTCGATACTTGGCTGGAACAAAAGCTGCTAAGCAATGGGGAG
CACAACTTCATTCCTTCTGTGTTGGTCTCGAGGGCTCACCAGATCTCAAGGCTGCAAAAGAAGTTGCTG
ACTTTTTAGGAACCGTTCACCATGAGTTTCACTTTACTGTTCAGGACGGTATTGATGCTATTGAAGATG
TTATATATCATATCGAGACGTATGATGTAACAACAATAAGAGCCAGCACTCCTATGTTCCTTATGTCGC
GTAAGATTAAATCACTAGGAGTGAAGATGGTCATATCAGGGGAAGGCGCTGACGAAATTTTTGGTGGTT
ACTTGTACTTCCACAAGGCTCCCAACAAGGAAGAGTTCCACACGGAAACATGTCGCAAGATAAAAGCGC
TTCACCAGTATGACTGTTTAAGAGCAAACAAGGCTACATCCGCGTGGGGCTTAGAAGCTAGAGTACCAT
TTCTGGATAAAGAGTTCCCCCACCTACCGCTTCCTGGAATAATTGATATGGAAGCCGCCTCTAAAATTG
AATAATTATACTGTTTTACATATTATATAAAGCAAGGTATAGCCCAATGAATTTTCTCATCAGTTTCGA
ACTGAAAGTGATTGTGAAGTTATTGCCCATCTTTATGAAGAATATGGAGAAAACTTCATTGACATGTTG
GATGGGATGTTCTCTTTTGTTCTTCTTGATACCCGGGATAAAAGTTTCATCGCTGCTCGGGATGCCATT
GGCATTACACCCCTTTATATGGGGTGGGGTCTTGATGGCTCCATATGGTTTTCCTCAGAGATGAAAGCC
TTAAGTGATGATTGTGAACGATTTGTTAGCTTCCTTCCCGGTCATATTTATTCAAGCAAAAATGGAGGA
CTTAGAAGATGGTACAACCCACCATGGTTTTCGGAAACCATTCCTTCTACACCATATGAGTGGGAGATT
CTCTAACCGACAACCACCACTATGAGCCTAAGTGGTGATACAGTGTCTTGTCCACGCTGCCAGAACTGT
CCTATACTTTGCCGTCATATAGAATGCTTAACTTAGTGGATCGACCAGTCTATGCTATCTAGAGTGATG
TGTGGTCTACAAAAAGGGGAATCTACCAAGCTACAGATGAACAAAAACAAAACAGAAATTGATTTCTGA
GAAGAAGAAGAAGAAGAGGAAGCATTCACATTTATCACCGATTACAGTAGGGTCAAATTCAGTAGGCAA
GAGAATCAAAATCAGAATAGATGAGATGAGATATGAAACAACGTTTATACACCATAACACGATTCATAA
TAGAATGTAGGGAAACATGCATGAAATCAGAAATAATTGGAGGAGATGAGTAAAAGTTACCACTTGTTG
AGCTGTGTGAGTGAGTGAGTGTGAGAATGAGGAGGTGCCTGCCTTATTTGTAGCAGGTTTCAGTGACAC
GTGTCAAGAGAATAGCGGGTGGCTATCCCTTAGCGGAAGGCAACTGTGGACACTGTATTATAGGGAAAT
GCTCATCGACAGTATTATGGGCCCTCTCTTTGTTGATTCACGGCTGGACTTCAACTTGGGCCTTGCAAT
GGGCCCCTCCGGTTCTGTCTCCTAGTATCTAAAAAACTGAACCAACTCCCTCCTACCGCTACCACTTGA
CATTCCTATGTCTCGTGTTAATTAAAATATTATTATAGTAATAAAAGATAATATCTAATGTACTGGTAC
TGGTCCCTCCACTAGAATTTTGTTGCATTTTTAGTATTAAGATTGAGATGCATGGTTCGAGCTC

SEQ ID NO.: 24 (P:PPO)

CTAGTAATACTGAGATTAGTTACCTGAGACTATTTCCTATCTTCTGTTTTGATTTGATTTATTAAGGAA
AATTATGTTTCAACGGCCATGCTTATCCATGCATTATTAATGATCAATATATTACTAAATGCTATTACT
ATAGGTTGCTTATATGTTCTGTAATACTGAATATGATGTATAACTAATACATACATTAAATTCTCTAAT
AAATCTATCAACAGAAGCCTAAGAGATTAACAAATACTACTATTATCCAGACTAAGTTATTTTTCTGTT
TACTACAGATCCTTCCAAGAACAAAAACTTAATAATTGTATGGCTGCTATAC

SEQ ID NO.: 25 (P:PHL)

CATCAAACCAAACAATGTATAAGAAATAATACTTGCATAACTAATGCACGCACTACTAATGCAAGCATT
ACTAATGCACCATATTTTGTATTTGTTCTTATACACTCTACCAAACGACCCCTTAGAGTGTGGGTAAGT
AATTAAGTTAGGGATTTGTGGAAATGGACAAATATAAGAGAGTGCAGGGGAGTAGTGCAGGAGATTTT
CGTGCTTTTATTGATAAATAAAAAAAGGGTGACATTTAATTTCCACAAGAGGACCGAACACAACACACT
TAATTCCTGTGTGTGAATCAATAATTGACTTCTCCAATCTTCATCAATAAAATAATTCACAATCCTCAC
TCTC

SEQ ID NO.: 26 (P:R1)

AAAATTCTTATGTTAACCAAATAAATTGAGACAAATTAATTCAGTTAACCAGAGTTAAGAGTAAAGTAC
TATTGCAAGAAAATATCAAAGGCAAAAGAAAAGATCATGAAAGAAAATATCAAAGAAAAAGAAGAGGTT
ACAATCAAACTCCCATAAAACTCCAAAAATAAACATTCAAATTGCAAAAACATCCAATCAAATTGCTCT
ACTTCACGGGGCCCACGCCGGCTGCATCTCAAACTTTCCCACGTGACATCCCATAACAAATCACCACCG
TAACCCTTCTCAAAACTCGACACCTCACTCTTTTTCTCTATATTACAATAAAAAATATACGTGTCC

SEQ ID NO: 27 (G:PPO)

GAAAATTCATTGGGCTATACCTTGCTTTATATAATATGTAAAACAGTATAATTATTCAATTTTAGAGGC
GGCTTCCATATCAATTATTCCAGGAAGCGGTAGGTGGGG

SEQ ID NO: 28 (GS1)

GTTCATCTTCTTCTCTCACTTCTCTTAACAACATCTTTTCTGCATTGGCCACTTAGTTGGTTAGGAGGT
GAACATGGCTCAGATTCTGGCTCCATCTGCACAATGGCAGATGAGAATGACAAAGAGCTCAACAGATGC
TAGTCCCTTGACTTCAAAGATGTGGAGCTCTGTGGTGCTGAAGCAGAACAAAAGACTTGCTGTTAAAAG
CTCTGCCAAATTTAGAGTTTTTGCCCTCCAGTCTGACAATGGCACCGTGAACAGAATGGAACAGCTGCT
AAACTTGGACGTAACTCCATACACTGATAAGATCATTGCTGAATATATTTGGATCGGGGGACTGGAAT
TGATGTGCGCAGTAAATCAAGGACTATTTCAAAACCAGTCAAGGATGCTTCTGAGCTCCCAAAGTGGAA
CTACGATGGATCAAGTACTGGACAAGCACCTGGAGAAGACAGTGAAGTCATTCTATATCCTCAGGCAAT
ATTCAAAGACCCTTTCCGTGGTGGTAACAACATCTTGGTTATCTGTGATACCTACACACCAGCTGGAGA
GCCAATTCCTACAAACAAACGCCATAAAGCTGCTCAAATTTTTAGCGACCCAAAAGTTGCATCTCAAGT
TCCATGGTTTGGAATAGAACAAGAGTACACCTTACTCCAGCCAAATGTAAACTGGCCCTTAGGTTGGCC
TGTTGGAGGCTACCCTGGACCTCAGGGTCCTTACTACTGTGGTGCTGGAGTGGAAAAGTCATTTGGCCG
AGATATATCAGATGCTCACTACAAGGCTTGCCTGTATGCTGGAATTAACATTAGTGGTACTAACGGAGA
GGTTATGCCAGGACAGTGGGAATTTCAAGTAGGACCTAGTGTTGGAATTGAAGGTGGAGATCATATCTG
GTGTGCTAGATACCTCCTCGAGAGAATTACTGAACAAGCAGGAGTTGTCCTCTCACTCGATCCAAAACC
AATTGAGGGTGACTGGAACGGTGCAGGATGCCACACTAACTACAGTACACTGAGTATGAGAGAAGAGGG
AGGCTTTGAAGTGATAAAGAAAGCAATTCTTAATCTATCCCTTCGCCACAAGGAACATATAAGTGCTTA
TGGAGAAGGAAATGAGAGAAGGTTGACCGGAAAGCATGAAACTGCTAGTATTGACCAATTTTCATGGGG
AGTTGCTAACCGTGGTTGCTCAATCCGTGTGGGGCGTGACACTGAGAAGGAAGGCAAGGGTTATTTGGA
AGACCGCCGCCCAGCTTCAAACATGGACCCCTATGTTGTGACCGCATTACTTGCCGAAACTACTATACT
GTGGGAGCCAACCCTTGAGGCTGAAGCTCTTGCTGCCCAAAAGATCTCATTGAAGGTTTAGAGTAATTG
AGGGGAAATTGTTTTCATCATAATCCTCTTAGAATTTATGAGATAAGTGCTGAAGCTTGTACCTTGTTG
AGATTCCCTTATTTGGGAAATTCTTGTAAAGGAATCAAAATTTACCAGTTCATCCTAGAAAGAGGTTCC
TTAAGACATGAGACTACTTTGGAGTTGAGGTGTAATTGTTGGACTACTTTGAACATCTTTACCTTTCTT
TTCTCCAGATGAATCCATTTCTCTGAAATTCCAATTGGTGTGATTTTTCCGAATTAAATCTTTGAACAC
ATAATCAATCATGTACACTTACAGTTTCAAACTAGCTAGTTAAGTTACTTATATGATATTATCTTCTGT
CTGCTATGTTCAAGCTCAGGTTCTTTAGAGAATTCATCATAATATTTTATTCATGTTGGCCATCAATC
TGCCACGACTCTCGTCCTTTATCTGGATTTAAACTTGGTTGCTTTCCAACATATACATATTCATGTTAC
ATGCACTTGAATATATGTATCCAGCATGCCATTTTCCAGAACTTTGTACTTGATGTGCAAAGTACATGA
GGACTTCCAAGTGAGTAAAAGATGCATAATCTCATGTACAAGC

SEQ ID NO: 29 (GS2)

GATCAAATTAATTAAATTCTCTCATTCAACAAATTCAAACTTTGAATTATTATTATTTTCACATAATGG
CTCATCTTTCAGATCTTGTCAATCTCAATCTCTCTGATTCCTCTGACAAAATCATTGCTGAATACATAT
GGATTGGTGGATCAGGAATGGATGTAAGGAGCAAAGCCAGGACTCTATCTGGTCCTGTTGATGATCCTT
CAAAGCTTCCCAAATGGAATTATGATGGTTCTAGCACAGGTCAAGCTCCTGGAGAAGACAGTGAAGTGA
TCCTATATCCTCAAGCAATTTTCAAGGATCCATTCAGGAGGGGCAACAATATCCTGGTCATCTGTGATT
GTTACACCCCAGCTGGTGAACCAATTCCAACAAACAAGAGGCACAATGCTGCTAAAATATTTAGCAACC
CTGATGTTGTTGTTGAGGAACCATGGTATGGTCTTGAGCAAGAATACACCTTGCTACAAAAGGAAATTA

ACTGGCCTCTTGGATGGCCTATTGGTGGTTTTCCTGGACCACAGGGACCATACTACTGTGGAATTGGAT
CTGGAAAGGCTTTTGGACGCGATATTGTTGATGCTCATTACAAGGCATGTATCTATGCCGGGATTAACA
TTAGTGGTATCAACGGAGAAGTGATGCCTGGACAGTGGGAATTTCAAGTTGGTCCTTCAGTTGGCATTG
CATCAGGTGACGAGTTGTGGGCAGCTCGTTACATTCTCGAGAGGATTACAGAGATTGCTGGAGTTGTCG
TGTCATTCGACCCCAAACCTATTCCGGGCGACTGGAATGGTGCAGGAGCGCATACAAATTACAGTACCA
AGTCCATGAGGAATGAGGGAGGGTATGAAGTTATCAAGAAGGCTATTGAGAAGCTTGGACTTAGGCACA
AGGAGCACATTGCAGCATATGGTGAAGGCAATGAACGTCGTCTCACTGGAAGACACGAAACAGCTGACA
TCAACACGTTCAAATGGGGTGTTGCAAATCGTGGTGCATCCATTCGTGTGGGAAGAGACACGGAGAAGG
AAGGCAAGGGATACTTTGAGGACAGGAGGCCTGCATCGAACATGGATCCATACATCGTGACCTCTATGA
TCGCGGAGACTACCCTCCTGTGGAACCCTTGAACGCGTATGGATGAATATTCTCGGGTGCAACATATG
GAGAAAGAATTGAATTTCTTAACAGCCCTTTCCTCACATGTCCTTAAGAGAGTTATGTAGCTAGTAATT
TTGATATATTATGTTGTTTTCTAAGTTTCAATTTGTATTGTACTCAGCAAGCCTGAGTTCATTGCCAAA
ATGATTTGGCAATGTTGTTAAAAATAAGAGTTTTAATCTTATTAATAACAATATGGAAGGGTTTAACTT

SEQ ID NO: 30 (GS3)

GATCTAATAGAGAATTTCAATTTCAAGAAGTTATCATCATGTCTCTGCTTTCAGATCTTATCAACCTCA
ATCTCTCAGATGATACTCAGAAGATCATTGCTGAATACATATGGATTGGTGGATCAGGCATGGACATGA
GGAGCAAAGCCAGGACTCTCCCTGGTCCAGTTACTAGTCCTGCAGAACTACCCAAATGGAACTATGATG
GATCAAGCACAGGTCAAGCTCCTGGAGAAGACAGTGAAGTGATCATATACCCACAAGCAATCTTCAAGG
ATCCATTCAGGAGAGGCAACAATATCTTGGTCATGTGTGATGCCTATACTCCTGCTGGTGAGCCCATCC
CAACAAACAAGAGGCACGCCGCTGCCAAGGTCTTCTGCCACCCTGATGTGGCTGCTGAGGAAACTTGGT
ATGGTATTGAACAAGAATATACCTTGCTGCAAAAGGAGGTCAACTGGCCTCTTGGATGGCCCATTGGCG
GTTTTCCTGGACCCCAGGGACCATACTACTGTGGAACTGGAGCTGACAAGGCCTTTGGACGTGACATTG
TGGACGCCCATTACAAGGCATGTCTCTATGCTGGGATTAATATCAGCGGAATCAATGGTGAAGTCATGC
CGGGACAGTGGGAATTCCAAGTGGGACCTTCTGTTGGCATCTCAGCCGGTGATGAAGTGTGGGTAGCTC
GTTACATTCTAGAGAGGATTGCAGAGATTGCTGGGGTGGTCGTGTCATTCGACCCCAAGCCTATTCCGG
GCGACTGGAACGGCGCAGGTGCTCACACAAATTACAGCACCAAGTCGATGAGGGAAGACGGAGGCTATA
AAATAATCTTGAAGGCTATTGAGAAGCTTGGCCTGAAGCACAAAGAACACATTGCTGCATATGGTGAAG
GCAATGAGCGTCGTCTCACTGGAAAGCACGAAACAGCCAACATCAACACCTTCAAATGGGGGGTTGCAA
ACCGTGGTGCATCTGTCCGTGTTGGAAGAGACACAGAGAAGGCAGGCAAGGGATACTTTGAGGACAGAA
GGCCAGCCTCAAATATGGACCCATACGTCGTTACCTCCATGATCGCAGAAACCACCATCATCGGTTAAC
CTTGAAGACATTTTACTATGGATGGCTCGGGGGATCGCTTGTTTCTGGTTTGCACAATTTGGGATAGGA
GAAAAGATTGAATTGTGAAACGACCCTTTCGACTTCACCTGTGTTAATTTTTAGTTATAGGGGTAGATT
GTCTCTTGTTATTTTTCTGTTTATTTGCCAGTTGAATTGTATTTTCATACAGCAAGGCCTTATACATTG
TCTATGATTTGGCAATGCTGTGTTACAAAACAATGTTATTCTTATTAATAACAAAGATAATGAAAGGGT
TTGATTCTATTGCTCATTGCACT

SEQ ID NO: 31 (Asparaginase gene from E. coli)

ATGGGCAAAGCAGTCATTGCAATTCATGGTGGCGCAGGTGCAATTAGCCGCGCGCAGATGAGTCTGCAA
C
AGGAATTACGCTACATCGAGGCGTTGTCTGCCATTGTTGAAACCGGGCAGAAAATGCTGGAAGCGGGCG
A
AAGTGCGCTGGATGTGGTGACGGAAGCGGTGCGTCTGCTGGAAGAGTGTCCACTGTTTAACGCCGGAAT
T
GGCGCCGTCTTTACGCGTGATGAAACGCATGAACTGGACGCCTGTGTGATGGATGGTAACACCCTGAAA
G
CCGGTGCGGTGGCGGGCGTTAGTCATCTGCGTAATCCGGTTCTTGCCGCCCGGCTGGTGATGGAGTAAA
G
CCCGCATGTGATGATGATTGGCGAAGGGGCAGAAAATTTTGCGTTTGCTCATGGCATGGAGCGCGTCTC
A
CCGGAGATTTTCTCCACGCCTTTGCGTTATGAACAACTAATGGCAGCGCGCGAGGAAGGGGCAACAGTC
C
TCGACCATAGCGGTGCGCCACTGGATGAAAAACAGAAAATGGGCACCGTGGGGGCCGTGGCGTTGGATT
T
AGACGGCAATCTGGCGGCAGCCACGTCCACGGGCGGAATGACCAATAAATTACCCGGACGAGTTGGCGA
T
AGCCCCTTAGTGGGTGCCGGATGCTACGCCAATAACGCCAGTGTGGCGGTTTCTTGTACCGGCACGGGC
G
AAGTCTTCATCCGCGCGCTGGCGGCATATGACATCGCCGCGTTAATGGATTACGGCGGATTAAGTCTCG
C
GGAAGCCTGCGAGCGGGTAGTAATGGAAAAACTCCCTGCGCTTGGCGGTAGCGGTGGCTTAATCGCTAT
C
GACCATGAAGGGAATGTCGCGCTACCGTTTAACACCGAAGGAATGTATCGCGCCTGGGGCTACGCAGGC
G
ATACGCCAACCACCGGTATCTACCGTGAAAAAGGGGACACCGTTGCCACACAGTGA

SEQ ID NO: 32 (Asparaginase gene from Agrobacterium)

ATGACGAAGATCGCACTGGCCATTCACGGTGGTTGCGGCGTGATGCCGGAAGACAGCATGACGGCGGCG
G
AATGGGCCGCGGCCCGTGAAGATCTGGCAGCAGCGCTGCGGGCCGGTTATGGCGTGCTGAAGGCGGGCG
G
AACAGCGCTCGAGGCCGTTGAGGCAGCGGTCGTCGTCATGGAGGACAGCCCGCACTTCAATGCGGGACA
C
GGGGCGGCGCTGAACGAAAACGGCATTCACGAACTCGATGCCTCGATCATGGACGGGGCCACGCTTTCG
G

CAGGCGCGATCAGCGCATCCCGCGCCATTCGCAATCCTGTGAAGGCGGCCCGCGCACTGATGGTGGATG
A
ACGGGCGGTCTATCTCACAGGAGAGGCTGCGGATCGCTTTGCCACGGAGAAGGGTCTCGCCACCGAACC
T
CAGTCCTATTTCACCACGCAAAAACGCCTCGAGGCACTGGCAGCGATGAAGCGCCATGCAGCCACAGGC
A
CGGAAGCGACGGAAAACGAAAAGCACGGAACCGTCGGCGCGGTGGCGCTCGATGCGGCGGGCACCTTG
C
TGCGGCCACCTCAACCGGCGGCTATACCAACAAGCCGGATGGCCGGGTGGGCGACAGCCCCGTGATCGG
C
GCCGGCACCTATGCGCGCGACGGCGCCTGCGCGGTCTCCGGCACCGGCAAGGGTGAGTTTTTCATCCGT
T
ATGTCGTCGGCCACGAGATCGCGTCACGCGTCGCCTATCTCGGACAGGATCTGGAAACCGCCGCCGGCA
A
TCTCGTGCACAGGGACCTGGCTCCCTATGATATCGGTGCCGGTCTGGTCGCCATTGATGCGAAGGGCGG
C
ATTACCGCTCCGTACAATACACCAGGCATGTTCCGCGGCTGGGTTACGGCGTCTGGAGAGGCGTTTGTG
G
CCACTCACGCTGAAGCTTACGCCGTCAAATTATAA

SEQ ID NO: 33 (Asparaginase gene from barley)

ATGGCGCGCTGGGCCATTGCCATCCACGGCGGCGCGGGCGTGGACCCGAACCTGCCGGAGCACAGGCAG
G
AGGAGGCCAAGCGGGTGCTGGCCCGGTGCCTGCAGGTGGGCGTCGACCTGCTGCGCGCCGGCGCCACCG
C
GCTGGACGTGGTGGAGGCCGTGGTGCGGGAGCTGGAGACGGACCCCTGCTTCAACTCGGGCCGCGGCTC
C
GCGCTCACCCGCGCCGGCACCGTCGAGATGGAGGCCAGCATCATGGACGGCCGCGGCCGCCGCTGCGGC
G
CCGTCTCCGGCGTCTCCACCGTTAAAAACCCCGTCTCCCTCGCCCGCCGCGTCATGGACAGGTCCCCGC
A
CTCCTACCTCGCCTTCGACGGCGCCGAGGATTTCGCCCGCGAGCAGGGTCTTGAGGTTGTGGACAACAG
C
TACTTCATCACGGAGGAGAACGTGGGCATGCTCAAGCTCGCCAAGGAGGCCAACAGCATCCTCTTCGAC
T
ACCGCATCCCGCTCGCCGGGGCCGACACCTGCAGCGCGCAGGCGGCGGCGACCGAGAACCACAACAACA
A
CGGCATGGTGATGAACGGGCTGCCCATCAGCATCTACGCGCCGGAGACGGTGGGGTGCGCCGTGGTGGA
C

TGTAACGGCTTCACGGCGGCGGCCACCTCCACGGGCGGGCTCATGAACAAGATGACGGGCCGCATCGGC
G
ACTCGCCGCTCATCGGCGCTGGCACCTACGCGTGCGGGCACTGCGCCGTGTCGTGCACGGGCGAGGGCG
A
GGCCATCATCCGCTCCACGCTGGCGCGGACGTGGCCGCCGTGATGGAATCAAGGGGCTGCCTTCTGCA
G
GAGGCCGTGGACTTCTGCGTCAAGGAACGGCTCGACGAAGGGTTCGCCGGGCTCATCGCCGTGTCCGGC
A
CCGGCGAGGTGGCATACGGGTTCAACTGCACCGGCATGTTCAGAGGCTGCGCCACCGAGGACGGATTCA
T
GGAGGTCGGCATCTGGGAGTGA

SEQ ID NO: 34 (Ast gene from wheat)

CGCCGCTCCGTTCCGCCCGTACCTTACCCCTCCCCACCACCCGCGCCTGCGTCGCCGCCGGCGCCGTCG
CCGGCGACCGTCCCTCCTCGTCGGGCCGCCGCCGCCCCCGCCCCGTTCGTCCGCGGCGTCTGGCCAACG
AGGCGTGAGGTCCCGCCGGCCGCCACCATGTGCGGCATCCTCGCCGTCCTCGGCGTCGGCGACGTCTCC
CTCGCCAAGCGCTCCCGCATCATCGAGCTCTCCCGCCGATTACGGCACAGAGGCCCTGATTGGAGTGGT
ATACACAGCTTTGAGGATTGCTATCTTGCACACCAGCGGTTGGCTATTGTTGATCCCACATCTGGAGAC
CAGCCATTGTACAACGAGGACAAAACAGTTGTTGTGACGGTGAATGGAGAGATCTATAACCATGAAGAA
CTGAAAGCTAAGCTAAAATCTCATCAATTCCAAACTGGTAGTGATTGTGAAGTTATTGCTCACCTATAT
GAGGAATACGGGGAGGAATTTGTGGATATGCTGGATGGCATGTTCTCGTTTGTGCTTCTTGACACACGT
GATAAAAGCTTCATTGCTGCCCGTGATGCTATTGGCATCTGTCCTTTGTACATGGGCTGGGGTCTTGAT
GGGTCAGTTTGGTTTTCTTCAGAGATGAAGGCATTGAGTGATGATTGCGAGCGCTTCATATCGTTCCCC
CCTGGACACTTGTACTCAAGCAAAACAGGTGGCCTAAGGAGGTGGTACAACCCCCCATGGTTTTCAGAA
AGCATTCCCTCAGCCCCCTATGATCCTCTCCTCATCCGAGAGAGTTTTGAGAAGGCTGTTATTAAGAGG
CTAATGACTGATGTGCCATTTGGTGTTCTCTTGTCTGGTGGGCTTGACTCTTCTTTGGTGGCTTCTGTT
GTTTCACGCTACTTGGCAGAAACAAAAGTTGCTAGGCAGTGGGGAAACAAACTGCACACCTTTTGCATC
GGTTTGAAGGGTTCTCCTGATCTTAAAGCTGCTAAGGAAGTTGCTGACTACCTTGGCACAGTCCATCAT
GAATTACACTTTACAGTGCAGGAGGGCATTGATGCTTTTGGAAGAAGTTATATATCACATCGAGACGTA
TGACGTAACGACCATTAGAGCAAGTACCCCGATGTTTCTAATGTCTCGGAAAATCAAATCGTTGGGTGT
GAAGATGGTTCTTTCGGGTGAAGGTTCCGATGAAATATTTGGTGGTTATCTTTATTTTCATAAGGCACC
AAACAAAAAGGAACTCCATGAGGAAACATGTCGGAAGATAAAAGCTCTCCATTTATATGATTGTTTGAG
AGCGAACAAAGCAACTTCTGCCTGGGGTCTCGAGGCTCGTGTTCCATTCCTCGACAAAAACTTCATCAA
TGTAGCAATGGACCTGGATCCGGAATGTAAGATGATAAGGCGTGATCTTGGCCGGATCGAGAAATGGGT
CCTGCGTAATGCATTTGATGATGAGAAGAAGCCCTATTTACCCAAGCACATTCTTTACAGGCAAAAAGA
ACAGTTCAGCGATGGTGTTGGGTACAGTTGGATTGATGGATTGAAGGACCATGCTAATGCACATGTGTC
AGATTCCATGATGACGAACGCCAGCTTTGTTTACCCTGAAAACACACCCACAACAAAAGAAGCCTACTA
TTATAGGACAGTATTTGAGAAGTTTTATCCCAAGAATGCTGCTAGGCTAACGGTGCCAGGAGGTCCCAG
CGTTGCATGCAGCACCGCGAAAGCTGTTGAATGGGACGCCGCCTGGTCCAAGCTCCTCGACCCATCTGG

CCGTGCTGCTCTCGGTGTGCATGACGCGGCATATGAAGAAGAAAAGGCTCCTGCGTTGGCCGATCATGT
CTTCCGTCCACCAGCCCACGGGGAGAGCATCCTAGTCGAAACTGGTGTTCCAGCAGCAGCTGTTTAACT
TTCCATTCCATGGTTTCATAAAATGCTTGAGAAAATGTTGTCGCTTAGTTCAATTCTAGCGTTGCAACT
TGTCCGTAGCTTCAATCATTCAGTGTAGAAATTCCTGTGCACCATTTTCCTTGATGCTTGCTGGTATGT
CATGCTTTTCGCATGTATGTACTAAGTTTATGTGGTGAGCAGTGCATGGTAAATATTTCACCATGGTTG
TACATCCGAATTGCTCAAAGTCTGGGTTGCAACCTGGAAAAGTTTCATTAATAAACCCCAAGGTGT

SEQ ID NO: 35 (Ast gene from wheat)

TACGACAACCCACACGTCCGGGACTGGAGCACGAGGACACGGACATGGACTGACCCCGTAGAAATTCCC
ATCCTCTTTCAGAAGCACAGAGAGAGATCTTCTAGCTACATACTGTTGCCGTCGATCCAGCGAAAATGT
GCGGCATACTGGCGGTGCTGGGCTGCGCTGATGACACCCAGGGGAAGAGAGTGCGCGTGCTCGAGCTCT
CGCGCAGGCTCAAGCACCGCGGCCCCGACTGGAGCGGCATGCACCAGGTTGGCGACTGCTACCTCTCCC
ACCAGCGCCTCGCCATCATCGACCCTGCCTCTGGCGACCAGCCGCTCTACAACGAGGACAAGTCCATCG
TCGTCACAGTGAATGGAGAGATCTACAACCATGAACAGCTCCGGGCGCAGCTCTCCTCCCACACGTTCA
GGACAGGCAGCGACTGCGAGGTCATCGCACACCTGTACGAGGAGCATGGGGAGAACTTCATCGACATGC
TGGATGGTGTCTTCTCCTTCGTCTTGCTCGATACACGCGACAACAGCTTCATTGCTGCACGTGATGCCA
TTGGCGTCACACCCCTCTATATTGGCTGGGGAATTGATGGGTCGGTGTGGATATCATCAGAGATGAAGG
GCCTGAATGATGATTGTGAGCACTTTGAGATCTTTCCTCCTGGCCATCTCTACTCCAGCAAGCAGGGAG
GCTTCAAGAGATGGTACAACCCACCTTGGTTCTCCGAGGTCATTCCTTCAGTGCCATATGACCCACTTG
CTCTCAGGAAGGCTTTCGAAAAGGCTGTCATCAAGAGGCTTATGACGGACGTTCCATTCGGTGTTCTAC
TCTCTGGTGGCCTTGACTCATCATTGGTTGCAGCCGTTACAGTTCGTCACCTGGCAGGAACAAAGGCTG
CAAAGCGCTGGGGGACTAAGCTTCACTCTTTTTGTGTCGGACTTGAGGGGTCACCTGATCTGAAGGCTG
CAAAGGAGGTAGCCAATTACCTGGGCACCATGCACCATGAGTTCACCTTCACTGTTCAGGACGGCATTG
ATGCAATTGAGGATGTGATTTATCACACCGAAACATATGATGTGACGACAATCAGGGCAAGCACGCCAA
TGTTCCTGATGTCACGCAAGATCAAGTCACTTGGGGTCAAGATGGTCATCTCTGGTGAGGGTTCCGATG
AGATTTTCGGAGGGTACCTCTACTTCCACAAGGCACCCAACAAAGAGGAGCTCCACCGTGAGACATGTC
AAAAGATCAAAGCTCTGCATCAGTACGATTGCTTGAGGGCCAACAAGGCAACATCTGCATGGGGCCTCG
AAGCACGTGTGCCATTCTTGGACAAGGAGTTTATCAATGAGGCAATGAGCATTGATCCTGAGTGGAAGA
TGATCCGGCCTGATCTTGGAAGAATTGAGAAATGGGTGCTGAGGAAAGCATTTGATGACGAGGAGCAAC
CATTCCTGCCGAAGCACATTCTGTACAGGCAGAAAGAGCAGTTCAGTGATGGTGTTGGCTACAGCTGGA
TTGATGGCCTAAAGGCTCACGCAGAATCAAATGTGACAGATAAGATGATGTCAAATGCAAAGTTCATCT
ACCCACACAACACCCCGACTACAAAAGAGGCCTACTGTTACAGGATGATATTTGAGAGGTTCTTCCCCC
AGAACTCGGCGATCCTGACGGTGCCAGGTGGGCCAAGCGTTGCATGCAGCACGGCGAAGGCGGTAGAGT
GGGATGCCCAGTGGTCAGGGAACCTGGATCCCTCAGGGAGAGCAGCACTTGGAGTCCATCTCTCGGCCT
ATGAACAGGAGCATCTCCCAGCAACCATCATGGCAGGAACCAGCAAGAAGCCGAGGATGATCGAGGTTG
CGGCGCCTGGTGTCGCAATTGAGAGTTGATGGTGTCCTGTCCTGCTTGCCGTTTCTGATAAGAAATAAG
ATGTACCTGGTCTTGCCATTAGAGTGGTGCAGACCTAAGGTTTGAGTGAAGATTGTGCATTAATGTTTC
TATTGTTCTTATGACGATTTGTAATCCTTTTCTGGCAACTTCCATCAAAACATTATTACATGATGGTTA
TTATTTGACATAAACGGCTACATCTACCC

## Claims

1. A method for reducing the acrylamide content in a heat-processed plant product, comprising reducing asparagine levels in the plant that is used to produce the product by expressing in the plant a polynucleotide that has the complete or partial sense or antisense sequence of the coding or non-coding sequence of a gene that encodes an enzyme that catalyses the synthesis of asparagine from aspartate.

2. The method of claim 1, wherein the step of reducing asparagine levels comprises expressing in the plant a polynucleotide which comprises the complete or partial sequence of the promoter or coding sequence of a gene that encodes an enzyme that catalyses the synthesis of asparagine from aspartate.

3. The method of claim 1 or 2, wherein the polynucleotide comprises at least one of:
(a) the complete or partial sense and/or antisense sequence from a gene or promoter of that gene selected from (i) asparagine synthetase genes, (ii) nitrate reductase genes and (iii) hexokinase genes, wherein expression of the complete or partial sequence downregulates the mRNA levels of the endogenous copy of that gene, and
(b) the complete or partial sequence of a gene selected from (i) asparaginase genes and (ii) glutamine synthetase genes, wherein the sequence is overexpressed to upregulate total mRNA levels of that gene.

4. The method of claim 2 or 3, wherein the polynucleotide comprises:
(a) at least part of an asparagine synthetase gene and comprises a sequence that displays at least 90% identity to at least part of the sequence depicted in SEQ ID NOs: 1 or 2; and/or
(b) a functionally-active asparaginase gene and comprises a sequence that displays at least 70% identity to the sequence depicted in SEQ ID NOs: 9, 14, or 31-33.

5. The method of any one of claims 2 to 4, wherein the polynucleotide is operably linked to at least one tissue-specific plant promoter which may optionally be a tuber or seed-specific promoter.

6. The method of any one of claims 2 to 5, wherein the promoter is at least 70% identical to at least part of:
(a) a potato granule bound starch synthase promoter, which optionally comprises at least part of the sequence depicted in SEQ ID NO: 8;
(b) a potato ADP-glucose pyrophosphorylase gene promoter, which optionally comprises at least part of the sequence depicted in SEQ ID NO: 7;
(c) a potato patatin promoter, which optionally comprises at least part of the sequence depicted in SEQ ID NO: 22;
(d) a potato flavonoid 3'-monooxygenase gene promoter, which optionally comprises at least part of the sequence upstream from the sequence depicted in SEQ ID NO: 13; or
(e) a wheat puroindoline gene promoter.

7. The method of any one of claims 2 to 6, wherein:
(a) the polynucleotide comprises at least one copy of a sense and/or antisense fragment of an asparagine biosynthetic gene, and is expressed to down-regulate total asparagine synthetase mRNA levels in the tissues of a plant that are used to produce a heat-processed food product; and/or
(b) the polynucleotide is operably linked to a promoter at its 5'-end and is operably linked to a promoter at its 3'-end.

8. The method of any one of claims 2 to 7, further comprising expressing a second polynucleotide that comprises at least one copy, in the sense and/or antisense orientation, of a gene selected from an R1 gene and a phosphorylase L gene.

9. The method of claim 8, wherein the first and second polynucleotide are positioned within a transfer DNA and comprise a sequence that shares at least 70% identity with the sequence shown in SEQ ID NO.: 23.

10. The method of any one of the preceding claims, wherein the asparagine levels are reduced in the starchy tissue of the plant.

11. A method for producing an edible plant product from a plant tissue with reduced asparagine, comprising (1) increasing the level of asparaginase in the plant tissue or (2) decreasing the level of asparagine synthetase in the plant tissue.

12. The method of claim 11, wherein the step of increasing the level of asparaginase in the plant cell comprises expressing an asparaginase gene in the cell.

13. The method of claim 11 or 12, wherein the step of decreasing the level of asparagine synthetase in the plant cell comprises expressing in the plant cell a polynucleotide that comprises at least one fragment, in the sense and/or antisense orientation, of (i) an R1 gene, (ii) a phosphorylase L gene, and (iii) an asparagine synthetase gene.

14. The method of any one of claims 11 to 13, wherein the edible plant product is a tuber, French fry, chip, crisp, baked potato, or dehydrated potato.

15. A method for producing an edible plant product with low levels of acrylamide, comprising (i) downregulating the expression of an asparagine biosynthetic gene and/or upregulating the expression of a gene involved in asparagine metabolism, and (ii) downregulating the expression or activity of at least one of (a) the R1 gene and (b) the phosphorylase L gene in the tissue of a plant that produces a vegetable, seed, or fruit from which the product is made.

16. The method of claim 15, wherein the step of downregulating the R1 gene, phosphorylase L gene, and asparagine biosynthetic gene comprises expressing in the plant cell, in the sense and/or antisense orientation, at least one fragment of (i) an R1 gene, (ii) a phosphorylase L gene, and (iii) an asparagine synthetase gene.

17. The method of claim 15 or 16, wherein the edible plant product is a tuber, French fry, chip, crisp, or baked potato.

18. The method of any one of claims 11 to 16, wherein the edible plant product has a lower level of acrylamide after the product is heated compared to the level of acrylamide in a plant product:
(a) in which the level of asparaginase has not been increased or the level of asparagine synthetase has not been decreased; or
(b) of a non-transgenic plant, respectively.

19. A method for producing an edible transgenic plant product that has a lower acrylamide level after it is heated than the acrylamide level of an equivalently heated product from a non-transgenic plant, comprising reducing asparagine levels in the transgenic plant by altering the expression level of a gene involved in asparagine biosynthesis or asparagine metabolism in the plant.

20. The method of claim 19, wherein asparagine levels are reduced by expressing:
(a) at least one copy, in the sense and/or antisense orientation, of the complete or partial sequence from an asparagine synthetase gene, nitrate reductase gene, or hexokinase gene; and/or
(b) the complete or functionally-active partial sequence from an asparaginase gene or a glutamine synthetase gene in the plant.

21. A method for over-expressing a gene in a potato tuber by operably linking that gene to a sequence that displays at least 70% identity with at least part of the sequence shown in SEQ ID NO.: 13.

22. The method of claim 1, wherein the gene that encodes the enzyme that catalyses the synethesis of asparagine from aspartate is an asparagine synthetase gene.

23. A method for reducing the acrylamide content in a heat-processed plant product, comprising downregulating the expression of an asparagine synthetase gene in plant that is used to produce a heat-processed plant product.

24. The method of claim 23, wherein the step of downregulating the expression of an asparagine synthetase gene comprises expressing in the plant a sense-or antisense-oriented polynucleotide that is complementary to an asparagine synthetase gene sequence.

25. The method of claim 23 or 24, wherein the asparagine synthetase gene is the asparagine synthetase-1 gene or the asparagine synthetase-2 gene.

26. The method of claim 1, wherein the expression of an asparagine synthetase gene in plant that is used to produce a heat-processed plant product is downregulated by expressing in the plant a sense-or antisense-oriented polynucleotide that is partially or completely complementary to an asparagine synthetase gene sequence.

## Patentansprüche

1. Verfahren zum Verringern des Acrylamidgehalts in einem mit Wärme behandelten Pflanzenprodukt, umfassend das Verringern von Asparagin-Spiegeln in der Pflanze, die verwendet wird, um das Produkt durch Exprimieren eines Polynukleotids in der Pflanze zu produzieren, das die vollständige oder partielle Sense- oder Antisense-Sequenz der kodierenden oder nicht-kodierenden Sequenz eines Gens aufweist, das ein Enzym kodiert, das die Synthese von Asparagin aus Aspartat katalysiert.

2. Verfahren nach Anspruch 1, wobei der Schritt des Verringerns von Asparagin-Spiegeln das Exprimieren eines Polynukleotids in der Pflanze umfasst, welches die vollständige oder partielle Sequenz des Promotors oder der kodierenden Sequenz eines Gens umfasst, das ein Enzym kodiert, das die Synthese von Asparagin aus Aspartat katalysiert.

3. Verfahren nach Anspruch 1 oder 2, wobei das Polynukleotid mindestens eines der folgenden umfasst:
(a) die vollständige oder partielle Sense- und/oder Antisense-Sequenz eines Gens oder Promotors dieses Gens, das ausgewählt ist aus (i) Asparagin-Synthetase-Genen, (ii) Nitrat-Reduktase-Genen und (iii) Hexokinase-Genen, wobei die Expression der vollständigen oder partiellen Sequenz die mRNA-Spiegel der endogenen Kopie dieses Gens herunterreguliert, und
(b) die vollständige oder partielle Sequenz eines Gens, das ausgewählt ist aus (i) Asparaginase-Genen und (ii) Glutamin-Synthetase-Genen, wobei die Sequenz überexprimiert ist, um die gesamten mRNA-Spiegel dieses Gens hochzuregulieren.

4. Verfahren nach Anspruch 2 oder 3, wobei das Polynukleotid umfasst:
(a) mindestens einen Teil eines Asparagin-Synthetase-Gens und das eine Sequenz umfasst, die mindestens 90% Identität zu mindestens einem Teil der Sequenz zeigt, die in der SEQ ID Nr. 1 oder 2 dargestellt ist; und/oder
(b) ein funktionell aktives Asparaginase-Gen und das eine Sequenz umfasst, die mindestens 70% Identität zu der Sequenz zeigt, die in der SEQ ID Nr. 9, 14 oder 31-33 dargestellt ist.

5. Verfahren nach einem der Ansprüche 2 bis 4, wobei das Polynukleotid mit mindestens einem gewebespezifischen Pflanzenpromotor funktionsfähig verbunden ist, welcher gegebenenfalls ein Knollen- oder Samen-spezifischer Promotor ist.

6. Verfahren nach einem der Ansprüche 2 bis 5, wobei der Promotor mindestens 70% Identität zu mindestens einem Teil eines:
(a) Körnchen-gebundene Stärke-Synthase-Promotors (granule bound starch-Synthase-Promotor) der Kartoffel, welcher gegebenenfalls mindestens einen Teil der Sequenz umfasst, die in SEQ ID Nr. 8 dargestellt ist;
(b) ADP-Glukose-Pyrophosphorylase-Gen-Promotors der Kartoffel, welcher gegebenenfalls mindestens einen Teil der Sequenz umfasst, die in SEQ ID Nr. 7 dargestellt ist;
(c) Kartoffel-Patatin-Promotors, welcher gegebenenfalls mindestens einen Teil der Sequenz umfasst, die in SEQ ID Nr. 22 dargestellt ist;
(d) Flavonoid-3'-Monooxygenase-Gen-Promotors der Kartoffel, welcher gegebenenfalls mindestens einen Teil der Sequenz umfasst, die sich stromaufwärts zu der Sequenz befindet, die in SEQ ID Nr. 13 dargestellt ist; oder
(e) Weizen-Puroindolin-Gen-Promotors
aufweist.

7. Verfahren nach einem der Ansprüche 2 bis 6, wobei:
(a) das Polynukleotid mindestens eine Kopie eines Sense und/oder Antisense-Fragments eines biosynthetischen Asparagin-Gens umfasst, und exprimiert wird, um die Asparagin-Synthetase-mRNA-Gesamtspiegel in den Geweben einer Pflanze herunterzuregulieren, die verwendet wird, um ein mit Wärme behandeltes Nahrungsmittelprodukt herzustellen; und/oder
(b) das Polynukleotid an seinem 5'-Ende mit einem Promotor funktionsfähig verbunden ist und an seinem 3'-Ende mit einem Promotor funktionsfähig verbunden ist.

8. Verfahren nach einem der Ansprüche 2 bis 7, das ferner die Expression eines zweiten Polynukleotids umfasst, das mindestens eine Kopie in der Sense- und/oder Antisense-Richtung eines Gens umfasst, das aus einem R1-Gen und einem Phosphorylase L-Gen ausgewählt ist.

9. Verfahren nach Anspruch 8, wobei das erste und zweite Polynukleotid innerhalb einer Transfer-DNA positioniert sind und eine Sequenz umfassen, die mindestens 70% Identität mit der Sequenz teilt, die in SEQ ID Nr. 23 gezeigt ist.

10. Verfahren nach einem der vorangegangenen Ansprüche, wobei die Asparagin-Spiegel im stärkehaltigen Gewebe der Pflanze verringert sind.

11. Verfahren zum Produzieren eines essbaren Pflanzenprodukts aus einem Pflanzengewebe mit verringertem Asparagin, das (1) das Erhöhen des Asparaginase-Spiegels in dem Pflanzengewebe oder (2) das Verringern des Asparagin-Synthase-Spiegels in dem Pflanzengewebe umfasst.

12. Verfahren nach Anspruch 11, wobei der Schritt des Erhöhens des Asparaginase-Spiegels in der Pflanzenzelle das Exprimieren eines Asparaginase-Gens in der Zelle umfasst.

13. Verfahren nach Anspruch 11 oder 12, wobei der Schritt des Verringerns des Asparagin-Synthase-Spiegels in der Pflanzenzelle das Exprimieren eines Polynukleotids in der Pflanzenzelle umfasst, das mindestens ein Fragment in der Sense- und/oder Antisense-Richtung, (i) eines R1-Gens, (ii) eines Phosphorylase L-Gens und (iii) eines Asparagin-Synthetase-Gens umfasst.

14. Verfahren nach einem der Ansprüche 11 bis 13, wobei das essbare Pflanzenprodukt eine Knolle, Pommes Frites, Chips, Chipsletten, gebackene Kartoffel oder Trockenkartoffel ist.

15. Verfahren zum Produzieren eines essbaren Pflanzenprodukts mit niedrigen Acrylamid-Spiegeln, umfassend (i) das Herunterregulieren der Expression eines biosynthetischen Asparagin-Gens und/oder das Hochregulieren der Expression eines Gens, das am Asparagin-Metabolismus beteiligt ist und (ii) das Herunterregulieren der Expression oder der Aktivität von mindestens einem von (a) dem R1-Gen und (b) dem Phosphorylase L-Gen in dem Gewebe einer Pflanze, die ein Gemüse, einen Samen oder eine Frucht produziert, aus welchem das Produkt hergestellt wird.

16. Verfahren nach Anspruch 15, wobei der Schritt des Herunterregulierens des R1-Gens, des Phosphorylase L-Gens und des biosynthetischen Asparagin-Gens das Exprimieren mindestens eines Fragments (i) des R1-Gens, (ii) des Phosphorylase L-Gens und (iii) eines Asparagin-Synthetase-Gens in der Sense- und/oder Antisense-Richtung in der Pflanzenzelle umfasst.

17. Verfahren nach Anspruch 15 oder 16, wobei das essbare Pflanzenprodukt eine Knolle, Pommes Frites, Chips, Chipsletten oder gebackene Kartoffel ist.

18. Verfahren nach einem der Ansprüche 11 bis 16, wobei das essbare Pflanzenprodukt einen niedrigeren Acrylamid-Spiegel aufweist, nachdem das Produkt erhitzt worden ist, verglichen mit dem Acrylamid-Spiegel in einem Pflanzenprodukt:
(a) in welchem der Asparaginase-Spiegel nicht erhöht worden ist oder der Asparagin-Synthetase-Spiegel nicht verringert worden ist; oder
(b) jeweils einer nicht-transgenen Pflanze.

19. Verfahren zum Produzieren eines essbaren transgenen Pflanzenprodukts, das einen niedrigeren Acrylamid-Spiegel aufweist, nachdem es erhitzt worden ist, als der Acrylamid-Spiegel eines äquivalent erhitzten Produktes einer nicht-transgenen Pflanze, umfassend das Verringern von Asparagin-Spiegeln in der transgenen Pflanze durch Ändern des Expressionsspiegels eines Gens, das an der Asparagin-Biosynthese oder dem Asparagin-Metabolismus in der Pflanze beteiligt ist.

20. Verfahren nach Anspruch 19, wobei die Asparagin-Spiegel durch das Exprimieren
(a) mindestens einer Kopie in der Sense- und/oder Antisense-Richtung der vollständigen oder partiellen Sequenz eines Asparagin-Synthetase-Gens, Nitrat-Reduktase-Gens oder Hexokinase-Gens; und/oder
(b) der vollständigen oder funktionell aktiven Teilsequenz eines Asparaginase-Gens oder eines Glutamin-Synthetase-Gens in der Pflanze
verringert werden.

21. Verfahren zum Überexprimieren eines Gens in einer Kartoffelknolle durch funktionsfähiges Verbinden dieses Gens mit einer Sequenz, die mindestens 70% Identität mit mindestens einem Teil der Sequenz zeigt, die in SEQ ID Nr. 13 gezeigt ist.

22. Verfahren nach Anspruch 1, wobei das Gen, das das Enzym kodiert, das die Synthese von Asparargin aus Aspartat katalysiert, Asparagin-Synthetase-Gen ist.

23. Verfahren zum Verringern des Acrylamid-Gehalts in einem mit Wärme behandelten Pflanzenprodukt, umfassend das Herunterregulieren der Expression eines Asparagin-Synthetase-Gens in einer Pflanze, die verwendet wird, um ein mit Wärme behandeltes Pflanzenprodukt zu produzieren.

24. Verfahren nach Anspruch 23, wobei der Schritt des Herunterregulierens der Expression eines Asparagin-Synthetase-Gens das Exprimieren eines Polynukleotids in Sense- oder Antisense-Richtung in der Pflanze umfasst, das zu einer Asparagin-Synthetase-Gensequenz komplementär ist.

25. Verfahren nach Anspruch 23 oder 24, wobei das Asparagin-Synthetase-Gen das Asparagin-Synthetase-1-Gen oder das Asparagin-Synthetase-2-Gen ist.

26. Verfahren nach Anspruch 1, wobei die Expression eines Asparagin-Synthetase-Gens in einer Pflanze, die verwendet wird, um ein mit Wärme behandeltes Pflanzenprodukt zu produzieren, durch Exprimieren eines Polynukleotids in Sense- oder Antisense-Richtung in der Pflanze herunterreguliert wird, das teilweise oder vollständig zu einer Asparagin-Synthetase-Gensequenz komplementär ist.

## Revendications

1. Procédé permettant de réduire la teneur en acrylamide d'un produit à base de plante et traité à chaud, lequel procédé comporte le fait de réduire les taux d'asparagine chez la plante utilisée pour obtenir le produit, par expression chez la plante d'un polynucléotide qui comporte la séquence complète ou partielle, sens ou anti-sens, de la séquence codante ou non-codante d'un gène codant une enzyme qui catalyse la synthèse d'asparagine à partir d'aspartate.

2. Procédé conforme à la revendication 1, dans lequel l'étape de réduction des taux d'asparagine comporte l'expression chez la plante d'un polynucléotide qui comporte la séquence complète ou partielle de la séquence promoteur ou de la séquence codante d'un gène codant une enzyme qui catalyse la synthèse d'asparagine à partir d'aspartate.

3. Procédé conforme à la revendication 1 ou 2, dans lequel le polynucléotide comporte au moins l'une des séquences suivantes :
a) la séquence complète ou partielle, sens et/ou anti-sens, d'un gène ou de son promoteur, lequel gène est choisi parmi (i) les gènes d'asparagine synthétase, (ii) les gènes de nitrate réductase et (iii) les gènes d'hexokinase, l'expression de cette séquence complète ou partielle régulant à la baisse les taux d'ARNm de la copie endogène de ce gène,
b) et la séquence complète ou partielle d'un gène choisi parmi (i) les gènes d'asparaginase et (ii) les gènes de glutamine synthétase, cette séquence étant surexprimée pour réguler à la hausse les taux globaux d'ARNm de ce gène.

4. Procédé conforme à la revendication 2 ou 3, dans lequel le polynucléotide :
a) comporte au moins une partie d'un gène d'asparagine synthétase, et comprend une séquence identique, pour au moins 90 %, à au moins une partie de l'une des séquences présentées en tant que Séquence N° 1 et Séquence N° 2 ;
b) et/ou comporte un gène d'asparaginase fonctionnellement active et comprend une séquence identique, pour au moins 70 %, à l'une des séquences présentées en tant que Séquences N° 9, 14 et 31 à 33.

5. Procédé conforme à l'une des revendications 2 à 4, dans lequel le polynucléotide est opérationnellement attaché à au moins un promoteur de plante à spécificité tissulaire qui peut, en option, être un promoteur spécifique de semence ou de tubercule.

6. Procédé conforme à l'une des revendications 2 à 5, dans lequel le promoteur est identique, pour au moins 70 %, à au moins une partie :
a) d'un promoteur du gène d'amidon synthétase granulaire de pomme de terre, qui comprend, en option, au moins une partie de la séquence présentée en tant que Séquence N° 8 ;
b) d'un promoteur du gène d'ADP-glucose pyrophosphorylase de pomme de terre, qui comprend, en option, au moins une partie de la séquence présentée en tant que Séquence N° 7 ;
c) d'un promoteur du gène de patatine de pomme de terre, qui comprend, en option, au moins une partie de la séquence présentée en tant que Séquence N° 22 ;
d) d'un promoteur du gène de flavonoïde 3'-monooxygénase de pomme de terre, qui comprend, en option, au moins une partie de la séquence en amont de la séquence présentée en tant que Séquence N° 13 ;
e) ou d'un promoteur de gène de puroindoline de blé.

7. Procédé conforme à l'une des revendications 2 à 6, dans lequel :
a) le polynucléotide comprend au moins une copie d'un fragment sens et/ou anti-sens d'un gène de biosynthèse d'asparagine, et son expression régule à la baisse les taux globaux d'ARNm d'asparagine synthétase dans les tissus d'une plante qui sont utilisés pour la fabrication d'un produit alimentaire traité à chaud ;
b) et/ou le polynucléotide est opérationnellement attaché à un promoteur au niveau de son extrémité 5'-terminale et il est opérationnellement attaché à un promoteur au niveau de son extrémité 3'-terminale.

8. Procédé conforme à l'une des revendications 2 à 7, qui comporte en outre l'expression d'un deuxième polynucléotide qui comprend au moins une copie, orientée sens et/ou anti-sens, d'un gène choisi parmi un gène R1 et un gène de phosphorylase L.

9. Procédé conforme à la revendication 8, dans lequel les premier et deuxième polynucléotides sont placés au sein d'un ADN de transfert et comprennent une séquence identique, pour au moins 70 %, à la séquence présentée en tant que Séquence N° 23.

10. Procédé conforme à l'une des revendications précédentes, dans lequel c'est dans le tissu amylacé de la plante que les taux d'asparagine sont réduits.

11. Procédé de fabrication d'un produit végétal comestible à partir d'un tissu végétal à teneur réduite en asparagine, lequel procédé comporte :
1) le fait de faire augmenter le taux d'asparaginase dans le tissu végétal,
2) ou le fait de faire diminuer le taux d'asparagine synthétase dans le tissu végétal.

12. Procédé conforme à la revendication 11, dans lequel l'étape d'augmentation du taux d'asparaginase dans des cellules végétales comporte l'expression d'un gène d'asparaginase dans ces cellules.

13. Procédé conforme à la revendication 11 ou 12, dans lequel l'étape de diminution du taux d'asparagine synthétase dans des cellules végétales comporte l'expression, dans ces cellules, d'un polynucléotide qui comprend au moins un fragment, orienté sens ou anti-sens, (i) d'un gène R1, (ii) d'un gène de phosphorylase L, et (iii) d'un gène d'asparagine synthétase.

14. Procédé conforme à l'une des revendications 11 à 13, dans lequel le produit végétal comestible est des tubercules, des frites, des chips, des pommes de terre cuites au four ou des pommes de terre déshydratées.

15. Procédé de fabrication d'un produit végétal comestible à basse teneur en acrylamide, qui comporte :
i) le fait de réguler à la baisse l'expression d'un gène de biosynthèse de l'asparagine et/ou de réguler à la hausse l'expression d'un gène impliqué dans le métabolisme de l'asparagine,
ii) et le fait de réguler à la baisse l'expression ou l'activité d'au moins l'un (a) du gène R1 et (b) du gène de phosphorylase L dans un tissu d'une plante qui produit un légume, une semence ou un fruit à partir duquel ou de laquelle le produit est fabriqué.

16. Procédé conforme à la revendication 15, dans lequel la régulation à la baisse de l'expression du gène R1, du gène de phosphorylase L et d'un gène de biosynthèse de l'asparagine comporte l'expression, dans des cellules de la plante, d'au moins un fragment, orienté sens ou anti-sens, (i) d'un gène R1, (ii) d'un gène de phosphorylase L, et (iii) d'un gène d'asparagine synthétase.

17. Procédé conforme à la revendication 15 ou 16, dans lequel le produit végétal comestible est des tubercules, des frites, des chips ou des pommes de terre cuites au four.

18. Procédé conforme à l'une des revendications 11 à 16, dans lequel le produit végétal comestible présente, après chauffage, une teneur en acrylamide plus basse que celle d'un produit végétal
a) dans lequel on n'a pas fait augmenter le taux d'asparaginase ou diminuer le taux d'asparagine synthétase,
b) ou issu d'un plante non-transgénique, respectivement.

19. Procédé de fabrication d'un produit comestible issu d'une plante transgénique, présentant après chauffage une teneur en acrylamide plus basse que celle d'un produit issu d'une plante non-transgénique et chauffé de manière équivalente, lequel procédé comporte le fait de faire diminuer les taux d'asparagine dans la plante transgénique en modifiant le niveau d'expression d'un gène impliqué dans la biosynthèse de l'asparagine ou le métabolisme de l'asparagine chez la plante.

20. Procédé conforme à la revendication 19, dans lequel on fait diminuer les taux d'asparagine par expression chez la plante :
a) d'au moins une copie, orientée sens et/ou anti-sens, de la séquence complète ou partielle d'un gène d'asparagine synthétase, d'un gène de nitrate réductase ou d'un gène d'hexokinase ;
b) et/ou de la séquence complète, ou partielle et fonctionnellement active, d'un gène d'asparaginase ou d'un gène de glutamine synthétase.

21. Procédé permettant la sur-expression d'un gène dans un tubercule de pomme de terre, dans lequel on fait en sorte que ce gène soit opérationnellement attaché à une séquence qui est identique, pour au moins 70 %, à au moins une partie de la séquence présentée en tant que Séquence N° 13.

22. Procédé conforme à la revendication 1, dans lequel le gène gène codant une enzyme qui catalyse la synthèse d'asparagine à partir d'aspartate est un gène d'asparagine synthétase.

23. Procédé permettant de réduire la teneur en acrylamide d'un produit végétal traité à chaud, lequel procédé comporte la régulation à la baisse de l'expression d'un gène d'asparagine synthétase chez une plante utilisée pour fabriquer le produit végétal traité à chaud.

24. Procédé conforme à la revendication 23, dans lequel la régulation à la baisse de l'expression d'un gène d'asparagine synthétase comporte l'expression chez la plante d'un polynucléotide, orienté sens ou anti-sens, qui est complémentaire d'une séquence d'un gène d'asparagine synthétase.

25. Procédé conforme à la revendication 23 ou 24, dans lequel le gène d'asparagine synthétase est le gène asparagine synthétase-1 ou le gène asparagine synthétase-2.

26. Procédé conforme à la revendication 1, dans lequel l'expression d'un gène d'asparagine synthétase chez une plante utilisée pour fabriquer un produit végétal traité à chaud est régulée à la baisse par expression chez la plante d'un polynucléotide, orienté sens ou anti-sens, qui est complémentaire, en partie ou en totalité, d'une séquence d'un gène d'asparagine synthétase.
